# EUROPEAN PATENT SPECIFICATION

(11) **EP 0 518 819 B1**
(45) Date of publication and mention of the grant of the patent: **02.08.1995**
(21) Application number: 92810423.1
(22) Date of filing: 02.06.1992
(51) Int. Cl.: C07C 257/18, A61K 31/155, C07C 271/64, C07C 311/51, A61K 31/18

(54) **Amidino compounds, their manufacture and use as medicament**
Amidino-Verbindungen, ihre Herstellung und Verwendung als Arzneimittel
Dérivés d'amidine, leur préparation et utilisation comme médicament

(30) Priority: 11.06.1991 US 714108
(43) Date of publication of application: 16.12.1992
(73) Proprietor: CIBA-GEIGY AG, 4002 Basel (CH)
(72) Inventor: Morrissey, Michael M., Bloomfield, NJ 07003 (US)

(56) References cited:
- EP-A- 0 150 118
- EP-A- 0 366 066
- US-A- 4 808 604

## Description

The invention relates to the amidinophenoxyalkoxyphenyl derivatives and thio analogs as defined herein which are particularly useful as selective Leukotriene B₄ (LTB₄) receptor antagonists, methods for preparation thereof, pharmaceutical compositions comprising said compounds, and a method of antagonizing LTB-4 and of treating conditions or syndromes in mammals which are responsive to LTB-4 antagonism using said compounds or pharmaceutical compositions comprising said compounds of the invention.
EP-A-150118 discloses the structually divergent mono-(carboxamides, thiocarboxamides, carboxamidines) of formula I therein, the amides being derived from amino acids, and in which the second benzene ring carries the acyl group -C(=O)-R₄ in which R₄ is hydroxy, lower alkyl or lower alkoxy. The compounds mentioned in EP-A-150118 are indicated to be antagonists of leukotrienes B₄, C₄, D₄ and E₄. Furthermore there is no indication of any effect on interleukin activity.
Leukotriene B₄ (LTB₄) is an important inflammatory mediator being a potent chemotactic agent and activator of polymorphonuclear leucocytes (PMN's) and monocytes. It modulates the production and effects of other important inflammatory mediators e.g. Interleukin-1 and gamma interferon. LTB₄ has been implicated in the pathogenesis of a number of inflammatory diseases, such as rheumatoid arthritis, inflammatory bowel disease, psoriasis, non-steroidal-antiinflammatory-drug-induced gastropathy, adult respiratory distress syndrome (ARDS), myocardial infarction, allergic rhinitis, hemodialysis-induced neutropenia, and late phase asthma.

There is a strong need in the art in finding potent antagonists of LTB₄ on human PMN's, especially those which are orally active. It has been found that the compounds according to the present invention exhibit significant LTB₄ antagonistic activity on human PMN's and are orally active.

The invention relates to amidinophenoxyalkoxyphenyl derivatives and thio analogs of the formula 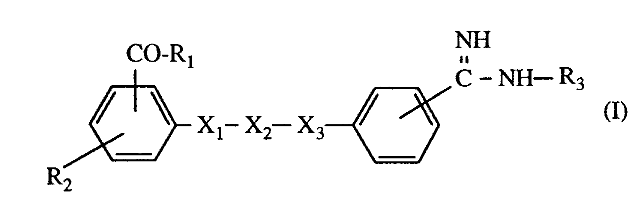
and pharmaceutically acceptable salts thereof, in which:
R₁ is amino which is mono- or disubstituted by a substituent selected from an aliphatic hydrocarbon radical, an araliphatic hydrocarbon radical, an aromatic radical, and a cycloaliphatic hydrocarbon radical or is amino which is disubstituted by a divalent aliphatic hydrocarbon radical;
R₂ is hydrogen, halogen, trifluoromethyl, an aliphatic hydrocarbon radical, or is hydroxy which is etherified by an aliphatic alcohol, araliphatic alcohol, or aromatic alcohol or which is esterified by an aliphatic or araliphatic carboxylic acid;
R₃ is hydrogen or an acyl radical which is derived from an organic carbonic acid, an organic carboxylic acid, a sulfonic acid, or a carbamic acid;
X₁ and X₃, independently of one another, are oxygen (-O-) or sulphur (-S-); and
X₂ is a divalent aliphatic hydrocarbon radical which may be interrupted by an aromatic radical;
wherein the phenyl rings of formula I may be, independently of one another, further substituted by one or more substituents selected from halogen, trifluoromethyl, an aliphatic hydrocarbon radical, hydroxy, and hydroxy which is etherified by an aliphatic alcohol or which is esterified by an aliphatic or araliphatic carboxylic acid;
wherein aryl in the above definitions may be, independently of one another, further substituted by one or more substituents selected from halogen, trifluoromethyl, an aliphatic hydrocarbon radical, hydroxy, and hydroxy which is etherified by an aliphatic alcohol or which is esterified by an aliphatic or araliphatic carboxylic acid;
wherein a cycloaliphatic hydrocarbon radical may be substituted by an aliphatic radical; which are particularly useful as selective LTB-4 antagonists, methods for preparation thereof, pharmaceutical compositions comprising said compounds, and a method of antagonizing LTB-4 and of treating diseases in mammals which are responsive to LTB-4 antagonism using said compounds or pharmaceutical compositions comprising said compounds of the invention.

As the compounds according to the invention have a basic centre, they can thus form acid addition salts, especially pharmaceutically acceptable salts. These are formed, for example, with inorganic acids, such as mineral acids, for example sulfuric acid, a phosphoric or hydrohalic acid, or with organic carboxylic acids, such as (C₁-C₄-)alkanecarboxylic acids which, for example, are unsubstituted or substituted by halogen, for example acetic acid, such as saturated or unsaturated dicarboxylic acids, for example oxalic, malonic, succinic, maleic, fumaric, phthalic or terephthalic acid, such as hydroxycarboxylic acids, for example ascorbic, glycolic, lactic, malic, tartaric or citric acid, such as amino acids, for example aspartic or glutamic acid, benzoic acid or with organic sulfonic acids, such as (C₁-C₄-)alkane- or arylsulfonic acids which are unsubstituted or substituted, for example, by halogen, for example methane- or toluenesulfonic acid. Preferred are salts formed with hydrochloric acid, methanesulfonic acid and maleic acid.
An aliphatic hydrocarbon radical is, for example, lower alkyl, lower alkenyl and secondarily lower alkynyl.

An araliphatic hydrocarbon radical is, for example, optionally substituted phenyl-lower alkyl and secondarily phenyl-lower alkenyl and phenyl-lower alkynyl.
A cycloaliphatic hydrocarbon radical is, for example, cycloalkyl and secondarily cycloalkenyl, which is unsubstituted or mono- or polysubstituted, for example, disubstituted, by lower alkyl.

An aromatic radical is, for example, unsubstituted or substituted such as monosubstituted, for example, disubstituted or secondarily trisubstituted, carboxyclic aryl, such as phenyl, naphthyl, indanyl or fluorenyl. Amino which is substituted by an aromatic radical preferably is amino which is monosubstituted by corresponding phenyl or secondarily by naphthyl.
A divalent aliphatic hydrocarbon radical is, for example, lower alkylene.
A divalent aliphatic hydrocarbon radical which is interrupted by an aromatic radical is, for example, lower alkylene-phenylene-lower alkylene or lower alkylene-naphthylene-lower alkylene.
An aliphatic alcohol is, for example, a lower alkanol or lower alkenol, and an araliphatic alcohol is, for example, a phenyl-lower alkanol.

An aromatic alcohol is, for example, a phenol which is unsubstituted or is further substituted such as monosubstituted, for example, disubstituted or secondarily trisubstituted.

Hydroxy which is etherified by an aliphatic or araliphatic alcohol is, for example, lower alkoxy or lower alkenyloxy and phenyl-lower alkoxy.

An aliphatic carboxylic acid is, for example, a lower alkanoic or lower alkenoic acid, and an araliphatic carboxylic acid is, for example, a phenyl-lower alkanoic acid.

Hydroxy which is esterified by an aliphatic or araliphatic carboxylic acid is, for example, lower alkanoyloxy, secondarily lower alkenoyloxy, or is phenyl-lower alkanoyloxy.

An acyl radical which is derived from an an organic carboxylic acid is, for example, lower alkanoyl, phenyl-lower alkanoyl or unsubstituted or substituted aroyl, such as benzoyl, naphthoyl, indanoyl or fluorenoyl, or heteroaroyl such as pyridylcarbonyl, thienylcarbonyl, pyrrolylcarbonyl, furanylcarbonyl, and imidazolylcarbonyl.

An acyl radical which is derived from an organic carbonic acid is, for example, alkoxycarbonyl or alkenyloxycarbonyl which in each case are unsubstituted or substituted by an aromatic radical or is cycloalkoxycarbonyl which unsubstituted or substituted by lower alkyl.

An acyl radical which is derived from a sulfonic acid is, for example, alkanesulfonyl, arylalkanesulfonyl, cycloalkanesulfonyl or arylsulfonyl.

An acyl radical which is derived from a carbamic acid is, for example, amino-carbonyl which is substituted by alkyl, arylalkyl or aryl.

An aromatic radical is, for example, unsubstituted or substituted such as monosubstituted or polysubstituted, for example, disubstituted or secondarily trisubstituted, carbocyclic aryl, such as phenyl, naphthyl, indanyl or fluorenyl, or heterocyclic aryl, such as pyridyl, thienyl, pyrrolyl, furanyl, and imidazolyl.

The phenyl rings of formulae I and IA as well as aromatic radicals referred to before and hereafter are generally unsubstituted or further substituted such as monosubstituted or polysubstituted, for example disubstituted or secondarily trisubstituted, in particular, for example, by a substituent selected from the group consisiting of halogen, trifluoromethyl, lower alkyl, lower alkenyl, lower alkynyl, hydroxy, lower alkoxy, lower alkenyloxy, phenyl-lower alkoxy, lower alkanoyloxy, lower alkenoyloxy, and phenyl-lower alkanoyloxy. Preferably, the phenyl rings of formula I and IA do not exhibit any additional substitutent.

Preferred positions of the following structural elements in the corresponding phenyl ring in formula I are: positions 4 (para) or 5 (meta) for -CO-R₁, position 2 (ortho) for R₂, and position 4 (para) for -C(=NH)-NH-R₃. The substituent R₃ may be located on either nitrogen of the -C(=NH)NH₂ grouping.

The general definitions used above and below have, if not defined differently, the following meanings:

The expression "lower" means that corresponding groups and compounds in each case contain in particular not more than 7, preferably not more than 4, carbon atoms.

Halogen is, in particular, fluorine, chlorine or bromine, and furthermore includes iodine. Lower alkyl is, in particular, C₁-C₇-alkyl and is, for example, methyl, ethyl, n-propyl, isopropyl, n-butyl, isobutyl, sec-butyl, tert-butyl and furthermore includes corresponding pentyl, hexyl and heptyl radicals. C₁-C₄-Alkyl is preferred.

Lower alkenyl is, in particular, C₃-C₇-alkenyl and is, for example, 2-propenyl or 1-, 2- or 3-butenyl. C₃-C₅-Alkenyl is preferred.

Lower alkynyl is, in particular, C₃-C₇-alkynyl and is preferably propargyl.

Phenyl-lower alkyl is, in particular, phenyl-C₁-C₄-alkyl and is preferably benzyl, 1- and 2-phenethyl, while phenyl-lower alkenyl and phenyl-lower alkynyl are, in particular, phenyl-C₂-C₅-alkenyl and -alkynyl, in particular 2-phenyl-vinyl, 3-phenylallyl and 3-phenylpropargyl.

Cycloalkyl is, in particular. C₃-C₇-cycloalkyl and is, for example, cyclopropyl, cyclobutyl, cyclopentyl, cyclohexyl and cycloheptyl. Cyclopentyl and cyclohexyl are preferred.

Cycloalkenyl is, in particular, C₃-C₇-cycloalkenyl and is preferably cyclopent-2- or -3-enyl, or cyclohex-2- and -3-en-yl.

Lower alkylene e.g. in amino which is disubstituted by lower alkylene is, in particular, C₂-C₆-alkylene and is, for example, butylene, pentylene, or 2,6-butylene. Preferred is C₄-C₅-alkylene, especially pentylene.

Lower alkylene X₂ is, in particular, C₂-C₈-alkylene, preferably straight-chain, and is, for example, ethylene, propylene, butylene, pentylene, hexylene, heptylene and also octylene. C₄-C₇-Alkylene is preferred, especially pentylene and also butylene, hexylene or heptylene.

Lower alkylene which is interrupted by a phenyl radical (X₂) is, in particular, lower alkylene-phenylene-lower alkylene or lower alkylene-naphthylene-lower alkylene such as C₂-C₄-alkylene-phenylene-C₂-C₄-alkylene or C₂-C₄-alkylene-naphthylene-C₂-C₄-alkylene, preferably straight-chain, and is, for example, methylene-phenylene-methylene, 1,2-ethylene-phenylene-1,2-ethylene, such as 1,2-ethylene-1,4-phenylene-1,2-ethylene, 1,3-propylene-phenylene-1,3-propylene, such as 1,3-propylene-1,4-phenylene-1,3-propylene, or butylene-phenylene-butylene radicals, also a corresponding 1,2-ethylene-naphthylene-1,2-ethylene radical.
C₂-C₄-alkylene-phenylene-C₂-C₄-alkylene or C₂-C₃-alkylene-naphthylene-C₂-C₃-alkylene is preferred, especially 1,2-ethylene-1,4-phenylene-1,2-ethylene.

Lower alkoxy is, in particular, C₁-C₇-alkoxy and is, for example, methoxy, ethoxy, n-propyloxy, isopropyloxy, n-butyloxy, isobutyloxy, sec-butyloxy, tert-butyloxy and furthermore includes corresponding pentyloxy, hexyloxy and heptyloxy radicals. C₁-C₄-Alkoxy is preferred.

Lower alkenyloxy is, in particular, C₃-C₇-alkenyloxy and is, for example, allyloxy or but-2-en- or but-3-enyloxy. C₃-C₅-Alkenyloxy is preferred.

Phenyl-lower alkoxy is, in particular, phenyl-C₁-C₄-alkoxy, such as benzyloxy, 1- or 2-phenylethoxy, or 1-, 2- or 3-phenylpropyloxy.

Lower alkanoyloxy is, in particular, C₂-C₈-alkanoyloxy, in particular, C₂-C₅-alkanoyloxy, such as acetyloxy, propionyloxy or pivaloyoxy.

Lower alkenoyloxy is, in particular, C₃-C₈-alkenoyloxy, in particular, C₃-C₅-alkenoyloxy, such as propenoyloxy.

Phenyl-lower alkanoyloxy is, in particular, phenyl-C₂-C₈-alkanoyloxy, in particular, phenyl-C₂-C₅-alkanoyloxy, such as phenylacetyloxy, phenylpropionyloxy or phenylpivaloyloxy.

Alkoxycarbonyl is, in particular, C₂-C₁₂-alkoxycarbonyl and is, for example, methoxy-, ethoxy-, propyloxy- pivaloyloxy- or octyloxy-carbonyl. C₂-C₉-Alkoxycarbonyl is preferred.

Alkenyloxycarbonyl is, in particular, C₃-C₁₂-alkenyloxycarbonyl, for example, allyloxycarbonyl. Preferred is C₃-C₅-alkenyloxycarbonyl.

Cycloalkyloxycarbonyl is, in particular, C₃-C₇-cycloalkoxycarbonyl, preferred is cyclopentyloxycarbonyl or cyclohexyloxycarbonyl.

Alkanesulfonyl is, in particular, C₁-C₇alkanesulfonyl and is, for example, methane-, ethane-, n-propane- or isopropanesulfonyl. C₁-C₄-alkanesulfonyl is preferred.

Arylalkanesulfonyl is, in particular, phenyl-C₁-C₇-alkanesulfonyl, for example, benzyl- or 1- or 2-phenylethan-sulfonyl. Phenyl-C₁-C₄-alkanesulfonyl is preferred.

Cycloalkanesulfonyl is, in particular, C₃-C₇-cycloalkanesulfonyl, preferred is cyclopentanesulfonyl or cyclohexanesulfonyl.

Naphthyl is 1- or 2-naphthyl.

Indanyl is, for example, 1-, 2-, 3- or 4-indanyl.

Fluorenyl is, for example, 1-, 2-, 3-, 4- or 5-fluorenyl.

Lower alkanoyl is, in particular, C₁-C₇-alkanoyl and is, for example, formyl, acetyl, propionyl, butyryl, isobutyryl or pivavolyl. C₂-C₅-Alkanoyl is preferred.

Phenyl-lower alkanoyl is, in particular, phenyl-C₂-C₇-alkanoyl and is, for example, phenylacetyl or 2- or 3-phenylpropionyl. Phenyl-C₂-C₄-alkanoyl is preferred.

Naphthoyl is 1- or 2-naphthoyl.

Indanoyl is, for example, 1-, 2-, 3- or 4-indanoyl.

Fluorenoyl is, for example, 1-, 2-, 3-, 4- or 5-fluorenoyl.

The compounds of the invention exhibit valuable pharmacological properties in mammals, and are particularly useful as selective Leukotriene B₄ (LTB₄) receptor antagonists, e.g. for the treatment of a condition or syndrome in a mammal responsive to the selective antagonism of LTB₄ receptors, such as rheumatoid arthritis, inflammatory bowel disease, psoriasis, non-steroidal-antiinflammatory-drug-induced gastropathy, adult respiratory distress syndrome (ARDS), myocardial infarction, allergic rhinitis, hemodialysis-induced neutropenia, and late phase asthma. The compounds of the invention are also useful as analgesics for the treatment of pain of any origin, and for the treatment of osteoarthritis.

The above-cited properties are demonstrable in in vitro and in vivo tests, using advantageously mammals, e.g. rats. Said compounds can be applied in vitro in the form of solutions, e.g. preferably aqueous solutions, and in vivo either enterally or parenterally, advantageously orally, e.g. as a suspension or in aqueous solution. The dosage in vitro may range between about 0.5 ng/ml and about 100 ng/ml. The dosage in vivo may range, depending on the route of administration, between about 1 and about 1000 mg/kg per day.

Beneficial effects are evaluated in pharmacological tests generally known in the art, e.g. as illustrated herein.

### Receptor Binding with [³H]-LTB₄ to Intact Human Neutrophils:

Neutrophils (PMN's) are prepared from uncoagulated human venous blood. Blood is dispersed into 50 ml polypropylene tubes containing 15 ml of HESPAN (Dupont, Wilmington, DE), and mixed. Tubes are allowed to stand at room temperature for 40 minutes until most of the red blood cells sediment. The supernatants are removed and centrifuged for 5-10 min at 400 x g. The remaining pellets are diluted in 70 ml of Phosphate Buffered Saline without calcium and magnesium (PBS without metals; GIBCO, Grand Island, NY) and 35 ml of this suspension are placed in each of two polypropylene tubes containing 15 nil of Ficoll-Paque (Sigma, St. Louis, MO). Gradients are then centrifuged for 15 minutes at 420 x g. The mononuclear cell layer is discarded and the remaining red blood cell pellet is resuspended in 10 ml of PBS without metals. Twenty ml of filtered deionized water are added to the suspension for approximately 20 sec followed by the same volume of buffer at two times the normal concentration. The cell suspension is mixed and centrifuged for 5 min at 200 x g, followed by one wash with buffer, and final resuspension.

Binding of [³H]LTB₄ to LTB₄ receptors is measured in intact human polymorphonuclear leukocytes, as described by Gorman and Lin (Gorman, R. and Lin, A Methods Enzymol. 141: 372-378, 1987). Intact human neutrophils are suspended in Hank's Balanced Salt Solution (HBSS) at a concentration of 3 x 106 cells/assay tube. An aliquot of the cell suspension (300 »l) is added to triplicate tubes containing 50 »l [3H]LTB₄ (specific activity 32 Ci/mmol, DuPont-NEN, Boston, MA) at a final concentration of 0.5 nM, 100 »l buffer and 50 »l drug or buffer. Nonspecific binding is determined in the presence of 300 nM LTB₄. The reaction is initiated by addition of cell suspension and continued at 0°C for 20 min. Bound radioactivity is isolated by vacuum filtration through Whatman GF/C glass fiber filters using a Brandel cell harvester and unbound radioactivity removed with 2 x 5 ml washes with ice-cold saline. Filters are placed in polyethylene scintillation mini-vials to which is added 3.5 ml of Formula-989 scintillation cocktail (NEN). After equilibration, radioactivity determinations and data calculations are performed using non-linear regression analysis on RS/1.

### LTB₄-Induced PMN Aggregation

Human PMNs are prepared as previously described. Neutrophil aggregation is assessed by monitoring the intensity of light passing through a suspension of cells (Craddock et al., J. Clin. Invest. 60: 260-264, 1977) using a Payton dual channel aggregometer (model 300BD). Cuvettes containing 0.25 ml of cell suspension (25 x 106 cells/ml) in PBS without calcium and magnesium are incubated with 5 »g/ml ml of cytochalasin B for 2 minutes at 37°C. 5 »l of 2 »M LTB₄ in PBS (20 nM final concentration) are added and the aggregation response monitored for 3-5 min, the time required for optimal response, Compounds are solubilized in 0.01M DMSO and then diluted in PBS to 0.001 M. 5 »l of compound solution is added along with cytochalasin B and cells as described above. Following the preincubation period 5 »l of 2 »M LTB₄ are added and aggregation is measured. Percent inhibition of aggregation is calculated by comparing peak heights in the presence and absence of compound. Percent inhibition is plotted as a function of the log concentration of compound and the IC₅₀ determined directly from the graph.

### LTB₄-Induced Neutropenia in the Rat

Male Sprague Dawley rats (crl: CDBR; Charles River, Wilmington, MA.) (250-300) grams are fasted overnight prior to the experiment. At least six animals are used per treatment group. Rats are given vehicle or compound either intravenously or orally and at intervals after dosing, neutrophil counts are determined from blood samples obtained just prior to and 20 seconds after intravenous infusion of 200 ng LTB₄. In studies are compound is administered orally, drug is given by gavage. When drug is administered intravenously, rats are first anesthetized with 50 mg/kg i.p. of Sodium Pentabarbital. The jugular vein is exposed and cleaned of the surrounding tissue. At 3, 4 or 18 hours following administration of compound or vehicle by either route, blood samples are taken (0.3 ml of blood in 1.5 ml polypropylene microcentrifuge tube containing 0.01 ml 7.5% EDTA). Blood neutrophil counts are determined using a Technicon H-1 hematology instrument. Antagonism of the LTB₄-induced neutropenia response for the compounds tested is calculated.

Analgesic activity can be demonstrated e.g. in the Randall-Selitto test for analgesia, e.g. as described in Arch. Int. Pharmacodyn. Ther. 111, 409 (1957).

Antiinflammatory activity can be demonstrated by measuring the inhibition of the edema and inhibition of the influx of polymorphonuclear (PMN's) and mononuclear leukocytes (monocytes and macrophages) after oral administration in the rat model in which pleurisy is first induced by injecting carrageenin into the pleural cavity, e.g. according to A.P. Almeida et al., J. Pharmacol. Exp. Therap. 214, 74 (1980), in particular during the late phase of the corrageenin-induced pleurisy.

Bronchial effects such as anti-asthmatic activity, can be demonstrated in the antigen-induced guinea pig bronchoconstriction test, e.g. as described by Anderson et al, Br. J. Pharmacol. 1983, 78, 67-74.

The trinitrobenzenesulfonic acid-induced chronic colitis test in the rat, e.g. as described by Wallace et al, Gastroenterology 1989, 96, 29-36, can be used to evaluate compounds for effects indicative of utility in inflammatory bowel diseases.

The arachidonic acid-induced mouse ear edema test, e.g. as described by Young et al, J. Invest, Dermatol. 1984, 82, 367-371 can be used to evaluate compounds for effects indicative of utility in dermatological disorders such as psoriasis.

The invention especially relates to compounds of formula I and pharmaceutically acceptable salts thereof, in which:
R₁ is amino which is mono- or disubstituted by a substituent selected from lower alkyl, lower alkenyl, lower alkynyl, phenyl-lower alkyl, phenyl-lower alkenyl, phenyl-lower alkynyl, phenyl, naphthyl, indanyl, fluorenyl, cycloalkyl, and cycloalkenyl, cycloalkyl and cycloalkenyl each being unsubstituted or mono- or polysubstituted by lower alkyl, or is amino which is disubstituted by lower alkylene;
R₂ is hydrogen, halogen, trifluoromethyl, lower alkyl, lower alkenyl, lower alkynyl, lower alkoxy, lower alkenyloxy, phenyl-lower alkoxy, phenoxy, lower alkanoyloxy, lower alkenoyloxy, or phenyl-lower alkanoyloxy;
R₃ is hydrogen, alkoxycarbonyl or alkenyloxycarbonyl, each of which is unsubstituted or substituted by phenyl, naphthyl, indanyl or fluorenyl, or is cycloalkoxycarbonyl being unsubstituted or mono- or polysubstituted by lower alkyl, or is lower alkanoyl or phenyl-lower alkanoyl, or is benzoyl, naphthoyl, indanoyl or fluorenoyl, or is C₁-C₇-alkanesulfonyl, phenyl-C₁-C₇-alkanesulfonyl, C₃-C₇-cycloalkanesulfonyl, or phenylsulfonyl, or is aminocarbonyl which is substituted by lower alkyl, phenyl-lower alkyl or phenyl;
X₁ and X₃, independently of one another, is O or S;
X₂ is lower alkylene, lower alkylene-phenylene-lower alkylene or lower alkylene-naphthylene-lower alkylene;
wherein the phenyl rings of formula I may be, independently of one another, substituted by one or more substituents selected from halogen, trifluoromethyl, lower alkyl, lower alkenyl, lower alkynyl, hydroxy, lower alkoxy, lower alkenyloxy, phenyl-lower alkoxy, lower alkanoyloxy, lower alkenoyloxy and phenyl-lower alkanoyloxy;
wherein the aromatic radicals in the above definitions may be, independently of one another, substituted by one or more substituents selected from halogen, trifluoromethyl, lower alkyl, lower alkenyl, lower alkynyl, hydroxy, lower alkoxy, lower alkenyloxy, phenyl-lower alkoxy, lower alkanoyloxy, lower alkenoyloxy and phenyl-lower alkanoyloxy.

The invention especially relates to compounds of formula I and pharmaceutically acceptable salts thereof, in which:
R₁ is amino which is mono- or disubstituted by a substituent selected from C₁-C₇-alkyl, phenyl-C₁-C₇-alkyl, phenyl and C₃-C₆-cycloalkyl, C₃-C₆-cycloalkyl being unsubstituted or mono- or polysubstituted by C₁-C₇-alkyl, or is amino which is disubstituted by C₃-C₆-alkylene;
R₂ is hydrogen, C₁-C₇-alkoxy or phenyl-C₁-C₄-alkoxy;
R₃ is hydrogen, C₁-C₁₂-alkoxycarbonyl, C₂-C₅-alkanoyl, phenyl-C₂-C₅-alkanoyl, benzoyl which is unsubstituted or substituted by halogen, trifluoromethyl, C₁-C₇-alkyl, or C₁-C₇-alkoxy, or is C₃-C₆-cycloalkylcarbonyl which is unsubstituted or substituted by C₁-C₇-alkyl, or is naphthoyl, indanoyl or fluorenoyl, or is C₁-C₇-alkanesulfonyl, phenyl-C₁-C₇-alkanesulfonyl, C₃-C₇-cycloalkanesulfonyl, or phenylsulfonyl, or is aminocarbonyl which is substituted by C₁-C₇-alkyl, phenyl-C₁-C₇-alkyl or phenyl; X₁ and X₃ each are -O-, or furthermore are, independently of one another, -O- or -S-; X₂ is C₂-C₇-alkylene or C₂-C₄-alkylene-phenylene-C₂-C₄-alkylene;
wherein the phenyl rings of formula I may be unsubstituted or, furthermore, independently of one another, substituted by one or more substituents selected from halogen, trifluoromethyl, C₁-C₇-alkyl, and C₁-C₇-alkoxy;
wherein phenyl in the above definitions is unsubstituted or, furthermore, independently of one another, substituted by one or more substituents selected from halogen, trifluoromethyl, C₁-C₇-alkyl, and C₁-C₇-alkoxy.

The invention especially relates to compounds of formula I and pharmaceutically acceptable salts thereof, in which -CO-R₁ is located in position 4 (para) or 3 or 5 (meta) of the corresponding phenyl ring with respect to -X₁-; R₂- is located in position 2 (ortho) of the corresponding phenyl ring with respect to -X₁-; and -C(=NH)-NH-R₃ is located in position 4 (para) of the corresponding phenyl ring with respect to -X₃-.

The invention especially relates to compounds of formula IA 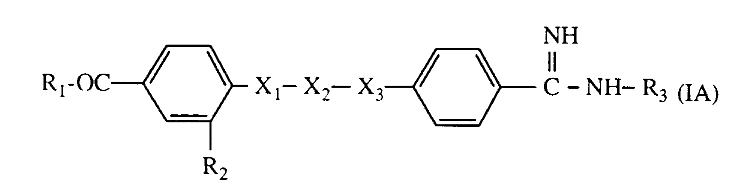
and pharmaceutically acceptable salts thereof, in which:
R₁ is di-C₁-C₄-alkylamino, such as di-ethylamino or di-isopropylamino, C₁-C₄-alkyl-phenyl-C₁-C₄-alkyl-amino, such as methyl-benzyl-amino, di-C₃-C₆-cycloalkylamino, such as di-cyclohexylamino which is unsubstituted or substituted by C₁-C₄-alkyl, 1-piperidino substituted by C₁-C₄-alkyl, such as 2-methyl-1-piperidino;
R₂ is hydrogen or C₁-C₄-alkoxy, such as methoxy;
R₃ is hydrogen, C₁-C₁₂-alkoxycarbonyl, such as methoxycarbonyl or octyloxycarbonyl, phenyl-C₁-C₄-alkoxycarbonyl, such as benzyloxycarbonyl, C₂-C₅-alkanoyl, such as acetyl, benzoyl which is unsubstituted or substituted by halogen, trifluoromethyl, C₁-C₄-alkyl, or C₁-C₄-alkoxy, such as 3,4-dimethoxybenzoyl, C₃-C₆-cycloalkylcarbonyl which is unsubstituted or substituted by C₁-C₄-alkyl, such as 2-isopropyl-5-methyl-cyclohexylcarbonyl;
X₁ and X₃ are -O-;
X₂ is C₄-C₇-alkylene, such as pentylene;
wherein the phenyl rings of formula IA may be unsubstituted or, furthermore, independently of one another, substituted by one or more substituents selected from halogen, trifluoromethyl, C₁-C₄-alkyl, and C₁-C₄-alkoxy.

The invention especially relates to compounds of formula IA and pharmaceutically acceptable salts thereof, in which:
R₁ is di-C₁-C₄-alkylamino, such as di-ethylamino or di-isopropylamino, C₁-C₄-alkyl-phenyl-C₁-C₄-alkylamino, such as methyl-benzyl-amino, di-C₃-C₆-cycloalkylamino, such as di-cyclohexylamino which is unsubstituted or substituted by C₁-C₄-alkyl, 1-piperidino substituted by C₁-C₄-alkyl, such as 2-methyl-1-piperidino;
R₂ is hydrogen or C₁-C₄-alkoxy, such as methoxy;
R₃ is C₁-C₄-alkanesulfonyl, such as methane-, ethane- or isopropanesulfonyl, phenyl-C₁-C₄-alkanesulfonyl, such as benzylsulfonyl, C₃-C₇-cycloalkane-sulfonyl, such as cyclohexanesulfonyl, or phenylsulfonyl, or is aminocarbonyl which is substituted by C₁-C₄-alkyl, phenyl-C₁-C₄-alkyl or phenyl;
X₁ and X₃ are -O-;
X₂ is C₄-C₇-alkylene, such as pentylene.

The invention especially relates to compounds of formula IA and pharmaceutically acceptable salts thereof, in which:
R₁ is di-C₁-C₄-alkylamino, such as di-ethylamino or di-isopropylamino, C₁-C₄-alkyl-phenyl-C₁-C₄-alkyl-amino, such as methyl-benzyl-amino, di-C₃-C₆-cycloalkylamino, such as di-cyclohexylamino which is unsubstituted or substituted by C₁-C₄-alkyl, 1-piperidino substituted by C₁-C₄-alkyl, such as 2-methyl-1-piperidino;
R₂ is hydrogen or C₁-C₄-alkoxy, such as methoxy;
R₃ is hydrogen;
X₁ and X₃ are -O-;
X₂ is C₄-C₇-alkylene, such as pentylene.

The invention further especially relates to compounds of formula IA and pharmaceutically acceptable salts thereof, in which:
R₁ is di-C₁-C₄-alkylamino, such as di-isopropylamino;
R₂ is hydrogen or C₁-C₄-alkoxy, such as methoxy;
R₃ is C₁-C₁₂-alkoxycarbonyl, such as methoxycarbonyl or octyloxycarbonyl, phenyl-C₁-C₄-alkoxycarbonyl, such as benzyloxycarbonyl, C₂-C₅-alkanoyl, such as acetyl, benzoyl which is unsubstituted or substituted by halogen, trifluoromethyl, C₁-C₄-alkyl, or C₁-C₄-alkoxy, such as 3,4-dimethoxybenzoyl, C₃-C₆-cycloalkylcarbonyl which is unsubstituted or substituted by C₁-C₄-alkyl, such as 2-isopropyl-5-methyl-cyclohexylcarbonyl;
X₁ and X₃ are -O-;
X₂ is C₄-C₇-alkylene, especially pentylene.

The invention further especially relates to compounds of formula IA and pharmaceutically acceptable salts thereof, in which:
R₁ is di-C₁-C₄-alkylamino, such as di-ethylamino or di-isopropylamino;
R₂ is hydrogen or C₁-C₄-alkoxy, such as methoxy;
R₃ is hydrogen;
X₁ and X₃ are -O-;
X₂ is C₄-C₇-alkylene, especially pentylene.

The invention relates in particular to the novel compounds itemized in the examples and to the manners of preparation described therein.

The invention further relates to methods for the preparation of the compounds according to the invention. The preparation of compounds of the formula I is, for example, characterized in that,
a) a compound of the formula IIa 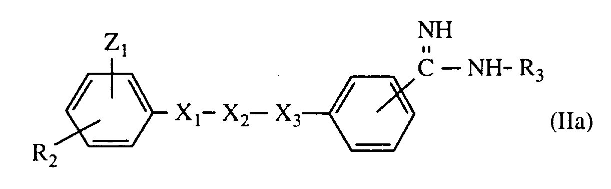 or a salt thereof in which Z₁ is a radical which can be converted into the variable -CO-R₁, Z₁ is converted into the variable -CO-R₁, or,
b) for the manufacture of compounds of the formula I in which R₃ is hydrogen, in a compound of the formula IIIa 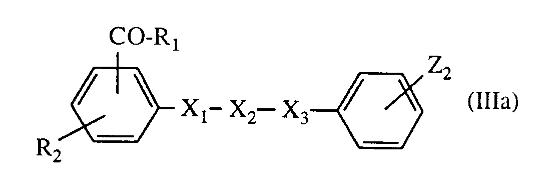 or a salt thereof in which Z₂ is a radical which can be converted into the variable -C(=NH)-NH-R₃, Z₂ is converted into the variable -C(=NH)-NH-R₃, or
c) a compound of the formula IVa 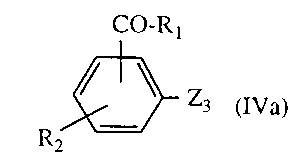 or a salt thereof is reacted with a compound of the formula IVb 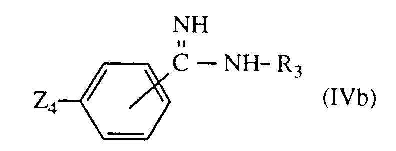 or a salt thereof in which Z₃ is a group of the formula -X₁-X₂-Z₅ and Z₄ is -Z₆, or Z₃ is -Z₆ and Z₄ is a group of the formula Z₅-X₂-X₃-, wherein one of the radicals Z₅ and Z₆ is hydroxy or mercapto and the other is hydroxy, mercapto or reactive esterified hydroxy,
   and, if desired, a compound of the formula I or a salt thereof obtainable according to the process or in another manner is converted into another compound or a salt thereof according to the invention, a free compound of the formula I obtainable according to the process is converted into a salt, a salt obtainable according to the process is converted into the free compound of the formula I or into another salt, or a mixture of isomers obtainable according to the process is resolved and the desired compound is isolated.

Salts of starting materials which contain at least one basic centre, for example of the formula IIa, are appropriate acid addition salts, while salts of starting materials which contain an acid group are present as salts with bases.

A radical Z₁ which can be converted into the variable -CO-R₁ is, for example, cyano, carboxy or a salt or activated carboxy.

A radical Z₂ which can be converted into the variable -C(=NH)-NH-R₃ is, for example, (lower) alkoxy-iminocarbonyl or halogeno-iminocarbonyl [Halogeno-C(=NH)-].

Reactive esterified hydroxy (e.g. Z₅ or Z₆) is, inparticular, hydroxy esterified with a strong inorganic acid or organic sulfonic acid, and is, for example, halogen, such as chlorine, bromine or iodine, sulfonyloxy, such as hydroxysulfonyloxy, halosulfonyloxy, such as, fluorosulfonyloxy, (C₁-C₇-)alkanesulfonyloxy which, if desired, is substituted, for example, by halogen, such as, methane- or trifluoromethanesulfonyloxy, (C₅-C₇-)cycloalkanesulfonyloxy, such as, cyclohexanesulfonyloxy, or benzenesulfonyloxy which, if desired, is substituted, for example by (C₁-C₇-)alkyl or halogen, such as, p-bromobenzene- or p-toluenesulfonyloxy.

The reactions described in the variants above and below are carried out in a manner known per se, for example in the absence or in the customary manner in the presence of a suitable solvent or diluent or a mixture thereof, the reaction being carried out, according to need, with cooling, at room temperature or with warming, for example in a temperature range from about -80°C up to the boiling point of the reaction medium, preferably from about -10°C to about +180°C, and, if necessary, in a closed vessel, under pressure, in an inert gas atmosphere and/or under anhydrous conditions.

### Process a):

A compound of the formula IIa in which Z₁ is activated carboxy (Z₁) is, for example, an anhydride thereof, including a mixed anhydride, such as an acid halide, for example chloride,
or an anhydride with a formic ester, an activated carboxylic ester such as cyanomethyl, (4-)nitrophenyl, polyhalogenophenyl, for example pentachlorophenyl, esters.

The reaction is, for example, carried out with an agent suitable to introduce R₃, for example, an amine of the formula H-R₁. The reaction with compounds of the formula IIa in which Z₁ carboxy or a salt thereof, for example, takes place under water-eliminating conditions, for example, with azeotropic removal of the water of reaction, or by treatment with a suitable condensing agent, for example, N,N'-dicyclohexyl-carbodiimide. The reaction with an activated carboxy derivative may advantageously be carried out in the presence of a base.

Suitable bases are, for example, alkali metal hydroxides, hydrides, amides, alkanolates, carbonates, triphenylmethylides, di(lower alkyl)amides, aminoalkylamides or lower alkyl silylamides, or naphthaleneamines, lower alkylamines, basic heterocycles, ammonium hydroxides, and also carbocyclic amines. Examples which may be mentioned are sodium hydroxide, sodium hydride, sodium amide, sodium (m)ethoxide, potassium tert-butoxide, potassium carbonate, lithium triphenylmethylide, lithium diisopropylamide, potassium 3-(aminopropyl)amide, potassiumbis(trimethylsilyl)amide, dimethylaminonaphthalene, di- or triethylamine, or ethyldiisopropylamine, N-methylpiperidine, pyridine, benzyltrimethylammonium hydroxide, 1,5-diazabicyclo[4.3.0]non-5-ene (DBN) and 1,8-diazabicyclo[5.4.0]undec-7-ene (DBU).

Preferably, those compounds of the formula IIa are employed in which Z₁ is activated carboxy. The corresponding reaction is carried out using an amine of the formula H-R₁. Preferred compounds of the formula IIa are corresponding acid halides such as acid chlorides or bromides derivatives thereof.

### Process b):

Alkoxy-iminocarbonyl is, for example C₁-C₄-alkoxy-iminocarbonyl such as methoxy- or ethoxy-iminocarbonyl, whereas halogeno-iminocarbonyl is, for example chloro-iminocarbonyl.

Preferably, those compounds of the formula IIIa are employed in which Z₂ is C₁-C₄-alkoxy-iminocarbonyl such as methoxy- or ethoxy-iminocarbonyl. The reaction is preferably carried out by reacting with ammonia and using an acid resulting in the corresponding acid addition salt of the product. As acids are used, for example, inorganic acids, such as mineral acids, for example sulfuric acid, a phosphoric or hydrohalic acid, or with organic carboxylic acids, such as (C₁-C₄)-alkanecarboxylic acids which, for example, are unsubstituted or substituted by halogen, for example acetic acid, such as saturated or unsaturated dicarboxylic acids, for example oxalic, malonic, succinic, maleic, fumaric, phthalic or terephthalic acid, such as hydroxycarboxylic acids, for example ascorbic, glycolic, lactic, malic, tartaric or citric acid, such as amino acids, for example aspartic or glutamic acid, benzoic acid or with organic sulfonic acids, such as (C₁-C₄)-alkane- or arylsulfonic acids which are unsubstituted or substituted, for example, by halogen, for example methane- or toluenesulfonic acid. Preferred acids are hydrohalic acids, especially hydrochloric acid, organic sulfonic acids, especially methanesulfonic acid, or dicarboxylic acids, especially maleic acid.

### Process c):

Preferably, those compounds of the formulae IVa and IVb are employed in which Z₃ is a group of the formula -X₁-X₂-Z₅ , wherein Z₅ is halogen, especially bromine, and Z₄ is hydroxy.

The reaction is carried out preferably in the presence of a base e.g. as mentioned above, such as cesium carbonate.

The starting material can be prepared following methods known per se.

In order to prepare the starting material of the formula IIa, for example, a compound of the formula 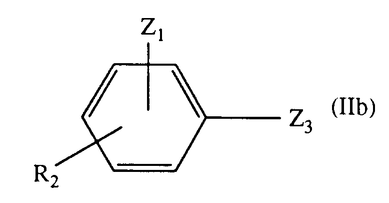
in which Z₃ preferably is a group of the formula -X₁-X₂-Z₅, wherein Z₅ preferably is reactive esterified hydroxy, is reacted with a compound of the formula 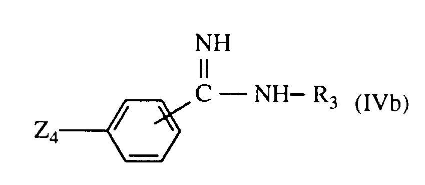
in which Z₄ is hydroxy or mercapto, following the method as described in process c). If one of variables Z₃ and Z₅ represents reactive esterified hydroxy, the other preferably represents hydroxy or mercapto.
A compound of the formula IVb can be obtained, for example, by converting Z₂ of a compound of the formula 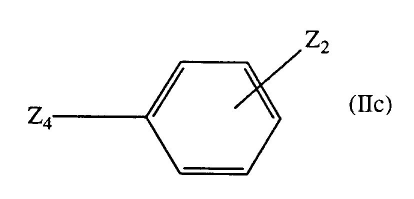
in which Z₂ is a radical which can be converted into the variable -C(=NH)-NH-R₃ following the method as described in process b). Compounds of formulae (IIb) and (IIc) are known or can be prepared according to methods known per se.

Preferably, Z₃ is a group of the formula X₁-X₂-Z₅, wherein Z₅ preferably is reactive esterified hydroxy, such as chlorine or bromine, and Z₄ is hydroxy or furthermore mercapto. A corresponding compound of the formula IIb can be obtained, for example, by reacting a compound of the formula Z₅-X₂-Z₅(IId) with a compound of the formula 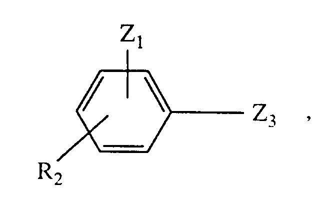
preferably in the presence of a base.

In order to prepare the starting material of the formula IIIa, Z₁ of a compound of the formula (IIb) is converted into radical -CO-R₁ treated following the method as described in process a) resulting in compound of the formula 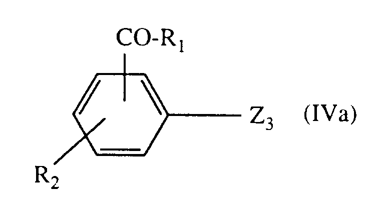
which, in the next step, is reacted with a compound of the formula (IIc) following the method as described in process c).

The invention further includes any variant of the present processes, in which an intermediate product obtainable at any stage thereof is used as starting material and the remaining steps are carried out, or the process is discontinued at any stage thereof, or in which the starting materials are formed under the reaction conditions, or in which the reaction components are used in the form of their salts or optically pure antipodes.

A compound according to the invention which is obtainable by the process can be converted into another compound according to the invention in a manner known per se.

If one of the variables contains mono-substituted amino (for example R₁), corresponding compounds of the formula I or salts thereof can be N-alkylated in a manner known per se; likewise, N-mono-substituted carbamoyl (for example R₁) can further be N-alkylated. The (aryl-)alkylation is carried out, for example, using a reactive ester of an (aryl-)C₁-C₇-alkyl halide, for example a bromide or iodide, an (aryl-)C₁-C₇-alkylsulfonate, for example a methanesulfonate or p-toluenesulfonate, or a di-C₁-C₇-alkyl sulfate, for example dimethyl sulfate, preferably under basic conditions, such as in the presence of sodium hydroxide solution or potassium hydroxide solution, and advantageously in the presence of a phase-transfer catalyst, such as tetrabutylammonium bromide or benzyltrimethylammonium chloride, where, however, stronger basic condensing agents, such as alkali metal amides, hydrides or alkoxides, for example sodium amide, sodium hydride or sodium ethoxide, may be necessary.

If R₃ is hydrogen, the corresponding amidino group can be N-acylated accordingly. The acylation is carried out in a manner known per se using a suitable acylating agent. An example of a suitable acylating agent is a compound of the formula Ac-Z₇ (IIIa), where Ac denotes an acyl radical corresponding to the variable R₃, and Z₇ denotes in particular reactive activated hydroxyl. Appropriate hydroxyl can be activated, for example, by strong acids such as hydrohalic or carboxylic acid, for example by hydrochloric, hydrobromic acid, an optionally substituted, for example by halogen, alkanecarboxylic acid or by an acid of the formula Ac-OH, or by suitable activating or coupling reagents of the type detailed hereinafter, in particular in situ. Ac-Z₇ can furthermore represent an activated ester, where Z₇ denotes, in particular, cyanomethoxy, (4-)nitrophenoxy or polyhalogeno-, such as pentachloro-, -phenoxy. Activating and coupling reagents which can be employed are, in particular, carbodiimides, for example N,N'-di-C₁-C₄-alkyl- or N,N'-di-C₅-C₇-cycloalkyl-carbodiimide, such as diisopropylcarbodiimide or N,N'-dicyclohexylcarbodiimide, advantageously with the addition of an activating catalyst such as N-hydroxysuccinimide or optionally substituted, for example by halogen, C₁-C₇-alkyl or C₁-C₇-alkoxy, N-hydroxy-benzotriazole or N-hydroxy-5-norbornene-2,3-dicarboxamide, furthermore C₁-C₄-alkyl halogenoformate, for example isobutyl chloroformate, suitable carbonyl compounds, for example N,N-carbonyldiimidazole, suitable 1,2-oxazolium compounds, for example 2-ethyl-5-phenyl-1,2-oxazolium 3'-sulfonate or 2-tert-butyl-5-methyl-isoxazolium perchlorate, suitable acylamino compounds, for example 2-ethoxy-1-ethoxycarbonyl-1,2-dihydroquinoline, or suitable phosphoryl cyanamides or azides, for example diethylphosphoryl cyanamide or diphenylphosphoryl azide, furthermore triphenylphosphine disulfide or 1-C₁-C₄-alkyl-2-halogeno-pyridinium halides, for example 1-methyl-2-chloropyridinium iodide. Z₇ preferably denotes halogen such as chlorine or bromine, and Ac-O-.

If the compounds of the formula (I) or (IA) contain unsaturated radicals, such as (lower)alkenyl groups, these can be converted into saturated radicals in a manner known per se. Thus, for example, multiple bonds are hydrogenated by catalytic hydrogenation in the presence of hydrogenation catalysts, suitable for this purpose being, for example, nickel, such as Raney nickel, and noble metals or their derivatives, for example oxides, such as palladium or platinum oxide, which may be applied, if desired, to support materials, for example to carbon or calcium carbonate. The hydrogenation may preferably carried out at pressures between 1 and about 100 at and at room temperature between about -80° to about 200°C, in particular between room temperature and about 100°C. The reaction is advantageously carried out in a solvent, such as water, a lower alkanol, for example ethanol, isopropanol or n-butanol, an ether, for example dioxane, or a lower alkanecarboxylic acid, for example acetic acid.

The invention also relates to any novel starting materials and processes for their manufacture and their use.

Depending on the choice of starting materials and methods, the new compounds may be in the form of one of the possible isomers or mixtures thereof, for example, as substantially pure geometric (cis or trans) isomers, optical isomers (antipodes), racemates, or mixtures thereof. The aforesaid possible isomers or mixtures thereof are within the purview of this invention.

Any resulting mixtures of isomers can be separated on the basis of the physico-chemical differences of the constituents, into the pure geometric or optical isomers, diastereoisomers, racemates, for example by chromatography and/or fractional crystallization.

Any resulting racemates of final products or intermediates can be resolved into the optical antipodes by known methods, e.g. by separation of the diastereoisomeric salts thereof, obtained with an optically active acid or base, and liberating the optically active acidic or basic compound. The amidine (wherein R₃ represents hydrogen) can thus be resolved into their optical antipodes e.g. by fractional crystallization of a salt formed with an optically active acid.

Finally, the compounds of the invention are either obtained in the free form, or as a salt thereof.

In view of the close relationship between the free compounds and the compounds in the form of their salts, whenever a compound is referred to in this context, a corresponding salt is also intended, provided such is possible or appropriate under the circumstances.

The compounds, including their salts, can also be obtained in the form of their hydrates, or include other solvents used for their crystallization.

The pharmaceutical compositions according to the invention are those suitable for enteral, such as oral or rectal, transdermal and parenteral administration to mammals, including man, to antagonize LTB₄ receptors, and for the treatment of a condition or syndrome responsive to the selective antagonism of LTB₄ receptors, comprising an effective amount of a pharmacologically active compound of the invention, alone or in combination, with one or more pharmaceutically acceptable carriers.

The novel pharmaceutical products contain, for example, from about 10 % to about 80 %, preferably from about 20 % to about 60 %, of the active compound. Examples of pharmaceutical products according to the invention for enteral or parenteral administration are those in dose-unit forms such as coated tablets, tablets, capsules or suppositories, as well as ampoules. These are prepared in a manner known per se, for example using conventional mixing, granulating, coating, dissolving or freeze-drying processes. Thus, pharmaceutical products for oral use can be obtained by combining the active compound with solid excipients, where appropriate granulating a mixture which is obtained, and processing the mixture or granules, if desired or necessary, after addition of suitable auxiliaries to tablets or cores of coated tablets.

The pharmacologically active compounds of the invention are useful in the manufacture of pharmaceutical compositions comprising an effective amount thereof in conjunction or admixture with excipients or carriers suitable for either enteral or parenteral application. Preferred are tablets and gelatin capsules comprising the active ingredient together with a) diluents, e.g. lactose, dextrose, sucrose, mannitol, sorbitol, cellulose and/or glycine; b) lubricants, e.g. silica, talcum, stearic acid, its magnesium or calcium salt and/or polyethyleneglycol; for tablets also c) binders e.g. magnesium aluminum silicate, starch paste, gelatin, tragacanth, methylcellulose, sodium carboxymethylcellulose and or polyvinylpyrrolidone; if desired d) disintegrants, e.g. starches, agar, alginic acid or its sodium salt, or effervescent mixtures; and/or e) absorbants, colorants, flavors and sweeteners. Injectable compositions are preferably aqueous isotonic solutions or suspensions, and suppositories are advantageously prepared from fatty emulsions or suspensions. Said compositions may be sterilized and/or contain adjuvants, such as preserving, stabilizing, wetting or emulsifying agents, solution promoters, salts for regulating the osmotic pressure and/or buffers. Cores of coated tablets are provided with suitable, optionally enteric, coatings, using, inter alia, concentrated sugar solutions which optionally contain gum arabic, talc, polyvinylpyrrolidone, polyethylene glycol and/or titanium dioxide, lacquer solutions in suitable organic solvents or solvent mixtures or, for the preparation of enteric coatings, solutions of suitable cellulose products such as acetyl cellulose phthalate or hydroxypropylmethylcellulose phthalate. Colorants or pigments can be added to the tablets or coatings of coated tablets, for example, to identify or to indicate various doses of active compound. In addition, they may also contain other therapeutically valuable substances. Said compositions are prepared according to conventional mixing, granulating or coating methods, respectively, and contain about 0.1 to 75 %, preferably about 1 to 50 %, of the active ingredient.

Suitable formulations for transdermal application include an effective amount of a compound of the invention with carrier. Advantageous carriers include absorbable pharmacologically acceptable solvents to assist passage through the skin of the host.

Characteristically, transdermal devices are in the form of a bandage comprising a backing member, a reservoir containing the compound optionally with carriers, optionally a rate controlling barrier to deliver the compound of the skin of the host at a controlled and predetermined rate over a prolonged period of time, and means to secure the device to the skin.

In conjunction with another active ingredient, a compound of the invention may be administered either simultaneously, before or after the other active ingredient, either separately by the same or different route of administration or together in the same pharmaceutical formulation.

The pharmaceutical compositions according to claim 1 can be used for example in cases of rheumatoid arthritis, inflammatory bowel disease, psoriasis, non-steroidal-antiinflammatory-drug-induced gastropathy, adult respiratory distress syndrome (ARDS), myocardial infarction, allergic rhinitis, hemadialysis-induced neutropenia, and late phase asthma.

The dosage of active compound administered is dependent on the species of warm-blooded animal (mammal), the body weight, age and individual condition, and on the form of administration. A unit dosage for oral administration to a mammal of about 70 kg may contain e.g. between about 1 and about 1000 mg/kg per day of the active ingredient.

The following examples are intended to illustrate the invention and are not to be construed as being limitations thereon. Temperatures are given in degrees Centrigrade. If not mentioned otherwise, all evaporations are performed under reduced pressure, preferably between about 15 and 100 mm Hg. The structure of final products, intermediates and starting materials is confirmed by standard analytical methods, e.g. microanalysis and spectroscopic characteristics (e.g. MS, IR, NMR). Abbreviations used are those conventional in the art.

Example 1: A stirred, 0 °C solution of ethyl 4-[5-[2-methoxy-4-[N,N-bis(1-methylethyl)aminocarbonyl]phenoxy]pentoxy]benzenecarboximidoate (420 mg 0.87 mmol) in 20 mL anhydrous ethanol is treated with anhydrous hydrogen chloride gas for 5 minutes. After warming to room temperature, the resulting solution is concentrated in vacuo, redissolved in chloroform (20 mL) and reconcentrated. The resulting hydrochloride salt is then dissolved in ethanol (40 mL), transferred to a pressure tube and treated with anhydrous ammonia for 10 minutes at 0 °C. The pressure tube is sealed and heated to 100 °C for 45 minutes. Upon cooling and concentrating in vacuo, the resulting material is purified by chromatography on silica gel (15 g) with 3-20% methanol/dichloromethane as the eluent to afford 4-[5-[4-(aminoiminomethyl)phenoxy]pentoxy]-3-methoxy-N,N-bis(1-methylethyl)benzamide monohydrochloride as a colorless foam:

| CHN calculated for C₂₆H₃₈N₃O₄Cl-1.0 H₂O; | | | |
|---|---|---|---|
| Theory: | % C: 61.22; | % H: 7.91; | % N: 8.24; |
| Found: | % C: 61.48; | % H: 7.57; | % N: 8.22. |

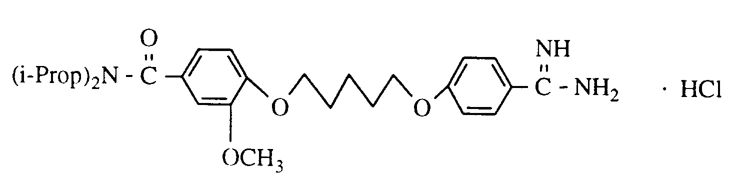

(i-Prop = isopropyl)
The starting material, ethyl 4-[5-(2-methoxy-4-[N,N-bis(1-methylethyl)aminocarbonyl]phenoxy]pentoxy]benzenecarboximidoate, can be prepared, for example, as follows:

A stirred, 0 °C solution of 4-hydroxybenzonitrile (50.7 g, 426 mmol) in 1400 mL of dichloromethane and 75 mL anhydrous ethanol is treated with anhydrous hydrogen chloride gas (110 g) over 1.5 hours. This solution is stirred a room temperature for 64 hours and the resulting solids collected and washed with 500 mL diethyl ether and 2 x 1000 mL of ethyl acetate. The remaining solids (60.4 g) were dissolved in 1200 mL of water and the residual solids filtered. To the filtrate is added a solution of sodium hydroxide (12.57 g) in 150 mL water. The resulting white solid is filtered and washed with water to afford ethyl 4-hydroxybenzenecarboximidoate.

A stirred solution of ethyl 4-hydroxybenzenecarboximidoate (32.0 g, 194 mmol) in 250 mL anhydrous N,N-dimethylformamide is treated with cesium carbonate (75.7 g, 232 mmol) and 1,5-dibromopentane (89.1 g, 387 mmol) and heated at 70 °C for 1.5 hours. After cooling to room temperature, the reaction is filtered and the resulting filtrate concentrated in vacuo (< 1 mtorr) to afford a yellow oil (85.7 g). This material is purified by chromatography on silica gel (850 g) with 10-60% ethyl acetate/hexanes as the eluent to afford ethyl 4-[5-bromopentoxy]benzenecarboximidoate as a colorless oil.

A stirred solution of 4-hydroxy-3-methoxy-N,N-bis(1-methylethyl)benzenecarboxamide (319 mg, 1.27 mmol) in 3.5 mL anhydrous N,N-dimethylformamide is treated with cesium carbonate (435 mg, 1.33 mmol) and ethyl 4-[5-bromopentoxy]benzenecarboximidoate (400 mg, 1.27 mmol) and heated at 70 °C for 1.0 hours. After cooling to room temperature, the reaction is partitioned between ethyl acetate and water and the organics washed with brine, dried over sodium sulfate and concentrated in vacuo to afford a yellow oil. This material is purified by chromatography on silica gel (22 g) with 30-75% ethyl acetate/hexanes as the eluent to afford ethyl 4-[5-[2-methoxy-4-[N,N-bis(1-methylethyl)aminocarbonyl]phenoxy]pentoxy]benzenecarboximidoate as a colorless oil.

Example 2: In a way analogously as described in example 1, the following compounds can be manufactured:
4-[5-[4-(aminoiminomethyl)phenoxy]pentoxy]-3-methoxy-N,N-diethylbenzamide monohydrochloride is obtained from ethyl 4-[5-[2-methoxy-4-[N,N-diethylaminocarbonyl)phenoxy]pentoxy]benzenecarboximidoate (1.25 g) as colorless foam

| CHN calculated for C₂₄H₃₄N₃O₄Cl-0.5 H₂O; | | | |
|---|---|---|---|
| Theory: | % C: 60.94; | % H: 7.46; | % N: 8.88; |
| Found: | % C: 61.10; | % H: 7.47; | % N: 8.99. |

4-[5-[4-(aminoiminomethyl)phenoxy]pentoxy]-3-methoxy-N-methylbenzamide monohydrochloride is obtained from ethyl 4-[5-[2-methoxy-4-[N-methylaminocarbonyl]phenoxy]pentoxy]benzenecarboximidoate (475 mg) as white crystals, mp = 105-108 °C.

1-[4-[5-[4-(aminoiminomethyl)phenoxy]pentoxy]-3-methoxybenzoyl] piperidine monohydrochloride is obtained from ethyl 4-[5-[2-methoxy-4-(1-piperidinylcarbonyl)phenoxy]pentoxy]benzenecarboximidoate (390 mg) as a colorless foam:

| CHN calculated for C₂₅H₃₄N₃O₄Cl; | | | |
|---|---|---|---|
| Theory: | % C: 63.08; | % H: 7.20; | % N: 8.83; |
| Found: | % C: 63.05; | % H: 6.97; | % N: 8.54. |

4-[5-[4-(aminoiminomethyl)phenoxy]pentoxy]-3-methoxy-N-(phenylmethyl)benzamide monohydrochloride is obtained from ethyl 4-[5-[2-methoxy-4-[N-(phenylmethyl)aminocarbonyl]phenoxy]pentoxy]benzenecarboximidoate (412 mg) as a colorless foam:

| CHN calculated for C₂₇H₃₂N₃O₄Cl-0.5 H₂O; | | | |
|---|---|---|---|
| Theory: | % C: 63.95; | % H: 6.56; | % N: 8.29; |
| Found: | % C: 64.11; | % H: 6.80; | % N: 8.29. |

4-[5-[4-(aminoiminomethyl)phenoxy]pentoxy]-3-methoxy-N,N-di-n-propylbenzamide monohydrochloride is obtained from ethyl 4-[5-[2-methoxy-4-[N,N-dipropylaminocarbonyl]phenoxy]pentoxy]benzenecarboximidoate (1.30 g) as a colorless foam:

| CHN calculated for C₂₆H₃₆N₃O₄Cl-0.5 H₂O; | | | |
|---|---|---|---|
| Theory: | % C: 62.45; | % H: 7.65; | % N: 8.40; |
| Found: | % C: 62.56; | % H: 7.78; | % N: 8.66. |

4-[5-[4-(aminoiminomethyl)phenoxy]pentoxy]-3-methoxy-N,N-dimethylbenzamide monohydrochloride is obtained from ethyl 4-[5-[2-methoxy-4-[N,N-dimethylaminocarbonyl]phenoxy]pentoxy]benzenecarboximidoate (474 mg) as a colorless foam:

| CHN calculated for C₂₂H₃₀N₃O₄Cl-1.0 H₂O; | | | |
|---|---|---|---|
| Theory: | % C: 58.21; | % H: 7.11; | % N: 9.25; |
| Found: | % C: 58.35; | % H: 6.82; | % N: 9.64. |

4-[5-[4-(aminoiminomethyl)phenoxy]pentoxy]-3-methoxy-N-methyl-N-(phenylmethyl)benzamide monohydrochloride is obtained from ethyl 4-[5-[2-methoxy-4-[N-methyl-N-(phenylmethyl)aminocarbonyl]phenoxy]pentoxy]benzenecarboximidoate (550 mg) as a colorless foam:

| CHN calculated for C₂₈H₃₄N₃O₄Cl-1.0 H₂O; | | | |
|---|---|---|---|
| Theory: | % C: 63.45; | % H: 6.85; | % N: 7.93; |
| Found: | % C: 63.74; | % H: 6.61; | % N: 7.96. |

4-[5-[4-(aminoiminomethyl)phenoxy]pentoxy]-3-methoxy-N-ethylbenzamide monohydrochloride is obtained from ethyl 4-[5-[2-methoxy-4-[N-ethylaminocarbonyl]phenoxy]pentoxy]benzenecarboximidoate (490 mg) as a colorless foam:

| CHN calculated for C₂₂H₃₀N₃O₄Cl-0.5 H₂O; | | | |
|---|---|---|---|
| Theory: | % C: 59.39; | % H: 7.02; | % N: 9.44; |
| Found: | % C: 59.58; | % H: 6.92; | % N: 9.46. |

4-[5-[4-(aminoiminomethyl)phenoxy]pentoxy]-3-methoxy-N-(1,1-dimethylethyl)benzamide monohydrochloride is obtained from ethyl 4-[5-[2-methoxy-4-N-(1,1-dimethylethyl)aminocarbonyl]phenoxy]pentoxy]benzenecarboximidoate (530 mg) as a colorless foam:

| CHN calculated for C₂₄H₃₄N₃O₄Cl-0.5 H₂O; | | | |
|---|---|---|---|
| Theory: | % C: 60.94; | % H: 7.46; | % N: 8.88; |
| Found: | % C: 60.90; | % H: 7.35; | % N: 8.84. |

4-[5-[4-(aminoiminomethyl)phenoxy]pentoxy]-3-methoxy-N-n-propylbenzamide monohydrochloride is obtained from ethyl 4-[5-[2-methoxy-4-[N-n-propylaminocarbonyl]phenoxy]pentoxy]benzenecarboximidoate (538 mg) as a colorless foam:

| CHN calculated for C₂₃H₃₂N₃O₄Cl-0.5 H₂O; | | | |
|---|---|---|---|
| Theory: | % C: 60.19; | % H: 7.25; | % N: 9.15; |
| Found: | % C: 60.27; | % H: 7.14; | % N: 9.09. |

1-[4-[5-[4-(aminoiminomethyl)phenoxy]pentoxy]-3-methoxybenzoyl]-2-methylpiperidine monohydrochloride is obtained from ethyl 4-[5-[2-methoxy-4-(2-methyl-1-piperidinylcarbonyl)phenoxy]pentoxy]benzenecarboximidoate (573 mg) as a colorless foam:

| CHN calculated for C₂₆H₃₆N₃O₄Cl-0.5 H₂O; | | | |
|---|---|---|---|
| Theory: | % C: 62.57; | % H: 7.47; | % N: 8.42; |
| Found: | % C: 62.55; | % H: 7.17; | % N: 8.32. |

4-[5-[4-(aminoiminomethyl)phenoxy]pentoxy]-3-methoxybenzoyl]morpholine monohydrochloride is obtained from ethyl 4-[5-[2-methoxy-4-[1-morpholino-carbonyl]phenoxy]pentoxy]benzenecarboximidoate as a colorless foam:

| CHN calculated for C₂₄H₃₂N₃O₅Cl-0.75 H₂O; | | | |
|---|---|---|---|
| Theory: | % C: 58.64; | % H: 6.87; | % N: 8.55; |
| Found: | % C: 58.61; | % H: 6.60; | % N: 8.58. |

1-[4-[5-[4-(aminoiminomethyl)phenoxy]pentoxy]-3-methoxybenzoyl]-N,N-diethyl-3-piperidinecarboxamide monohydrochloride is obtained from 1-[4-[5-[4-ethoxyiminomethyl)phenoxy]pentoxy]-2-methoxybenzoyl]-N,N-diethyl-3-piperidine carboxamide (1.00 g) as a colorless foam:

| CHN calculated for C₃₀H₄₂N₄O₅Cl-1.0 H₂O; | | | |
|---|---|---|---|
| Theory: | % C: 60.85; | % H: 7.49; | % N: 9.46; |
| Found: | % C: 60.86; | % H: 7.51; | % N: 9.40. |

1-[4-[5-[4-(aminoiminomethyl)phenoxy]pentoxy]-3-methoxybenzoyl]pyrrolidine monohydrochloride is obtained from ethyl 4-[5-[2-methoxy-4-(1-pyrrolidinylcarbonyl)phenoxy]pentoxy]benzenecarboximidoate (731 mg) as a colorless foam:

| CHN calculated for C₂₄H₃₄N₃O₄Cl-0.75 H₂O; | | | |
|---|---|---|---|
| Theory: | % C: 60.62; | % H: 7.10; | % N: 8.84; |
| Found: | % C: 60.31; | % H: 6.87; | % N: 8.84. |

4-[5-[4-(aminoiminomethyl)phenoxy]pentoxy]-N,N-diethylbenzamide monohydrochloride is obtained from ethyl 4-[5-[4-[N,N-diethylaminocarbonyl]phenoxy]pentoxy]benzenecarboximidoate (678 mg) as a colorless foam:

| CHN calculated for C₂₃H₃₂N₃N₃Cl-0.75 H₂O; | | | |
|---|---|---|---|
| Theory: | % C: 61.73; | % H: 7.55; | % N: 9.39; |
| Found: | % C: 61.69; | % H: 7.29; | % N: 9.29. |

3-[5-[4-(aminoiminomethyl)phenoxy]pentoxy]-N,N-diethylbenzamide monohydrochloride (327 mg) is obtained from ethyl 3-[5-[4-[N,N-diethylaminocarbonyl]phenoxy]pentoxy]benzenecarboximidoate (475 mg) as a colorless foam:

| CHN calculated for C₂₃H₃₂N₃O₃Cl-1.25 H₂O; | | | |
|---|---|---|---|
| Theory: | % C: 60.52; | % H: 7.62; | % N: 9.20; |
| Found: | % C: 60.71; | % H: 7.31; | % N: 9.14. |

2-[5-[4-(aminoiminomethyl)phenoxy]pentoxy]-N,N-diethylbenzamide monohydrochloride is obtained from ethyl 2-[5-[4-[N,N-diethylaminocarbonyl]phenoxy]pentoxy]benzenecarboximidoate (278 mg) as a colorless foam:

| CHN calculated for C₂₃H₃₂N₃O₃Cl-1.25 H₂O; | | | |
|---|---|---|---|
| Theory: | % C: 60.52; | % H:7.62; | % N: 9.20; |
| Found: | %C: 60.51; | % H: 7.18; | % N: 9.36. |

4-[5-[4-(aminoiminomethyl)phenoxy]pentoxy]-N,N-dicyclohexyl-3-methoxybenzamide monohydrochloride is obtained from ethyl 4-[5-[2-methoxy-4-[N,N-dicyclohexylaminocarbonyl]phenoxy]pentoxy]benzenecarboximidoate (663 mg) as a colorless foam:

| CHN calculated for C₃₂H₄₆N₃O₄Cl-0.75 H₂O; | | | |
|---|---|---|---|
| Theory: | % C: 65.74; | % H: 8.02; | % N: 7.19; |
| Found: | % C: 65.83; | % H: 7.93; | % N: 7.18. |

4-[5-[4-(aminoiminomethyl)phenoxy]pentoxy]-3-methoxy-N-(1-methylethyl)-N-(phenylmethyl)benzamide monohydrochloride is obtained from ethyl 4-[5-[2-methoxy-4-[N-(1-methylethyl)-N-(phenylmethyl)aminocarbonyl]phenoxy]pentoxy]benzenecarboximidoate (483 mg) as a colorless foam:

| CHN calculated for C₃₀H₃₈N₃O₄Cl-0.75 H₂O; | | | |
|---|---|---|---|
| Theory: | % C: 65.09; | % H: 7.19; | % N: 7.59; |
| Found: | % C: 65.08; | % H: 6.90; | % N: 7.64. |

4-[5-[4-(aminoiminomethyl)phenoxy]pentoxy]-3-methoxy-N-cyclohexyl-N-(1-methylethyl)benzamide monohydrochloride is obtained from ethyl 4-[5-[3-methoxy-4-[N-cyclohexyl-N-(1-methylethyl)aminocarbony l]phenoxy]pentoxy]benzenecarboximidoate (727 mg) as a colorless foam:

| CHN calculated for C₂₉H₄₂N₃O₄Cl-0.5 H₂O; | | | |
|---|---|---|---|
| Theory: | % C: 64.36; | % H: 8.01; | % N: 7.76; |
| Found: | % C: 64.44; | % H: 7.80; | % N: 7.77. |

4-[5-[4-(aminoiminomethyl)phenoxy]pentoxy]-N,N-bis(1-methylethyl)benzamide monohydrochloride is obtained from ethyl 4-[5-[4-[N,N-bis(1-methylethyl)aminocarbonyl]phenoxy]pentoxy]benzenecarboximidoate (419 mg) as a colorless foam:

| CHN calculated for C₂₅H₃₆N₃O₃Cl-1.0 H₂O; | | | |
|---|---|---|---|
| Theory: | % C: 62.55; | % H: 7.97; | % N: 8.75; |
| Found: | % C: 62.20; | % H: 7.57; | % N: 8.68. |

4-[5-[4-(aminoiminomethyl)-3-methoxyphenoxy]pentoxy]-N,N-bis(1-methylethyl)benzamide is obtained from ethyl 4-[5-[4-[N,N-bis(1-methylethyl)aminocarbonyl]phenoxy]pentoxy]-2-methoxybenzenecarboximidoate (1.00 g) as a colorless foam:

| CHN calculated for C₂₆H₃₇N₃O₄Cl; | | | |
|---|---|---|---|
| Theory: | % C: 68.54; | % H: 8.18; | % N: 9.22; |
| Found: | % C: 68.18; | % H: 7.82; | % N: 8.82. |

4-[5-[4-(Aminoiminomethyl)phenoxy]pentoxy]-N-methyl-N-phenylb enzamide monohydrochloride is obtained from ethyl 4-[5-[4-[N-methyl-N-phenylaminocarbonyl]phenoxy]pentoxy]benzenecarboximidoate as a colorless foam:

| CHN calculated for C₂₆H₃₀N₃O₃Cl- 0.75H₂O; | | | |
|---|---|---|---|
| Theory: | %C: 64.86; | % H: 6.59; | % N: 8.73 |
| Found: | %C: 65.02; | % H: 6.36; | % N: 8.80. |

4-[5-[4-(aminoiminomethyl)phenoxy]pentoxy]-N-(1-methylethyl)-N-phenylbenzamide monomethanesulfonate is obtained from ethyl 4-[5-[4-[N-(1-methylethyl)-N-phenylaminocarbonyl]phenoxy]pentoxy]benzenecarboximidoate as a colorless foam:

| CHN calculated for C₂₉H₃₇N₃SO₆; | | | |
|---|---|---|---|
| Theory: | % C: 62.68; | % H: 6.71; | % N: 7.56; |
| Found: | % C: 62.64; | % H: 6.68; | % N: 7.67. |

4-[5-[4-(aminoiminomethyl)phenoxy]pentoxy]-2-chloro-N,N-bis(1-methylethyl)benzamide monohydrochloride is obtained from ethyl 4-[5-[3-chloro-4-[N,N-bis(1-methylethyl)aminocarbonyl]phenoxy]pentoxy]benzenecarboximidoate as a colorless foam, m.p. 125-128°C.

| CHN calculated for C₂₅H₃₅N₃O₃Cl₂-0.5 H₂O; | | | |
|---|---|---|---|
| Theory: | % C: 59.57; | % H: 7.28; | % N: 8.01; |
| Found: | % C: 59.40; | % H: 7.17; | % N: 8.31. |

4-[5-[4-(aminoiminomethyl)phenoxy]pentoxy]-2-methyl-N,N-bis(1-methylethyl)benzamide monohydrochloride is obtained from ethyl 4-[5-[3-methyl-4-[N,N-bis(1-methylethyl)aminocarbonyl]phenoxy]pentoxy]benzenecarboximidoate as a colorless foam:

| CHN calculated for C₂₀H₃₇N₃O₃-1.0 H₂O; | | | |
|---|---|---|---|
| Theory: | % C: 63.20; | % H: 8.16; | % N: 8.50; |
| Found: | % C: 63.59; | % H: 8.18; | % N: 8.19. |

4-[5-[4-(aminoiminomethyl)phenoxy]pentoxy]-2-methoxy-N,N-bis(1-methylethyl)benzamide monohydrochloride is obtained from ethyl 4-[5-[3-methoxy-4-[N,N-bis(1-methylethyl)aminocarbonyl]phenoxy]pentoxy]benzenecarboximidoate as a colorless foam, m.p. 130-134°C.

| CHN calculated for C₂₆H₃₈N₃O₄Cl-0.5 H₂O; | | | |
|---|---|---|---|
| Theory: | % C: 62.32; | % H: 7.84; | % N: 8.38; |
| Found: | % C: 61.94; | % H: 7.73; | % N: 7.90. |

4-[5-[4-(aminoiminomethyl)phenoxy]pentoxy]-2,6-dichloro-N,N-bis(1-methylethyl)benzamide monohydrochloride is obtained from ethyl 4-[5-[3,5-dichloro-4-[N,N-bis(1-methylethyl)aminocarbonyl]phenoxy]pentoxy]benzenecarboximidoate as a colorless foam:

| CHN calculated for C₂₅H₃₄N₃O₃Cl₃-0.5 H₂O; | | | |
|---|---|---|---|
| Theory: | % C: 55.61; | % H: 6.53; | % N: 7.78; |
| Found: | % C: 55.81; | % H: 6.39; | % N: 7.64. |

4-[5-[4-(aminoiminomethyl)phenoxy]pentoxy]-2,6-dimethyl-N,N-bis(1-methylethyl)benzamide monohydrochloride is obtained from ethyl 4-[5-[3,5-dimethyl-4-[N,N-bis(1-methylethyl)aminocarbonyl]phenoxy]pentoxy]benzenecarboximidoate as a colorless foam:

| CHN calculated for C₂₇H₄₀N₃O₃Cl-1.0 H₂O; | | | |
|---|---|---|---|
| Theory: | % C: 63.82; | % H: 8.33; | % N: 8.26; |
| Found: | % C: 63.82; | % H: 7.82; | % N: 7.99. |

4-[5-[4-(aminoiminomethyl)phenoxy]pentoxy]-3,5-dibromo-N,N-bis(1-methylethyl)benzamide monomethanesulfonate is obtained from ethyl 4-[5-[2,6-dibromo-4-[N,N-bis(1-methylethyl)aminocarbonyl]phenoxy]pentoxy]benzenecarboximidoate as a colorless foam:

| CHN calculated for C₂₆H₃₇N₃O₆SBr₂; | | | |
|---|---|---|---|
| Theory: | % C: 45.96; | % H: 5.49; | % N: 6.18; |
| Found: | % C: 45.86; | % H: 5.53; | % N: 6.10. |

4-[5-[4(aminoiminomethyl)phenoxy]pentoxy]-2-hydroxy-N,N-bis(1-methylethyl)benzamide monohydrochloride is obtained from ethyl 4-[5-[3-hydroxy-4-[N,N-bis(1-methylethyl)aminocarbonyl]phenoxy]pentoxy]benzenecarboximidoate as a colorless foam:

| CHN calculated for C₂₆H₃₆N₃O₄Cl-1.0 H₂O; | | | |
|---|---|---|---|
| Theory: | % C: 60.53; | % H: 7.72; | % N: 8.47; |
| Found: | % C: 60.57; | % H: 7.31; | % N: 8.06. |

3-[5-[4-(aminoiminomethyl)phenoxy]pentoxy]-4-methoxy-N,N-bis(1-methylethyl)benzamide monohydrochloride, m.p. 87-90 °C.

3-[5-[4-(aminoiminomethyl)phenoxy]pentoxy]-4-methoxy-N,N-diethyl)benzamide monohydrochloride, m.p. 73-75 °C.

3-[5-[4-(aminoiminomethyl)-3-chlorophenoxy]pentoxy]-N,N-bis(1-methylethyl)benzamide monohydrochloride, m.p. 72-76 °C.

4-[5-[4-aminoiminomethyl-3-hydroxyphenoxy]-N,N-bis-(1-methylethyl)benzamide hydrochloride.

Example 3: A stirred, 0°C solution of 4-[5-[4(aminoiminomethyl)phenoxy]pentoxy]-3-methoxy-N,N-bis(1-methylethyl)benzamide monohydrochloride (1.9 g, 3.86 mmol) in 20 mL water is treated at 0°C with 20 mL of 1.0 N aqueous sodium hydroxide. The solution is extracted sequentially with four 50 mL portions of dichloromethane. The combined organic solution is washed with brine, dried over sodium sulfate and concentrated to give 4-[5-[4-(aminoiminomethyl)phenoxy]pentoxy]-3-methoxy-N,N-bis(1-methyl ethyl)benzamide (1.75 g) as a colorless foam. A portion of this material (500 mg, 1.1 mmol) is dissolved in 1 mL ethanol and treated dropwise with a solution of maleic acid (127 mg) in 1 mL ethanol. Diethyl ether (10 mL) is added slowly dropwise to induce crystallization. The crystalline product is collected by filtration, washed with ether and dried to give 4-[5-[4-(aminoiminomethyl)phenoxy]pentoxy]-3-methoxy-N,N-bis(1-methyl ethyl)benzamide (Z)-2-butenedioate (1:1) (525 mg), mp = 145-146 °C.

| CHN calculated for C₃₀H₄₁N₃O₈; | | | |
|---|---|---|---|
| Theory: | % C: 63.03; | % H: 7.23; | % N: 7.35; |
| Found: | % C: 62.97; | % H: 7.04; | % N: 7.29. |

Example 4: In a way analogously as described in example 3, the following compounds can be manufactured:
4-[5-[4-(aminoiminomethyl)phenoxy]pentoxy]-3-methoxy-N,N-bis(1-methylethyl)benzamide monomethanesulfonate is obtained from 4-[5-[4-(aminoiminomethyl)phenoxy]pentoxy]-3-methoxy-N,N-bis(1-methylethyl)benzamide (500 mg) and methanesulfonic acid, mp = 122-123 °C.

4-[5-[4-(aminoiminomethyl)phenoxy]pentoxy]-3-methoxy-N,N-bis(1-methylethyl)benzamide mono-2-hydroxyethanesulfonate is obtained from 4-[5-[4-(aminoiminomethyl)phenoxy]pentoxy]-3-methoxy-N,N-bis(1-methylethyl)benzamide (500 mg) and ammonium 2-hydroxyethanesulfonate, mp = 118-120 °C.

Example 5: A stirred solution of 4-[5-[4-(aminoiminomethyl)phenoxy]pentoxy]-3-methoxy-N,N-bis(1-methylethyl)benzamide monohydrochloride (200 mg) in 4.0 mL anhydrous methylene chloride is treated at 0°C sequentially with triethylamine (0.068 mL, 0.48 mmol) and methyl chloroformate (39 mg). The solution is stirred 30 minutes, concentrated in vacuo and the resulting solids are partitioned between ethyl acetate and water. The organic layer is washed with water and brine, dried over sodium sulfate and concentrated. The resulting material is purified by chromatography on silica gel (15 g) with 70-100% ethyl acetate/hexanes as the eluent to afford 4-[5-[4-[imino(methoxycarbonylamino)methyl]phenoxy]pentoxy]-3-methoxy-N,N-bis(1-methylethyl)benzamide 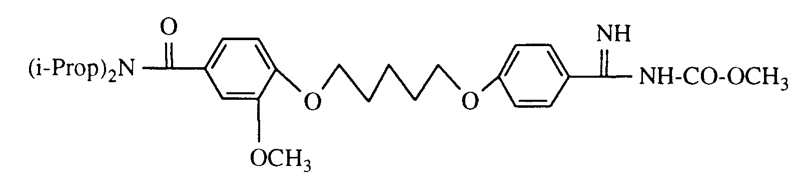
(i-Prop = isopropyl) as a colorless foam:

| CHN calculated for C₂₈H₃₉N₃O₆; | | | |
|---|---|---|---|
| Theory: | % C: 65.48; | % H: 7.65; | % N: 8.18; |
| Found: | % C: 65.46; | % H: 7.65; | % N: 8.05. |

Example 6: In a way analogously as described in example 5, the following compounds can be prepared:
4-[5-[4-[imino(phenylmethoxycarbonylamino)methyl]phenoxy]pentoxy]-3-methoxy-N,N-bis(1-methylethyl)benzamide is obtained from 4-[5-[4-(aminoiminomethyl)phenoxy]pentoxy]-3-methoxy-N,N-bis(1-methylethyl)benzamide monohydrochloride (200 mg) and benzyl chloroformate (73 mg) as a colorless foam:

| CHN calculated for C₃₄H₄₃N₃O₆-0.5 H₂O; | | | |
|---|---|---|---|
| Theory: | % C: 68.21; | % H: 7.41; | % N: 7.02; |
| Found: | % C: 68.14; | % H: 7.26; | % N: 6.85. |

4-[5-[4-[(benzoylamino)iminomethyl]phenoxy]pentoxy]-3-methoxy-N,N-bis(1-methylethyl)benzamide is obtained from 4-[5-[4-(aminoiminomethyl)phenoxy]pentoxy]-3-methoxy-N,N-bis(1-methylethyl)benzamide monohydrochloride (200 mg) and benzoyl chloride (61 mg) as a colorless foam:

| CHN calculated for C₃₃H₄₁N₃O₅; | | | |
|---|---|---|---|
| Theory: | % C: 70.82; | % H: 7.38; | % N: 7.51 |
| Found: | % C: 71.11; | % H: 7.65; | % N: 7.06. |

4-[5-[4-[imino(1-oxo-3-phenyl-2-propenylamino)methyl]phenoxy]pentoxy]-3-methoxy-N,N-bis(1-methylethyl)benzamide is obtained from 4-[5-[4-(aminoiminomethyl)phenoxy]pentoxy]-3-methoxy-N,N-bis(1-methylethyl)benzamide monohydrochloride (400 mg) and cinnamoyl chloride (142 mg) as a colorless foam:

| CHN calculated for C₃₅H₄₃N₃O₅; | | | |
|---|---|---|---|
| Theory: | % C: 71.77; | % H: 7.40; | % N: 7.17; |
| Found: | % C: 71.95; | % H: 7.60; | % N: 6.91. |

4-[5-[4-[imino[[[2-(1-methylethyl)-5-methylcyclohexyl]oxycarbonyl]amino]methyl]phenoxy]pentoxy]-3-methoxy-N,N-bis(1-methylethyl)benzamide is obtained from 4-[5-[4-(aminoiminomethyl)phenoxy]pentoxy]-3-methoxy-N,N-bis(1-methylethyl)benzamide monohydrochloride (300 mg) and L-menthol chloroformate (140 mg) as a colorless foam:

| CHN calculated for C₃₇H₅₅N₃O₆; | | | |
|---|---|---|---|
| Theory: | % C: 69.67; | % H: 8.69; | % N: 6.59; |
| Found: | % C: 70.35; | % H: 8.38; | % N: 6.18. |

4-[5-[4-[imino[octyloxycarbonyl]amino]methyl]phenoxy]pentoxy]-3-methoxy-N,N-bis(1-methylethyl)benzamide is obtained from 4-[5-[4-(aminoiminomethyl)phenoxy]pentoxy]-3-methoxy-N,N-bis(1-methylethyl)benzamide monohydrochloride (300 mg) and octyl chloroformate (123 mg) as a colorless foam:

| CHN calculated for C₃₅H₅₃N₃O₆; | | | |
|---|---|---|---|
| Theory: | % C: 68.71; | % H: 8.73; | % N: 6.87; |
| Found: | % C: 68.61; | % H: 8.50; | % N: 6.39. |

4-[5-[4-[[[9H-fluoren-9-yl)methoxycarbonyl]amino]iminomethyl]phenoxy]pentoxy]-3-methoxy-N,N-bis(1-methylethyl)benzamide is obtained from 4-[5-[4-(aminoiminomethyl)phenoxy]pentoxy]-3-methoxy-N,N-bis(1-methylethyl)benzamide monohydrochloride (300 mg) and 9-fluorenylmethyl chloroformate (166 mg) as a colorless foam:

| CHN calculated for C₄₁H₄₇N₃O₆; | | | |
|---|---|---|---|
| Theory: | % C: 72.65; | % H: 6.99; | % N: 6.20; |
| Found: | % C: 72.40; | % H: 7.09; | % N: 5.93. |

4-[5-[4-[imino[(methylsulfonyl)amino]methyl]phenoxy]pentoxy]-3-methoxy-N,N-bis(1-methylethyl)benzamide is obtained from 4-[5-[4-(aminoiminomethyl)phenoxy]pentoxy]-3-methoxy-N,N-bis(1-methylethyl)benzamide monohydrochloride (300 mg) and methane sulfonyl chloride (73 mg) as a colorless foam:

| CHN calculated for C₂₇H₃₉N₃O₆S; | | | |
|---|---|---|---|
| Theory: | % C: 60.77; | % H: 7.37; | % N: 7.87; |
| Found: | % C: 60.85; | % H: 7.55; | % N: 7.66. |

4-[5-[4-[imino[(phenylsulfonyl)amino]methyl]phenoxy]pentoxy]-3-methoxy-N,N-bis(1-methylethyl)benzamide is obtained from 4-[5-[4-(aminoiminomethyl)phenoxy]pentoxy]-3-methoxy-N,N-bis(1-methylethyl)benzamide monohydrochloride (300 mg) as a colorless foam:

| CHN calculated for C₃₂H₄₁N₃O₆S; | | | |
|---|---|---|---|
| Theory: | % C: 64.51; | % H: 6.94; | % N: 7.05; |
| Found: | % C: 64.73; | % H: 7.17; | % N: 6.86. |

4-[5-[4-[imino[octyloxycarbonyl]amino]methyl]phenoxy]pentoxy]-N,N-bis(1-methylethyl)benzamide is obtained from 4-[5-[4-(aminoiminomethyl)phenoxy]pentoxy]-N,N-bis(1-methylethyl)benzamide monohydrochloride (250 mg) and octyl chloroformate (103 mg) as a colorless foam:

| CHN calculated for C₃₄H₅₁N₃O₅; | | | |
|---|---|---|---|
| Theory: | % C: 70.19; | % H: 8.84; | % N: 7.72; |
| Found: | % C: 70.28; | % H: 8.82; | % N: 7.05. |

4-[5-[4-[[[2-(dimethylamino)ethyl]methylamino]carbonyl]amino]iminomethyl]phenoxy]pentoxy]-3-methoxy-N,N-bis(1-methylethyl)benzamide dihydrochloride is obtained from 4-[5-[4-(aminoiminomethyl)phenoxy]pentoxy]-3-methoxy-N,N-bis(1-methylethyl)benzamide (455 mg) and phenyl [2-(dimethylamino)ethyl]methylcarbamate (450 mg) as a colorless foam:

| CHN calculated for C₃₂H₅₁N₅O₅Cl₂-1.0 H₂O; | | | |
|---|---|---|---|
| Theory: | % C: 56.97; | % H: 7.91; | % N: 10.38; |
| Found: | % C: 56.64; | % H: 7.83; | % N: 10.10. |

4-[5-[4-[[(1,1-dimethylethoxy)carbonylamino]iminomethyl]phenoxy]pentoxy]-3-methoxy-N,N-bis(1-methylethyl)benzamide is obtained from 4-[5-[4-(aminoiminomethyl)phenoxy]pentoxy]-3-methoxy-N,N-bis(1-methylethyl)benzamide monohydrochloride and t-butoxycarbonyl (BOC) anhydride, as a colorless foam:

| CHN calculated for C₃₁H₄₅N₃O₆-0.5 H₂O; | | | |
|---|---|---|---|
| Theory: | % C: 65.93; | % H: 8.21; | % N: 7.44; |
| Found: | % C: 66.26; | % H: 8.29; | % N: 7.44. |

4-[5-[4-[[2,2-dimethyl-1-oxopropyl)amino]iminomethyl]phenoxy]pentoxy]-3-methoxy-N,N-bis(1-methylethyl)benzamide is obtained from 4-[5-[4-(aminoiminomethyl)phenoxy]pentoxy]-3-methoxy-N,N-bis(1-methylethyl)benzamide monohydrochloride and pivaloyl chloride as a colorless foam:

| CHN calculated for C₃₁H₄₅N₃O₅-0.5 H₂O; | | | |
|---|---|---|---|
| Theory: | % C: 68.99; | % H: 8.40; | % N: 7.79; |
| Found: | % C: 68.89; | % H: 8.43; | % N: 7.55. |

4-[5-[4-[(1-oxobutylamino)iminomethyl]phenoxy]pentoxy]-3-methoxy-N,N-bis(1-methylethyl)benzamide is obtained from 4-[5-[4-(aminoiminomethyl)phenoxy]pentoxy]-3-methoxy-N,N-bis(1-methylethyl)benzamide monohydrochloride and butyryl chloride as a colorless foam:

| CHN calculated for C₃₀H₄₃N₃O₅; | | | |
|---|---|---|---|
| Theory: | % C: 68.54; | % H: 8.25; | % N: 7.99; |
| Found: | % C: 68.52; | % H: 8.09; | % N: 7.68. |

4-[5-[4-[[(1-methylethoxycarbonyl)-amino]iminomethyl]phenoxy]pentoxy]-3-methoxy-N,N-bis(1-methylethyl)benzamide is obtained from 4-[5-[4-(aminoiminomethyl)phenoxy]pentoxy]-3-methoxy-N,N-bis(1-methylethyl)benzamide monohydrochloride and isopropyl chloroformate as a colorless foam:

| CHN calculated for C₃₀H₄₃N₃O₆; | | | |
|---|---|---|---|
| Theory: | % C: 66.52; | % H: 8.00; | % N: 7.76; |
| Found: | % C: 66.15; | % H: 7.80; | % N: 7.48. |

4-[5-[4-[(acetylamino)iminomethyl]phenoxy]pentoxy]-3-methoxy-N,N-bis(1-methylethyl)benzamide is obtained from 4-[5-[4-(aminoiminomethyl)phenoxy]pentoxy]-3-methoxy-N,N-bis(1-methylethyl)benzamide monohydrochloride and acetyl chloride as a colorless foam:

| CHN calculated for C₂₈H₃₉N₃O₅; | | | |
|---|---|---|---|
| Theory: | % C: 67.58; | % H: 7.90; | % N: 8.44; |
| Found: | % C: 67.46; | % H: 8.10; | % N: 8.10. |

Example 7: 4-[5-[4-(Aminoiminomethyl)phenoxy]pentoxy]-3-methoxy-N,N-bis(1-methylethyl)benzamide (3.65 kg, 8.01 mole) is dissolved in acetonitrile (29.2 L) with warming up to 60°C and filtered gravemetrically into a 20 gallon reactor. The product crystallizes out and is rewarmed to effect solution. A hot filtered solution of maleic acid (930 g, 8.02 mole) in acetonitrile (9.2 L) is added rapidly and an exotherm occurs to 63°C. A solid crystallizes and then redissolves. The reaction mixture is stirred to 55°C at which point crystallization begins. The hot water bath is replaced with an ice bath and the suspension cooled to 10°C and filtered. A second reaction mixture of the same size is run simultaneously and filtered along with the first reaction mixture. The product is washed with acetonitrile (4 x 2L) and dried in vacuo (80°C, 3 mm Hg) at for 24 hours and at 83°C for another 24 hours to give the crude product (95.9%, HPLC 99.3%). Acetonitrile (43.9 L), water (4.39 L) and 4-39 L) and 4-[5-[4-(aminoiminomethyl)-phenoxy]pentoxy]-3-methoxy-N,N-bis (1 -methylethyl)benzamide (Z)-2-butenedioate (1:1) (8.780 g) are combined in a 20 gallon reactor and heated to 60°C to effect a solution charcoal G-60 (440 g m) is added and the reaction mixture stirred 1/2 hour at 60°C and filtered gravemetrically through a heated funnel into another 20 gallon reactor. The product is allowed to crystallize at ambient temperature for 66 hours. Stirred gently overnight 15 hours and filtered, washed with water (3 x 4 L) and dried in vacuo (85°C, 3 mm Hg) for 24 hours under a nitrogen sweep to give 4-[5-[4-(aminoiminomethyl)phenoxy]pentoxy]-3-methoxy-N,N-bis(1-methylethyl)benzamide (Z)-2-butenedioate (1:1), mp = 164-166°C.

The starting material can be prepared, for example, as follows:
To a solution of 1-bromo-5-chloropentane (3794 g, 20.45 mol) in acetonitrile (38 L) is added 4-cyanophenol (2437 g, 20.45 mol) and powdered potassium carbonate (2827 g, 20.45 mol). This suspension is heated with stirring under nitrogen at 96°C bath temperature (82°C-internal) for 20 hours. Additional powdered potassium carbonate (2827 g 20.45 mol.) is then added to the hot reaction mixture followed by potassium iodide (3397 g, 20.45 mol) and methyl vanillate (3725 g, 20.45 mol). The reaction mixture is heated under nitrogen for another 3 days until TLC shows no intermediate chloro compound present. The bath is removed and stirring stopped. The reaction mixture is filtered hot through a filter crock and into another 20 gallon reactor. The filtrate is stirred and cooled with an ice bath to 10°C for 3 hours and the product filtered, washed with diethyl ether (4 x 1 L) and dried in vacuo (50°C, 3 mm Hg) to give methyl 4-[5-(4-cyanophenoxy)-pentoxy]-3-methoxybenzoate, mp 105-107°C, HPLC indicating a purity of 99.8%. Methyl 4-[5-(4-cyanophenoxy)-pentoxy]-3-methoxybenzoate (3600 g, 9.74 mol) is dissolved in tetrahydrofuran (36 L) with warming to 30°C. The solution is then cooled to 20°C and a solution of tetrabutylammonium hydroxide (40% in water, 7.1 L, 1.64 m) is added over 20 minutes. The reaction mixture is then stirred at an internal temperature of 25°C for 7 hours and overnight at room temperature (23°C). After cooling to 10°C with an ice bath, hydrochloric acid (1.0 N, 14.4 L) is added over 1 hour. After stirring and cooling to 10°C for three hours, the product is filtered, washed with water (24 L) and dried in vacuo (76°C, 3 mm Hg) to give 4-[5-(4-cyanophenoxy)pentoxy]-3-methoxybenzoic acid, mp 159-161°C. HPLC indicates 99.1% purity. A second crop of acid is obtained from the filtrates after diluting further with water (20 L). The product is filtered, washed with water (4 x 3 L) and dried in vacuo to give 4-[5-(4-cyanophenoxy)pentoxy]-3-methoxybenzoic acid, mp 158-160°C, (HPLC indicates 98.3% purity).

4-[5-(4-cyanophenoxy)pentoxy-3-methoxybenzoic acid (3600 g, 10.13 mol) is suspended in dichloromethane (36 L) and to this is added thionyl chloride (1345 g 11.30 mol) dropwise over 20 minutes followed by dimethylformamide (74.4 g, 10.0 mol). The reaction mixture is stirred at room temperature for 21 hours; after 6 hours a complete solution is obtained. The solution is concentrated in vacuo (50°C, 3 mm Hg) to give 4-[5-(4-cyanophenoxy)pentoxy]-3-methoxybenzoyl chloride as a solid, which is then redissolved in dichloromethane (36 L) without further purification and cooled to 5°C with an ice bath. Diisopropylamine (3077 g, 30.40 mol) is added dropwise over a period of 1 hour. The reaction mixture is stirred at 20°C for 3 hours and then, N,N-dimethylethylenediamine (80.3 g, 0.91 mol) is added. The reaction is followed by TLC (2:1 ethyl acetate/hexanes, silica plates) until no more byproduct (bis-anhydride of 4-[5-(4-cyanophenoxy)pentoxy]-3-methoxybenzoic acid) is detected. The reaction mixture is then stirred overnight at room temperature. Water (18 L) is added,stirred and the layers separated. The organic layer is washed with hydrochloric acid (1.0 N, 23 L), water (18 L), ammonium hydroxide (0.5 N, 2 x 23 L), water (18 L) and brine solution (18 L). The separated dichloromethane solution is dried over anhydrous magnesium sulfate, filtered and concentrated in vacuo (50°C, 3 mm Hg) to give 4-[5-(4-cyanophenoxy)pentoxy]-3-methoxy-N,N-bis(1-methylethyl )- benzamide as a solid. The solid is dissolved in 2 B ethanol (21.5 L) by warming and to the solution is added water (8.6 L). The product is allowed to crystallize overnight in an ice bath. The product is filtered, washed with EtOH/H₂O (5:2, 2 x 2 L) and dried in vacuo (54°C, 3 mm Hg) to give 3700 g (83.2%) of 4-[5-(4-cyanophenoxy)pentoxy]-3-methoxy-N,N-bis(1-methylethyl )- benzamide, mp 90-92°C. HPLC indicates a purity of >99.7%.

Ethanol (2B,6.0 L) is cooled in an ice bath to 0°C and saturated with anhydrous hydrogen chloride over a period of 7 1/2 hours. The solution is left in the ice bath overnight and the hydrogen chloride addition is continued an additional hour at 0°C. 4-[5-(4-Cyanophenoxy)pentoxy]-3-methoxy-N,N-bis(1-methylethyl )- benzamide (4200 g, 9.57 mole) is added rapidly over 30 minutes and an exotherm raises the reaction mixture temperature to 10°C. Hydrogen chloride addition is continued for 6 hours and the purple solution stirred overnight at 20°C. The reaction mixture is followed by TLC (ethyl acetate/hexane, 2:1) using silica plates. A total of 4-5 days is required for completion of this reaction with periodic resaturation with hydrogen chloride. The thick purple solution is transferred into a 20 gallon reactor and triturated with anhydrous diethyl ether (2 x 30 L), removing the ether solution after each wash. The reactor is put under vacuum (3 mm Hg) for 0.5 hour to dispel most of the hydrogen chloride leaving ethyl 4-[5-[3-methoxy-4-[N,N-bis(1-methylethyl)aminocarbonyl]phenox y] pentoxy]benzenecarboximidoate monohydrochloride as a thick purple oil.

Ethanol 2 B (42 L) is added to the reactor and the ethyl 4-[5-[3-methoxy-4-[N,N-bis(1-methylethyl)aminocarbonyl]phenox y] -pentoxy]benzenecarboximidoate monohydrochloride is dissolved. The solution is cooled to 5°C and anhydrous ammonia is bubbled into the solution for 7.5 hours until saturation is obtained. After stirring overnight in the ice bath, and ammonia addition is continued another 7.5 hours at 5°C. The reaction mixture is followed by TLC (CH₂Cl₂/MeOH, 9:1) using silica plates. A total of four days is required for completion of reaction with periodic resaturation with ammonia gas. The reaction mixture is filtered and the filtrate divided into two equal portions. Concentration in vacuo (55°C 3 mm Hg) gives an oily viscous suspension which is charged into a 20 gallon reactor. This is admixed with hydrochloric acid (12 N, 560 ml) diluted with water (44 L). At this point, the compound dissolves (pH is 1.62). The solution is washed with diethyl ether (3 x 18 L), and the pH adjusted to 12.6 with a 6 N sodium hydroxide solution (3.0 L). The oily suspension crystallizes after stirring overnight at 20°C. The product is filtered, washed with water (5 x 4 L) and dried in vacuo (60°C 3 mm Hg) to give the product. The second half of the reaction mixture is worked up in a similar manner to give 4-[5-[4-(aminoiminomethyl)phenoxy]pentoxy]-3-methoxy-N,N-bis(1-methylethyl)-benzamide is obtained, mp 112-114°C. HPLC indicates a purity of 98.5%.

Example 8:
A stirred, solution of 4-[5-[4-(aminoiminomethyl)phenoxy]pentoxy]-3-methoxy-N,N-bis(1-methylethyl)benzamide monohydrochloride (200 mg, 0.41 mmol) in 4.0 mL anhydrous methylene chloride is treated at 0°C sequentially with triethylamine (0.068 mL, 0.48 mmol) and methyl isocyanate (0.030 mL, 0.49 mmol). The solution is stirred 30 minutes, concentrated in vacuo and the resulting solids are partitioned between ethyl acetate and water. The organic layer is washed with water and brine, dried over sodium sulfate and concentrated. The resulting material is purified by chromatography on silica gel (15 g) with 70-100% ethyl acetate/hexanes as the eluent to afford 4-[5-[4-[imino(methylaminocarbonylamino)methyl]phenoxy]pentoxy]-3-methoxy-N,N-bis(1-methylethyl)benzamide as a colorless foam:

| CHN calculated for C₂₈H₄₀N₄O₅; | | | |
|---|---|---|---|
| Theory: | %C: 65.60; % | H: 7.87; % | N: 10.93; |
| Found: | %C: 65.79; % | H: 7.61; % | N: 10.90. |

Example 9: In a way analogously as described, for example, in example 8, the following compounds can be manufactured:
4-[5-[4-[Imino[(3,4-dimethoxyphenyl)carbonylamino]methyl]phenoxy]pentoxy]-3-methoxy-N,N-bis(1-methylethyl)benzamide is obtained from 4-[5-[4-(aminoiminomethyl)phenoxy]pentoxy]-3-methoxy-N,N-bis(1-methylethyl)benzamide monohydrochloride (400 mg) and 3,4-dimethoxybenzoyl chloride (170 mg) as a colorless foam:

| CHN calculated for C₃₅H₄₅N₃O₇; | | | |
|---|---|---|---|
| Theory: | %C: 67.83; | % H: 7.32; | % N: 6.78; |
| Found: | %C: 68.19; | % H: 7.55; | % N: 6.23. |

4-[5-[4-[Imino[[[(1-methylethyl)amino]carbonyl]amino)methyl]phenoxy]pentoxy]-3-methoxy-N,N-bis(1-methylethyl)benzamide is obtained from 4-[5-[4-(aminoiminomethyl)phenoxy]pentoxy]-3-methoxy-N,N-bis(1-methylethyl)benzamide monohydrochloride (300 mg) and isopropyl isocyanate (54 mg) as a colorless foam:

| CHN calculated for C₃₀H₄₄N₄O₅; | | | |
|---|---|---|---|
| Theory: | %C: 66.64; % | H: 8.20; % | N: 10.36; |
| Found: | %C: 66.94; % | H: 8.09; % | N: 9.99. |

4-[5-[4-[(Ethylamino)carbonyl]amino]iminomethyl]phenoxy]pentoxy]-3-methoxy-N,N-bis(1-methylethyl)benzamide is obtained from 4-[5-[4-(aminoiminomethyl)phenoxy]pentoxy]-3-methoxy-N,N-bis(1-methylethyl)benzamide monohydrochloride (300 mg) and ethyl isocyanate (45 mg) as a colorless foam:

| CHN calculated for C₂₉H₄₂N₄O₅; | | | |
|---|---|---|---|
| Theory: | %C: 66.13; | % H: 8.0; | % N: 10.64; |
| Found: | %C: 66.35; | % H: 7.96; | % N: 10.25. |

4-[5-[4-[Imino[[(phenylamino)carbonyl]amino]methyl]phenoxy]pentoxy]-3-methoxy-N,N-bis(1-methylethyl)benzamide (240 mg) is obtained from 4-[5-[4-(aminoiminomethyl)phenoxy]pentoxy]-3-methoxy-N,N-bis(1-methylethyl)benzamide monohydrochloride (300 mg) and phenyl isocyanate (76 mg) as a colorless foam:

| CHN calculated for C₃₃H₄₂N₄O₅; | | | |
|---|---|---|---|
| Theory: | %C: 68.97; | % H: 7.37; | % N: 9.75; |
| Found: | %C: 69.20; | % H: 7.75; | % N: 9.37. |

Example 10: In a way analogously as described, for example, in one of the preceeding examples, the following compounds can be manufactured:
4-[5-[4-(aminoiminomethyl)phenoxy]pentoxy]-N-ethyl-N-phenylbenzamide monohydrochloride;
4-[5-[4-(aminoiminomethyl)phenoxy]pentoxy]-2,6-dimethoxy-N,N-bis(1-methylethyl)benzamide monohydrochloride;
4-[5-[4-(aminoiminomethyl)phenoxy]pentoxy]-3,5-dimethoxy-N,N-bis(1-methylethyl)benzamide monohydrochloride;
4-[5-[4-(aminoiminomethyl)phenoxy]pentoxy]-2,3-dimethoxy-N,N-bis(1-methylethyl)benzamide monohydrochloride;
4-[5-[4-(aminoiminomethyl)phenoxy]pentoxy]-3,5-dichloro-N,N-b is(1-methylethyl)benzamide monohydrochloride;
4-[5-[4-(aminoiminomethyl)phenoxy]pentoxy]-2-methoxy-6-methyl -N,N-bis(1-methylethyl)benzamide monohydrochloride;
4-[5-[4-(aminoiminomethyl)phenoxy]pentoxy]-3-chloro-N,N-bis(1-methylethyl)benzamide monohydrochloride;
4-[5-[4-(aminoiminomethyl)phenoxy]pentoxy]-3-bromo-N,N-bis(1- - methylethyl)benzamide monohydrochloride;
5-[5-[4-(aminoiminomethyl)phenoxy]pentoxy]-2-methoxy-N,N-bis( 1-methylethyl)benzamide monohydrochloride;
5-[5-[4-(aminoiminomethyl)phenoxy]pentoxy]-2-bromo-N,N-bis(1- - methylethyl)benzamide monohydrochloride;
1-[4-[5-[4-(aminoiminomethyl)phenoxy]pentoxy]benzoyl]-2,2,6,6 -tetramethylbenzamide monohydrochloride;
4-[5-[4-[(3-pyridylcarbonylamino)iminomethyl]phenoxy]pentoxy] - 3-methoxy-N,N-bis(1-methylethyl)benzamide;
4-[5-[4-[(4-pyridylcarbonylamino)iminomethyl]phenoxy]pentoxy]-3-methoxy-N,N-bis(1-methylethyl)benzamide;
4-[5-[4-[1-imidazolylcarbonylamino)iminomethyl]phenoxy]pentox y]-3-methoxy-N,N-bis(1-methylethyl)benzamide.

Example 11: a) Preparation of 10,000 tablets each containing 20 mg of the active ingredient, for example,
4-[5-[4-(Aminoiminomethyl)phenoxy]pentoxy]-3-methoxy-N,N-bis( 1-methylethyl)benzamide (Z)-2-butenedioate (1:1):

| | |
|---|---|
| active ingredient | 200.0 g |
| Lactose | 2.535.00 g |
| Corn starch | 125.00g |
| Polyethylene glycol 6,000 | 150.00 g |
| Magnesium stearate | 40.00 g |
| Purified water | q.s. |

Procedure: All the powders are passed through a screen with openings of 0.6 mm. The drug substance, lactose, magnesium stearate and half of the starch are mixed in a suitable mixer. The other half of the starch is suspended in 65 ml of water and the suspension added to the boiling solution of the polyethylene glycol in 250 ml of water. The paste formed is added to the powders, which are granulated, if necessary, with an additional amount of water. The granulate is dried overnight at 35°C broken on a screen with 1.2 mm openings and compressed into tablets, using concave punches uppers bisected.

Example 12:
Analogously tablets are prepared, containing about 10-100 mg of one of the other compounds disclosed and exemplified herein.
b) Preparation of 1,000 capsules each containing 40 mg of the active ingredient, for example, 4-[5-[4-(Aminoiminomethyl)phenoxy]pentoxy]-3-methoxy-N,N-bis( 1-methylethyl)benzamide (Z)-2-butenedioate (1:1):

| | |
|---|---|
| active ingredient | 40.00 g |
| Lactose | 177.00g |
| Modified starch | 80.00 g |
| Magnesium stearate | 3.00 g |

Procedure: All the powders are passed through a screen with openings of 0.6 mm. The drug substance is placed in a suitable mixer and mixed first with the magnesium stearate, then with the lactose and starch until homogenous. No. 2 hard gelatin capsules are filled with 300 mg of said mixture each, using a capsule filling machine.

Analogously capsules are prepared, containing about 10-100 mg of the other compounds disclosed and exemplified herein.

## Claims (Claims for the following Contracting State(s): AT, BE, CH, DE, DK, FR, GB, IT, LI, LU, NL, PT, SE)

1. A compound of the formula 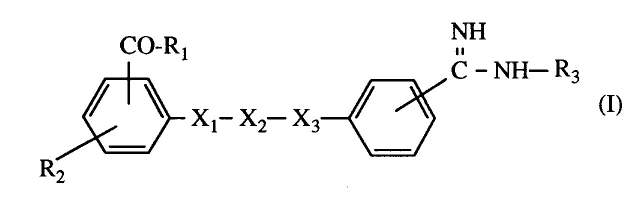 in which:
R₁ is amino which is mono- or disubstituted by a substituent selected from an aliphatic hydrocarbon radical, an araliphatic hydrocarbon radical, an aromatic radical, and a cycloaliphatic hydrocarbon radical or is amino which is disubstituted by a divalent aliphatic hydrocarbon radical;
R₂ is hydrogen, halogen, trifluoromethyl, an aliphatic hydrocarbon radical, or is hydroxy which is etherified by an aliphatic alcohol, araliphatic alcohol, or aromatic alcohol or which is esterified by an aliphatic or araliphatic carboxylic acid;
R₃ is hydrogen or an acyl radical which is derived from an organic carbonic acid, an organic carboxylic acid, a sulfonic acid, or a carbamic acid;
X₁ and X₃, independently of one another, are oxygen (-O-) or sulphur (-S-); and X₂ is a divalent aliphatic hydrocarbon radical which may be interrupted by an aromatic radical;
wherein the phenyl rings of formula I may be, independently of one another, further substituted by one or more substituents selected from halogen, trifluoromethyl, an aliphatic hydrocarbon radical, hydroxy, and hydroxy which is etherified by an aliphatic alcohol or which is esterified by an aliphatic or araliphatic carboxylic acid;
wherein aryl in the above definitions may be, independently of one another, further substituted by one or more substituents selected from halogen, trifluoromethyl, an aliphatic hydrocarbon radical, hydroxy, and hydroxy which is etherified by an aliphatic alcohol or which is esterified by an aliphatic or araliphatic carboxylic acid;
wherein a cycloaliphatic hydrocarbon radical may be substituted by an aliphatic radical; or a pharmaceutically acceptable salt thereof.

2. A compound according to claim 1 wherein
R₁ is amino which is mono- or disubstituted by a substituent selected from C₁-C₇-alkyl, C₃-C₇-alkenyl, C₃-C₇-alkynyl, phenyl-C₁-C₇-alkyl, phenyl-C₂-C₇-alkenyl, phenyl-C₂-C₇-alkynyl, phenyl, naphthyl, indanyl, fluorenyl, cycloalkyl, and cycloalkenyl, cycloalkyl and cycloalkenyl each being unsubstituted or mono- or polysubstituted by C₁-C₇-alkyl, or is amino which is disubstituted by C₂-C₆-alkylene;
R₂ is hydrogen, halogen, trifluoromethyl, C₁-C₇-alkyl, C₃-C₇- alkenyl, C₃-C₇-alkynyl, C₁-C₇-alkoxy, C₃-C₇-alkenyloxy, phenyl-C₁-C₇-alkoxy, phenoxy, C₂-C₈-alkanoyloxy, C₃-C₈- alkenoyloxy, or phenyl-C₂-C₈-alkanoyloxy;
R₃ is hydrogen, alkoxycarbonyl or alkenyloxycarbonyl, each of which is unsubstituted or substituted by phenyl, naphthyl, indanyl or fluorenyl, or is cycloalkoxycarbonyl being unsubstituted or mono- or polysubstituted by C₁-C₇-alkyl, or is C₁-C₇- alkanoyl or phenyl-C₂-C₇-alkanoyl, or is benzoyl, naphthoyl, indanoyl or fluorenoyl, or is C₁-C₇-alkanesulfonyl, phenyl-C₁-C₇-alkanesulfonyl, C₃-C₇-cycloalkanesulfonyl, or phenylsulfonyl, or is aminocarbonyl which is substituted by C₁-C₇-alkyl, phenyl-C₁-C₇-alkyl or phenyl;
X₁ and X₃, independently of one another, is O or S;
X₂ is C₂-C₈-alkylene, C₂-C₄-alkylene-phenylene-C₂-C₄-alkylene or C₂-C₄-alkylene-naphthylene-C₂-C₄-alkylene;
wherein the phenyl rings of formula I may be, independently of one another, substituted by one or more substituents selected from halogen, trifluoromethyl, C₁-C₇-alkyl, C₃-C₇-alkenyl, C₃-C₇-alkynyl, hydroxy, C₁-C₇-alkoxy, C₃-C₇-alkenyloxy, phenyl-C₁-C₇-alkoxy, C₂-C₈-alkanoyloxy, C₃-C₇-alkenoyloxy and phenyl-C₂-C₈-alkanoyloxy;
wherein the aromatic radicals in the above definitions may be, independently of one another, substituted by one or more substituents selected from halogen, trifluoromethyl, C₁-C₇- alkyl, C₃-C₇-alkenyl, C₃-C₇-alkynyl, hydroxy, C₁-C₇-alkoxy, C₃-C₇-alkenyloxy phenyl-C₁-C₇-alkoxy, C₂-C₈-alkanoyloxy, C₃-C₈-alkenoyloxy and phenyl-C₂-C₈-alkanoyloxy ;or a pharmaceutically acceptable salt thereof.

3. A compound according to claim 1 wherein
R₁ is amino which is mono- or disubstituted by a substituent selected from C₁-C₇-alkyl, phenyl-C₁-C₇-alkyl, phenyl and C₃-C₆-cycloalkyl, C₃-C₆-cycloalkyl being unsubstituted or mono- or polysubstituted by C₁-C₇-alkyl, or is amino which is disubstituted by C₃-C₆-alkylene;
R₂ is hydrogen, C₁-C₇-alkoxy or phenyl-C₁-C₄-alkoxy;
R₃ is hydrogen, C₁-C₁₂-alkoxycarbonyl, C₂-C₅-alkanoyl, phenyl-C₂-C₅-alkanoyl, benzoyl which is unsubstituted or substituted by halogen, trifluoromethyl, C₁-C₇-alkyl, or C₁-C₇-alkoxy, or is C₃-C₆-cycloalkylcarbonyl which is unsubstituted or substituted by C₁-C₇-alkyl, or is naphthoyl, indanoyl or fluorenoyl, or is C₁-C₇alkanesulfonyl, phenyl-C₁-C₇alkanesulfonyl, C₃-C₇-cycloalkanesulfonyl, or phenylsulfonyl, or is aminocarbonyl which is substituted by C₁-C₇-alkyl, phenyl-C₁-C₇-alkyl or phenyl; X₁ and X₃ each are -O-, or furthermore are, independently of one another, -O- or -S-; X₂ is C₂-C₇-alkylene or C₂-C₄-alkylene-phenylene-C₂-C₄-alkylene;
wherein the phenyl rings of formula I may be unsubstituted or, furthermore, independently of one another, substituted by one or more substituents selected from halogen, trifluoromethyl, C₁-C₇-alkyl, and C₁-C₇-alkoxy;
wherein phenyl in the above definitions is unsubstituted or, furthermore, independently of one another, substituted by a substituent selected from halogen, trifluoromethyl, C₁-C₇-alkyl, and C₁-C₇-alkoxy; or a pharmaceutically acceptable salt thereof.

4. A compound according to claim 1 wherein
-CO-R₁ is located in position 4 (para) or 5 (meta); R₂- is located in position 2 (ortho); and
-C(=NH)-NH-R₃ is located in position 4 (para) of the corresponding phenyl ring.

5. A compound according to claim 1 of formula IA 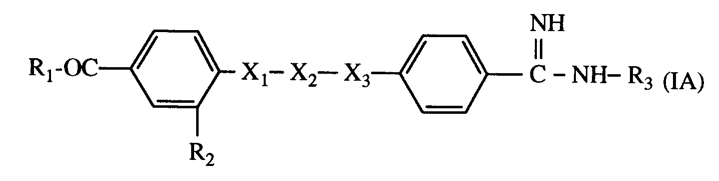 wherein
R₁ is di-C₁-C₄-alkylamino, C₁-C₄-alkyl-phenyl-C₁-C₄-alkyl-amino, di-C₃-C₆-cycloalkylamino which is unsubstituted or substituted by C₁-C₄-alkyl or 1-piperidino substituted by C₁-C₄-alkyl;
R₂ is hydrogen or C₁-C₄-alkoxy;
R₃ is hydrogen, C₁-C₁₂-alkoxycarbonyl, phenyl-C₁-C₄-alkoxycarbonyl, C₂-C₅-alkanoyl, benzoyl which is unsubstituted or substituted by halogen, trifluoromethyl, C₁-C₄-alkyl, or C₁-C₄-alkoxy, C₃-C₆-cycloalkylcarbonyl which is unsubstituted or substituted by C₁-C₄-alkyl;
X₁ and X₃ are -O-;
X₂ is C₄-C₇-alkylene;
wherein the phenyl rings of formula IA may be unsubstituted or, furthermore, independently of one another, substituted by one or more substituents selected from halogen, trifluoromethyl, C₁-C₄-alkyl, and C₁-C₄-alkoxy; or a pharmaceutically acceptable salt thereof.

6. A compound according to claim 1 of formula IA wherein
R₁ is di-C₁-C₄-alkylamino, C₁-C₄-alkyl-phenyl-C₁-C₄-alkyl-amino, di-C₃-C₆-cycloalkylamino which is unsubstituted or substituted by C₁-C₄-alkyl, 1-piperidino substituted by C₁-C₄-alkyl;
R₂ is hydrogen or C₁-C₄-alkoxy;
R₃ is C₁-C₄alkanesulfonyl, phenyl-C₁-C₄-alkanesulfonyl, C₃-C₇-cycloalkane-sulfonyl, or phenylsulfonyl, or is aminocarbonyl which is substituted by C₁-C₄-alkyl, phenyl-C₁-C₄-alkyl or phenyl;
X₁ and X₃ are -O-;
X₂ is C₄-C₇-alkylene; or a pharmaceutically acceptable salt thereof.

7. A compound according to claim 5 of formula IA wherein
R₁ is di-C₁-C₄-alkylamino;
R₂ is hydrogen or C₁-C₄-alkoxy;
R₃ is C₁-C₁₂-alkoxycarbonyl, phenyl-C₁-C₄-alkoxycarbonyl, C₂-C₅-alkanoyl, benzoyl which is unsubstituted or substituted by halogen, trifluoromethyl, C₁-C₄-alkyl, or C₁-C₄-alkoxy, C₃-C₆-cycloalkylcarbonyl which is unsubstituted or substituted by C₁-C₄-alkyl;
X₁ and X₃ are -O-;
X₂ is C₄-C₇-alkylene; or a pharmaceutically acceptable salt thereof.

8. A compound according to claim 5 of formula IA wherein
R₁ is di-C₁-C₄-alkylamino;
R₂ is hydrogen or C₁-C₄-alkoxy;
R₃ is hydrogen;
X₁ and X₃ are -O-;
X₂ is C₄-C₇-alkylene; or a pharmaceutically acceptable salt thereof.

9. A compound according to claim 5 of formula IA wherein
R₁ is di-ethylamino or di-isopropylamino;
R₂ is hydrogen or methoxy;
R₃ is hydrogen;
X₁ and X₃ are -O-;
X₂ is pentylene; or a pharmaceutically acceptable salt thereof.

10. 4-[5-[4-(aminoiminomethyl)phenoxy]pentoxy]-3-methoxy-N,N-bis(1-methylethyl)benzamide or a pharmaceutically acceptable salt thereof according to claim 1.

11. 4-[5-[4-(aminoiminomethyl)phenoxy]pentoxy]-3-methoxy-N,N-diethylbenzamide or a pharmaceutically acceptable salt thereof according to claim 1.

12. 4-[5-[5-(aminoiminomethyl)phenoxy]pentoxy]-3-methoxy-N-(1-methylethyl)-N-(phenylmethyl)benzamide or a pharmaceutically acceptable salt thereof according to claim 1.

13. 4-[5-[4-(aminoiminomethyl)phenoxy]pentoxy]-N,N-bis(1-methylethyl)benzamide or a pharmaceutically acceptable salt thereof according to claim 1.

14. 3-[5-[4-(aminoiminomethyl)phenoxy]pentoxy]-N,N-bis(1-methylethyl)benzamide or a pharmaceutically acceptable salt thereof according to claim 1.

15. 4-[5-[4-aminoiminomethyl)phenoxy]pentoxy]-2-hydroxy-N,N-bis(1-methylethyl)benzamide or a pharmaceutically acceptable salt thereof according to claim 1.

16. 4-[5-[4-[(benzoylamino)iminomethyl]phenoxy]pentoxy]-3-methoxy-N,N-bis(1-methylethyl)benzamide or a pharmaceutically acceptable salt thereof according to claim 1.

17. 4-[5-[4-[imino[octyloxycarbonyl]amino]methyl]phenoxy]pentoxy]-3-methoxy-N,N-bis(1-methylethyl)benzamide or a pharmaceutically acceptable salt thereof according to claim 1.

18. 4-[5-[4-[imino[octyloxycarbonyl]amino]methyl]phenoxy]pentoxy]-N,N-bis(1-methylethyl)benzamide or a pharmaceutically acceptable salt thereof according to claim 1.

19. 4-[5-[4-[imino(phenylmethoxycarbonylamino)methyl]phenoxy]pentoxy]-3-methoxy-N,N-bis(1-methylethyl)benzamide or a pharmaceutically acceptable salt thereof according to claim 1.

20. Process for the production of compounds of formula I defined in claim 1 and pharmaceutically acceptable salts thereof comprising
a) converting a compound of the formula IIa 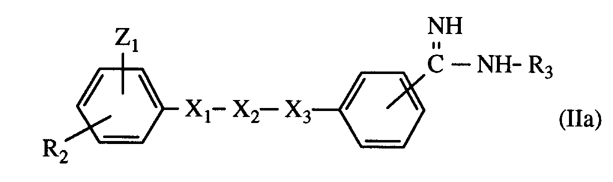 or a salt thereof in which Z₁ is a radical which can be converted into the variable -CO-R₁, Z₁ into the variable -CO-R₁, or,
b) for the manufacture of compounds of the formula I in which R₃ is hydrogen, in a compound of the formula IIIa 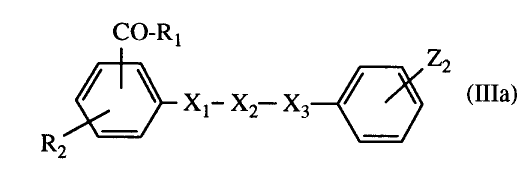 or a salt thereof in which Z₂ is a radical which can be converted into the variable -C(=NH)-NH-R₃, converting Z₂ into the variable -C(=NH)-NH-R₃, or
c) reacting a compound of the formula IVa 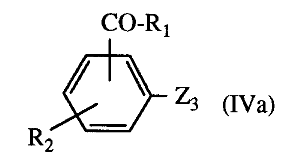 or a salt thereof with a compound of the formula IVb 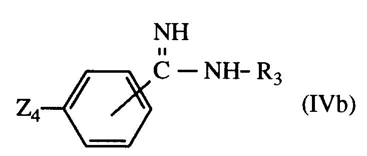 or a salt thereof in which Z₃ is a group of the formula -X₁-X₂-Z₅ and Z₄ is -Z₆, or Z₃ is -Z₆ and Z₄ is a group of the formula Z₅-X₂-X₃-, wherein one of the radicals Z₅ and Z₆ is hydroxy or mercapto and the other is hydroxy, mercapto or reactive esterified hydroxy,
and, if desired, converting a compound of the formula I or a salt thereof obtainable according to the process or in another manner into another compound or a salt thereof according to the invention, converting a free compound of the formula I obtainable according to the process into a salt, converting a salt obtainable according to the process into the free compound of the formula I or into another salt, or resolving a mixture of isomers obtainable according to the process and isolating the desired compound.

21. A pharmaceutical preparation containing a compound of formula I according to claim 1 and customary carriers.

22. The use of compounds of formula I according to claim 1 or salts thereof and customary carriers for producing pharmaceutical preparations.

23. A compound of the formula I according to claim 1 or salts thereof for use in a method for the therapeutic treatment of the human or animal body.

24. The compounds of the formula I according to claim 1 or salts thereof for use as antagonist of LTB₄ on human polymorphonuclear leucocytes.

25. A pharmaceutical composition suitable for antagonizing LTB₄ receptors in mammals comprising an effective LTB₄ receptors antagonizing amount of a compound of claim 1 or a pharmaceutically acceptable salt thereof and a carrier.

## Claims (Claims for the following Contracting State(s): ES, GR)

1. A process for the manufacture of a compound of the formula I 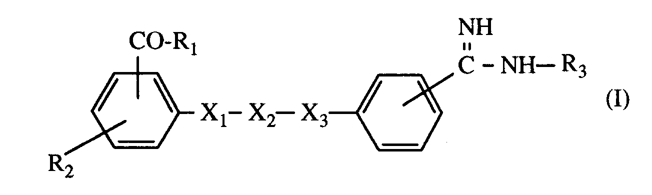 in which:
R₁ is amino which is mono- or disubstituted by a substituent selected from an aliphatic hydrocarbon radical, an araliphatic hydrocarbon radical, an aromatic radical, and a cycloaliphatic hydrocarbon radical or is amino which is disubstituted by a divalent aliphatic hydrocarbon radical;
R₂ is hydrogen, halogen, trifluoromethyl, an aliphatic hydrocarbon radical, or is hydroxy which is etherified by an aliphatic alcohol, araliphatic alcohol, or aromatic alcohol or which is esterified by an aliphatic or araliphatic carboxylic acid;
R₃ is hydrogen or an acyl radical which is derived from an organic carbonic acid, an organic carboxylic acid, a sulfonic acid, or a carbamic acid;
X₁ and X₃, independently of one another, are oxygen (-O-) or sulphur (-S-); and
X₂ is a divalent aliphatic hydrocarbon radical which may be interrupted by an aromatic radical;
wherein the phenyl rings of formula I may be, independently of one another, further substituted by one or more substituents selected from halogen, trifluoromethyl, an aliphatic hydrocarbon radical, hydroxy, and hydroxy which is etherified by an aliphatic alcohol or which is esterified by an aliphatic or araliphatic carboxylic acid;
wherein aryl in the above definitions may be, independently of one another, further substituted by one or more substituents selected from halogen, trifluoromethyl, an aliphatic hydrocarbon radical, hydroxy, and hydroxy which is etherified by an aliphatic alcohol or which is esterified by an aliphatic or araliphatic carboxylic acid;
wherein a cycloaliphatic hydrocarbon radical may be substituted by an aliphatic radical; or a pharmaceutically acceptable salt thereof comprising,
a) converting a compound of the formula IIa 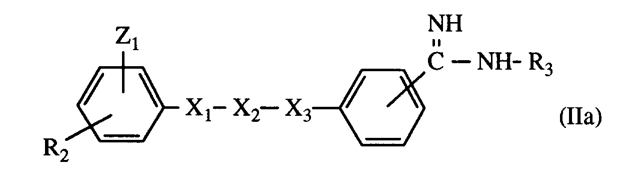 or a salt thereof in which Z₁ is a radical which can be converted into the variable -CO-R₁, Z₁ into the variable -CO-R₁, or,
b) for the manufacture of compounds of the formula I in which R₃ is hydrogen, in a compound of the formula IIIa 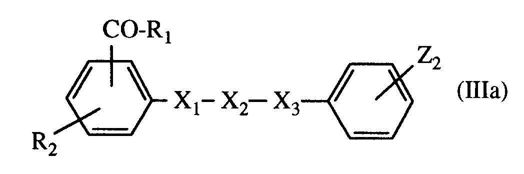 or a salt thereof in which Z₂ is a radical which can be converted into the variable -C(=NH)-NH-R₃, converting Z₂ into the variable -C(=NH)-NH-R₃, or
c) reacting a compound of the formula IVa 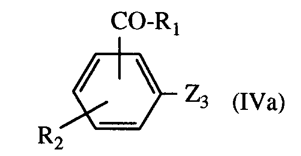 or a salt thereof with a compound of the formula IVb 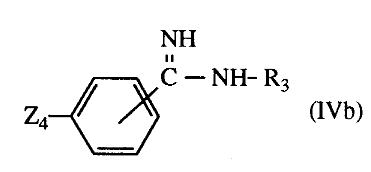 or a salt thereof in which Z₃ is a group of the formula -X₁-X₂-Z₅ and Z₄ is -Z₆, or Z₃ is -Z₆ and Z₄ is a group of the formula Z₅-X₂-X₃-, wherein one of the radicals Z₅ and Z₆ is hydroxy or mercapto and the other is hydroxy, mercapto or reactive esterified hydroxy,
and, if desired, converting a compound of the formula I or a salt thereof obtainable according to the process or in another manner into another compound or a salt thereof according to the invention, converting a free compound of the formula I obtainable according to the process into a salt, converting a salt obtainable according to the process into the free compound of the formula I or into another salt, or resolving a mixture of isomers obtainable according to the process and isolating the desired compound

2. A process according to claim 1 wherein there are produced compounds of formula I wherein R₁ is amino which is mono- or disubstituted by a substituent selected from C₁-C₇-alkyl, C₃-C₇-alkenyl, C₃-C₇-alkynyl, phenyl-C₁-C₇-alkyl, phenyl-C₂-C₇-alkenyl, phenyl-C₂-C₇- alkynyl, phenyl, naphthyl, indanyl, fluorenyl, cycloalkyl, and cycloalkenyl, cycloalkyl and cycloalkenyl each being unsubstituted or mono- or polysubstituted by C₁-C₇-alkyl, or is amino which is disubstituted by C₂-C₆-alkylene;
R₂ is hydrogen, halogen, trifluoromethyl, C₁-C₇-alkyl, C₃-C₇- alkenyl, C₃-C₇-alkynyl, C₁-C₇-alkoxy, C₃-C₇-alkenyloxy, phenyl-C₁-C₇-alkoxy, phenoxy, C₂-C₈-alkanoyloxy, C₃-C₈- alkenoyloxy, or phenyl-C₂-C₈-alkanoyloxy;
R₃ is hydrogen, alkoxycarbonyl or alkenyloxycarbonyl, each of which is unsubstituted or substituted by phenyl, naphthyl, indanyl or fluorenyl, or is cycloalkoxycarbonyl being unsubstituted or mono- or polysubstituted by C₁-C₇-alkyl, or is C₁-C₇- alkanoyl or phenyl-C₂-C₇-alkanoyl, or is benzoyl, naphthoyl, indanoyl or fluorenoyl, or is C₁-C₇-alkanesulfonyl, phenyl-C₁-C₇-alkanesulfonyl, C₃-C₇-cycloalkanesulfonyl, or phenylsulfonyl, or is aminocarbonyl which is substituted by C₁-C₇-alkyl, phenyl-C₁-C₇-alkyl or phenyl;
X₁ and X₃, independently of one another, is O or S;
X₂ is C₂-C₈-alkylene, C₂-C₄-alkylene-phenylene-C₂-C₄-alkylene or C₂-C₄-alkylene-naphthylene-C₂-C₄-alkylene;
wherein the phenyl rings of formula I may be, independently of one another, substituted by one or more substituents selected from halogen, trifluoromethyl, C₁-C₇-alkyl, C₃-C₇-alkenyl, C₃-C₇-alkynyl, hydroxy, C₁-C₇-alkoxy, C₃-C₇- alkenyloxy, phenyl-C₁-C₇-alkoxy, C₂-C₈-alkanoyloxy, C₃-C₇- alkenoyloxy and phenyl-C₂-C₈-alkanoyloxy;
wherein the aromatic radicals in the above definitions may be, independently of one another, substituted by one or more substituents selected from halogen, trifluoromethyl, C₁-C₇- alkyl, C₃-C₇-alkenyl, C₃-C₇-alkynyl, hydroxy, C₁-C₇-alkoxy, C₃-C₇-alkenyloxy phenyl-C₁-C₇-alkoxy, C₂-C₈-alkanoyloxy, C₃-C₈-alkenoyloxy and
phenyl-C₂-C₈-alkanoyloxy; or a pharmaceutically acceptable salt thereof.

3. A process according to claim 1 wherein there are produced compounds of formula I wherein
R₁ is amino which is mono- or disubstituted by a substituent selected from C₁-C₇-alkyl, phenyl-C₁-C₇-alkyl, phenyl and C₃-C₆-cycloalkyl, C₃-C₆-cycloalkyl being unsubstituted or mono- or polysubstituted by C₁-C₇-alkyl, or is amino which is disubstituted by C₃-C₆-alkylene;
R₂ is hydrogen, C₁-C₇-alkoxy or phenyl-C₁-C₄-alkoxy;
R₃ is hydrogen, C₁-C₁₂-aIkoxycarbonyl, C₂-C₅-alkanoyl, phenyl-C₂-C₅-alkanoyl, benzoyl which is unsubstituted or substituted by halogen, trifluoromethyl, C₁-C₇-alkyl, or C₁-C₇-alkoxy, or is C₃-C₆-cycloalkylcarbonyl which is unsubstituted or substituted by C₁-C₇-alkyl, or is naphthoyl, indanoyl or fluorenoyl, or is C₁-C₇alkanesulfonyl, phenyl-C₁-C₇alkanesulfonyl, C₃-C₇-cycloalkanesulfonyl, or phenylsulfonyl, or is aminocarbonyl which is substituted by C₁-C₇-alkyl, phenyl-C₁-C₇-alkyl or phenyl; X₁ and X₃ each are -O-, or furthermore are, independently of one another, -O- or -S-; X₂ is C₂-C₇-alkylene or C₂-C₄-alkylene-phenylene-C₂-C₄-alkylene;
wherein the phenyl rings of formula I may be unsubstituted or, furthermore, independently of one another, substituted by one or more substituents selected from halogen, trifluoromethyl, C₁-C₇-alkyl, and C₁-C₇-alkoxy;
wherein phenyl in the above definitions is unsubstituted or, furthermore, independently of one another, substituted by a substituent selected from halogen, trifluoromethyl, C₁-C₇-alkyl, and C₁-C₇-alkoxy; or a pharmaceutically acceptable salt thereof.

4. A process according to claim 1 wherein there are produced compounds of formula I wherein -CO-R₁ is located in position 4 (para) or 5 (meta); R₂- is located in position 2 (ortho); and -C(=NH)-NH-R₃ is located in position 4 (para) of the corresponding phenyl ring.

5. A process according to claim 1 wherein there are produced compounds of formula IA 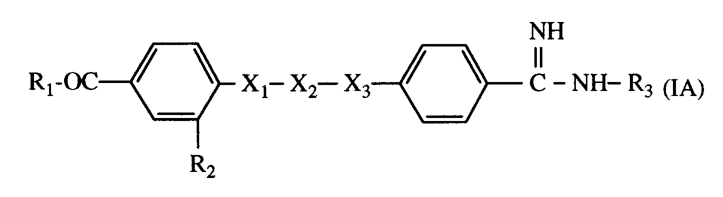 wherein
R₁ is di-C₁-C₄-alkylamino, C₁-C₄-alkyl-phenyl-C₁-C₄-alkyl-amino, di-C₃-C₆-cycloalkylamino which is unsubstituted or substituted by C₁-C₄-alkyl or 1-piperidino substituted by C₁-C₄-alkyl;
R₂ is hydrogen or C₁-C₄-alkoxy;
R₃ is hydrogen, C₁-C₁₂-alkoxycarbonyl, phenyl-C₁-C₄-alkoxycarbonyl, C₂-C₅-alkanoyl, benzoyl which is unsubstituted or substituted by halogen, trifluoromethyl, C₁-C₄-alkyl, or C₁-C₄-alkoxy, C₃-C₆-cycloalkylcarbonyl which is unsubstituted or substituted by C₁-C₄-alkyl;
X₁ and X₃ are -O-;
X₂ is C₄-C₇-alkylene;
wherein the phenyl rings of formula IA may be unsubstituted or, furthermore, independently of one another, substituted by one or more substituents selected from halogen, trifluoromethyl, C₁-C₄-alkyl, and C₁-C₄-alkoxy; or a pharmaceutically acceptable salt thereof.

6. A process according to claim 1 wherein there are produced compounds of formula IA wherein
R₁ is di-C₁-C₄-alkylamino, C₁-C₄-alkyl-phenyl-C₁-C₄-alkyl-amino, di-C₃-C₆-cycloalkylamino which is unsubstituted or substituted by C₁-C₄-alkyl, 1-piperidino substituted by C₁-C₄-alkyl;
R₂ is hydrogen or C₁-C₄-alkoxy;
R₃ is C₁-C₄alkanesulfonyl, phenyl-C₁-C₄-alkanesulfonyl, C₃-C₇-cycloalkane-sulfonyl, or phenylsulfonyl, or is aminocarbonyl which is substituted by C₁-C₄-alkyl, phenyl-C₁-C₄-alkyl or phenyl;
X₁ and X₃ are -O-;
X₂ is C₄-C₇-alkylene; or a pharmaceutically acceptable salt thereof.

7. A process according to claim 1 wherein there are produced compounds of formula IA wherein
R₁ is di-C₁-C₄-alkylamino;
R₂ is hydrogen or C₁-C₄-alkoxy;
R₃ is C₁-C₁₂-alkoxycarbonyl, phenyl-C₁-C₄-alkoxycarbonyl, C₂-C₅-alkanoyl, benzoyl which is unsubstituted or substituted by halogen, trifluoromethyl, C₁-C₄-alkyl, or C₁-C₄-alkoxy, C₃-C₆-cycloalkylcarbonyl which is unsubstituted or substituted by C₁-C₄-alkyl;
X₁ and X₃ are -O-;
X₂ is C₄-C₇-alkylene; or a pharmaceutically acceptable salt thereof.

8. A process according to claim 1 wherein there are produced compounds of formula IA wherein
R₁ is di-C₁-C₄-alkylamino;
R₂ is hydrogen or C₁-C₄-alkoxy;
R₃ is hydrogen;
X₁ and X₃ are -O-;
X₂ is C₄-C₇-alkylene; or a pharmaceutically acceptable salt thereof.

9. A process according to claim 1 wherein there are produced compounds of formula IA wherein
R₁ is di-ethylamino or di-isopropylamino;
R₂ is hydrogen or methoxy;
R₃ is hydrogen;
X₁ and X₃ are -O-;
X₂ is pentylene; or a pharmaceutically acceptable salt thereof.

10. A process according to claim 1 wherein there is produced 4-[5-[4-(aminoiminomethyl)phenoxy]pentoxy]-3-methoxy-N,N-bis(1-methylethyl)benzamide or a pharmaceutically acceptable salt thereof according to claim 1.

11. A process according to claim 1 wherein there is produced 4-[5-[4-(aminoiminomethyl)phenoxy]pentoxy]-3-methoxy-N,N-diethylbenzamide or a pharmaceutically acceptable salt thereof.

12. A process according to claim 1 wherein there is produced 4-[5-[4-(aminoiminomethyl)phenyoxy]pentoxy]-3-methoxy-N-(1-methylethyl)-N-(phenylmethyl)benzamide or a pharmaceutically acceptable salt thereof.

13. A process according to claim 1 wherein there is produced 4-[5-[4-(aminoiminomethyl)phenoxy]pentoxy]-N,N-bis(1-methylethyl)benzamide or a pharmaceutically acceptable salt thereof.

14. A process according to claim 1 wherein there is produced 3-[5-[4-(aminoiminomethyl)phenoxy]pentoxy]-N,N-bis(1-methylethyl)benzamide or a pharmaceutically acceptable salt thereof.

15. A process according to claim 1 wherein there is produced 4-[5-[4-(aminoiminomethyl)phenoxy]pentoxy]-2-hydroxy-N,N-bis(1-methylethyl)benzamide or a pharmaceutically acceptable salt thereof.

16. A process according to claim 1 wherein there is produced 4-[5-[4-[(benzoylamino)iminomethyl]phenoxy]pentoxy]-3-methoxy-N,N-bis(1-methylethyl)benzamide or a pharmaceutically acceptable salt thereof.

17. A process according to claim 1 wherein there is produced 4-[5-[4-[imino[octyloxycarbonyl]amino]methyl]phenoxy]pentoxy]-3-methoxy-N,N-bis(1-methylethyl)benzamide or a pharmaceutically acceptable salt thereof.

18. A process according to claim 1 wherein there is produced 4-[5-[4-[imino[octyloxycarbonyl]amino]methyl]phenoxy]pentoxy]-N,N-bis(1-methylethyl)benzamide or a pharmaceutically acceptable salt thereof.

19. A process according to claim 1 wherein there is produced 4-[5-[4-[imino(phenylmethoxycarbonylamino)methyl]phenoxy]pentoxy]-3-methoxy-N,N-bis(1-methylethyl)benzamide or a pharmaceutically acceptable salt thereof.

## Patentansprüche (Patentansprüche für folgende(n) Vertragsstaat(en): AT, BE, CH, DE, DK, FR, GB, IT, LI, LU, NL, PT, SE)

1. Verbindung der Formel 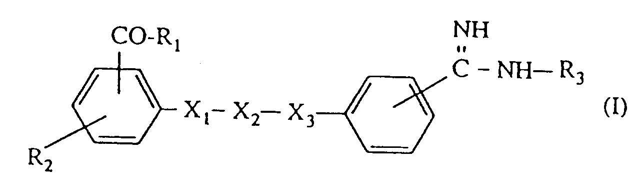 worin:
R₁ Amino darstellt, das mono- oder disubstituiert ist mit einem Substituenten, ausgewählt aus einem aliphatischen Kohlenwasserstoffrest, einem araliphatischen Kohlenwasserstoffrest, einem aromatischen Rest und einem cycloaliphatischen Kohlenwasserstoffrest oder Amino ist, das disubstituiert ist mit einem zweiwertigen aliphatischen Kohlenwasserstoffrest;
R₂ Wasserstoff, Halogen, Trifluormethyl, einen aliphatischen Kohlenwasserstoffrest darstellt oder Hydroxy ist, das mit einem aliphatischen Alkohol, araliphatischen Alkohol oder aromatischen Alkohol verethert ist oder das mit einer aliphatischen oder araliphatischen Carbonsäure verestert ist;
R₃ Wasserstoff oder einen Acylrest darstellt, der von einer organischen Kohlensäure, einer organischen Carbonsäure, einer Sulfonsäure oder einer Carbaminsäure abgeleitet ist;
X₁ und X₃ unabhängig voneinander Sauerstoff (-O-) oder Schwefel (-S-) darstellen; und
X₂ einen zweiwertigen aliphatischen Kohlenwasserstoffrest darstellt, der durch einen aromatischen Rest unterbrochen sein kann;
wobei die Phenylringe der Formel I unabhängig voneinander weiterhin substituiert sein können mit einem oder mehreren Substituenten, ausgewählt aus Halogen, Trifluormethyl, einem aliphatischen Kohlenwasserstoffrest, Hydroxy und Hydroxy, das mit einem aliphatischen Alkohol verethert ist oder mit einer aliphatischen oder araliphatischen Carbonsäure verestert ist;
wobei Aryl in den vorstehenden Definitionen unabhängig voneinander zusätzlich substituiert sein kann mit einem oder mehreren Substituenten, ausgewählt aus Halogen, Trifluormethyl, einem aliphatischen Kohlenwasserstoffrest, Hydroxy und Hydroxy, das mit einem aliphatischen Alkohol verethert ist oder mit einer aliphatischen oder araliphatischen Carbonsäure verestert ist;
wobei ein cycloaliphatischer Kohlenwasserstoffrest mit einem aliphatischen Rest substituiert sein kann; oder ein pharmazeutisch verträgliches Salz davon.

2. Verbindung nach Anspruch 1, worin
R₁ Amino, das mono- oder disubstituiert ist mit einem Substituenten, ausgewählt aus C₁-C₇-Alkyl, C₃-C₇-Alkenyl, C₃-C₇-Alkinyl, Phenyl-C₁-C₇-alkyl, Phenyl-C₂-C₇-alkenyl, Phenyl-C₂-C₇-alkinyl, Phenyl, Naphthyl, Indanyl, Fluorenyl, Cycloalkyl und Cycloalkenyl, Cycloalkyl und Cycloalkenyl, jeweils unsubstituiert oder mono- oder polysubstituiert mit C₁-C₇-Alkyl, oder Amino darstellt, das mit C₂-C₆-Alkylen disubstituiert ist;
R₂ Wasserstoff, Halogen, Trifluormethyl, C₁-C₇-Alkyl, C₃-C₇-Alkenyl, C₃-C₇-Alkinyl, C₁-C₇-Alkoxy, C₃-C₇-Alkenyloxy, Phenyl-C₁-C₇-alkoxy, Phenoxy, C₂-C₈-Alkanoyloxy, C₃-C₈-Alkenoyloxy oder Phenyl-C₂-C₈-alkanoyloxy darstellt;
R₃ Wasserstoff, Alkoxycarbonyl oder Alkenyloxycarbonyl darstellt, jeweils unsubstituiert oder substituiert mit Phenyl, Naphthyl, Indanyl oder Fluorenyl oder Cycloalkoxycarbonyl ist, das unsubstituiert oder mono- oder polysubstituiert mit C₁-C₇-Alkyl ist oder C₁-C₇-Alkanoyl oder Phenyl-C₂-C₇-alkanoyl ist oder Benzoyl, Naphthoyl, Indanoyl oder Fluorenoyl ist, oder C₁-C₇-Alkansulfonyl, Phenyl-C₁-C₇-alkansulfonyl, C₃-C₇-Cycloalkansulfonyl oder Phenylsulfonyl ist oder Aminocarbonyl ist, das mit C₁-C₇-Alkyl, Phenyl-C₁-C₇-alkyl oder Phenyl substituiert ist;
X₁ und X₃ unabhängig voneinander O oder S sind;
X₂ C₂-C₈-Alkylen, C₂-C₄-Alkylenphenylen-C₂-C₄-alkylen oder C₂-C₄-Alkylennaphthylen-C₂-C₄-alkylen ist;
wobei die Phenylringe der Formel I unabhängig voneinander mit einem oder mehreren Substituenten substituiert sein können, ausgewählt aus Halogen, Trifluormethyl, C₁-C₇-Alkyl, C₃-C₇-Alkenyl, C₃-C₇-Alkinyl, Hydroxy, C₁-C₇-Alkoxy, C₃-C₇-Alkenyloxy, Phenyl-C₁-C₇-alkoxy, C₂-C₈-Alkanoyloxy, C₃-C₇-Alkenoyloxy und Phenyl-C₂-C₈-alkanoyloxy;
wobei die aromatischen Reste in den vorstehenden Definitionen unabhängig voneinander mit einem oder mehreren Substituenten substituiert sein können, ausgewählt aus Halogen, Trifluormethyl, C₁-C₇-Alkyl, C₃-C₇-Alkenyl, C₃-C₇-Alkinyl, Hydroxy, C₁-C₇-Alkoxy, C₃-C₇-Alkenyloxy, Phenyl-C₁-C₇-alkoxy, C₂-C₈-Alkanoyloxy, C₃-C₈-Alkenoyloxy und Phenyl-C₂-C₈-alkanoyloxy oder ein pharmazeutisch verträgliches Salz davon.

3. Verbindung nach Anspruch 1, worin
R₁ Amino darstellt, das mono- oder disubstituiert ist mit einem Substituenten, ausgewählt aus C₁-C₇-Alkyl, Phenyl-C₁-C₇-alkyl, Phenyl und C₃-C₆-Cycloalkyl, C₃-C₆-Cycloalkyl, unsubstituiert oder mono- oder polysubstituiert mit einem C₁-C₇-Alkyl oder Amino ist, das mit C₃-C₆-Alkylen disubstituiert ist;
R₂ Wasserstoff, C₁-C₇-Alkoxy oder Phenyl-C₁-C₄-alkoxy darstellt;
R₃ Wasserstoff, C₁-C₁₂-Alkoxycarbonyl, C₂-C₅-Alkanoyl, Phenyl-C₂-C₅-alkanoyl, Benzoyl, das unsubstituiert oder substituiert ist mit Halogen, Trifluormethyl, C₁-C₇-Alkyl oder C₁-C₇-Alkoxy, oder C₃-C₆-Cycloalkylcarbonyl ist, das unsubstituiert oder substituiert ist mit C₁-C₇-Alkyl oder Naphthoyl, Indanoyl oder Fluorenoyl oder C₁-C₇-Alkansulfonyl, Phenyl-C₁-C₇-alkansulfonyl, C₃-C₇-Cycloalkansulfonyl oder Phenylsulfonyl ist oder Aminocarbonyl ist, das mit C₁-C₇-Alkyl, Phenyl-C₁-C₇-alkyl oder Phenyl substituiert ist;
X₁ und X₃ jeweils -O- sind oder außerdem unabhängig voneinander -O- oder -S- sind;
X₂ C₂-C₇-Alkylen oder C₂-C₄-Alkylenphenylen-C₂-C₄-alkylen ist;
wobei die Phenylringe der Formel I unsubstituiert sein können oder außerdem unabhängig voneinander mit einem oder mehreren Substituenten substituiert sind, ausgewählt aus Halogen, Trifluormethyl, C₁-C₇-Alkyl und C₁-C₇-Alkoxy;
wobei Phenyl in den vorstehenden Definitionen unsubstituiert ist oder außerdem unabhängig voneinander mit einem Substituenten substituiert ist, ausgewählt aus Halogen, Trifluormethyl, C₁-C₇-Alkyl und C₁-C₇-Alkoxy; oder ein pharmazeutisch verträgliches Salz davon.

4. Verbindung nach Anspruch 1, worin
-CO-R₁ in Stellung 4 (para) oder 5 (meta) angeordnet ist; R₂- in Stellung 2 (ortho) angeordnet ist; und -C(=NH)-NH-R₃ in Stellung 4 (para) des entsprechenden Phenylrings angeordnet ist.

5. Verbindung nach Anspruch 1 der Formel IA 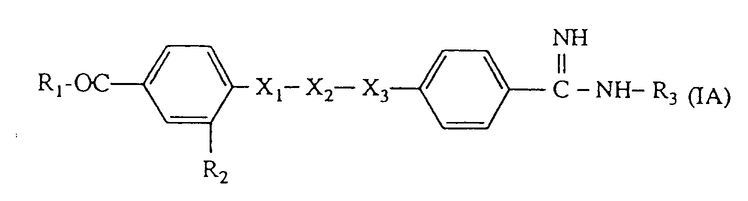 worin
R₁ Di-C₁-C₄-alkylamino, C₁-C₄-Alkylphenyl-C₁-C₄-alkylamino, Di-C₃-C₆-cycloalkylamino darstellt, das unsubstituiert oder substituiert ist mit C₁-C₄-Alkyl oder 1-Piperidino, substituiert mit C₁-C₄-Alkyl;
R₂ Wasserstoff oder C₁-C₄-Alkoxy darstellt;
R₃ Wasserstoff, C₁-C₁₂-Alkoxycarbonyl, Phenyl-C₁-C₄-alkoxycarbonyl, C₂-C₅-Alkanoyl, Benzoyl, das unsubstituiert oder substituiert ist mit Halogen, Trifluormethyl, C₁-C₄-Alkyl, oder C₁-C₄-Alkoxy, C₃-C₆-Cycloalkylcarbonyl, das unsubstituiert oder substituiert mit C₁-C₄-Alkyl ist, bedeutet;
X₁ und X₃ -O- sind;
X₂ C₄-C₇-Alkylen darstellt;
wobei die Phenylringe der Formel IA unsubstituiert oder außerdem unabhängig voneinander substituiert sein können mit einem oder mehreren Substituenten, ausgewählt aus Halogen, Trifluormethyl, C₁-C₄-Alkyl und C₁-C₄-Alkoxy; oder ein pharmazeutisch verträgliches Salz davon.

6. Verbindung nach Anspruch 1 der Formel IA, worin
R₁ Di-C₁-C₄-alkylamino, C₁-C₄-Alkylphenyl-C₁-C₄-alkylamino, Di-C₃-C₆-cycloalkylamino, das unsubstituiert oder substituiert ist mit C₁-C₄-Alkyl, 1-Piperidino, substituiert mit C₁-C₄-Alkyl, darstellt;
R₂ Wasserstoff oder C₁-C₄-Alkoxy darstellt;
R₃ C₁-C₄-Alkansulfonyl, Phenyl-C₁-C₄-alkansulfonyl, C₃-C₇-Cycloalkansulfonyl oder Phenylsulfonyl darstellt oder Aminocarbonyl darstellt, das substituiert ist mit C₁-C₄-Alkyl, Phenyl-C₁-C₄-alkyl oder Phenyl;
X₁ und X₃ -O- sind;
X₂ C₄-C₇-Alkylen darstellt; oder ein pharmazeutisch verträgliches Salz davon.

7. Verbindung nach Anspruch 5 der Formel IA, worin
R₁ Di-C₁-C₄-alkylamino darstellt;
R₂ Wasserstoff oder C₁-C₄-Alkoxy darstellt;
R₃ C₁-C₁₂-Alkoxycarbonyl, Phenyl-C₁-C₄-alkoxycarbonyl, C₂-C₅-Alkanoyl, Benzoyl, das unsubstituiert oder substituiert ist mit Halogen, Trifluormethyl, C₁-C₄-Alkyl, oder C₁-C₄-Alkoxy, C₃-C₆-Cycloalkylcarbonyl, das unsubstituiert oder substituiert ist mit C₁-C₄-Alkyl, darstellt;
X₁ und X₃ -O- sind;
X₂ C₄-C₇-Alkylen darstellt; oder ein pharmazeutisch verträgliches Salz davon.

8. Verbindung nach Anspruch 5 der Formel IA, worin
R₁ Di-C₁-C₄-alkylamino darstellt;
R₂ Wasserstoff oder C₁-C₄-Alkoxy darstellt;
R₃ Wasserstoff darstellt;
X₁ und X₃ -O- sind;
X₂ C₄-C₇-Alkylen darstellt; oder ein pharmazeutisch verträgliches Salz davon.

9. Verbindung nach Anspruch 5 der Formel IA, worin
R₁ Diethylamino oder Diisopropylamino darstellt;
R₂ Wasserstoff oder Methoxy darstellt;
R₃ Wasserstoff darstellt;
X₁ und X₃ -O- sind;
X₂ Pentylen darstellt; oder ein pharmazeutisch verträgliches Salz davon.

10. 4-[5-[4-(Aminoiminomethyl)phenoxy]pentoxy]-3-methoxy-N,N-bis(1-methylethyl)benzamid oder ein pharmazeutisch verträgliches Salz davon nach Anspruch 1.

11. 4-[5-[4-(Aminoiminomethyl)phenoxy]pentoxy]-3-methoxy-N,N-diethylbenzamid oder ein pharmazeutisch verträgliches Salz davon nach Anspruch 1.

12. 4-[5-[4-(Aminoiminonethyl)phenoxy]pentoxy]-3-methoxy-N-(1-methylethyl)-N-(phenylmethyl)benzamid oder ein pharmazeutisch verträgliches Salz davon nach Anspruch 1.

13. 4-[5-[4-(Aminoiminomethyl)phenoxy]pentoxy]-N,N-bis(1-methylethyl)benzamid oder ein pharmazeutisch verträgliches Salz davon nach Anspruch 1.

14. 3-[5-[4-(Aminoiminomethyl)phenoxy]pentoxy]-N,N-bis(1-methylethyl)benzamid oder ein pharmazeutisch verträgliches Salz davon nach Anspruch 1.

15. 4-[5-[4-(Aminoiminomethyl)phenoxy]pentoxy]-2-hydroxy-N,N-bis(1-methylethyl)benzamid oder ein pharmazeutisch verträgliches Salz davon nach Anspruch 1.

16. 4-[5-[4-[(Benzoylamino)iminomethyl]phenoxy]pentoxy]-3-methoxy-N,N-bis-(1-methylethyl)benzamid oder ein pharmazeutisch verträgliches Salz davon nach Anspruch 1.

17. 4-[5-[4-[Imino[octyloxycarbonyl]amino]methyl]phenoxy]pentoxy] -3-methoxy -N,N-bis-(1-methylethyl)benzamid oder ein pharmazeutisch verträgliches Salz davon nach Anspruch 1.

18. 4-[5-[4-[Imino[octyloxycarbonyl]amino]methyl]phenoxy]pentoxy]-N,N-bis(1-methylethyl)benzamid oder ein pharmazeutisch verträgliches Salz davon nach Anspruch 1.

19. 4-[5-[4-[Imino(phenylmethoxycarbonylamino)methyl]phenoxy]pentoxy] -3-methoxy -N,N-bis(1-methylethyl)benzamid oder ein pharmazeutisch verträgliches Salz davon nach Anspruch 1.

20. Verfahren zur Herstellung von Verbindungen definiert nach Formel I in Anspruch 1 und pharmazeutisch verträgliche Salze davon, umfassend
a) Umwandeln in einer Verbindung der Formel IIa 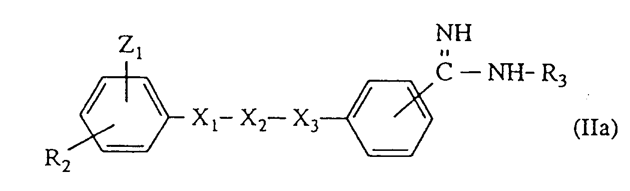 oder einem Salz davon, worin Z₁ ein Rest ist, der in den variablen Rest -CO-R₁ umgewandelt werden kann,
von Z₁ in den variablen Rest -CO-R₁, oder
b) zur Herstellung von Verbindungen der Formel I, worin R₃ Wasserstoff darstellt, in einer Verbindung der Formel IIIa 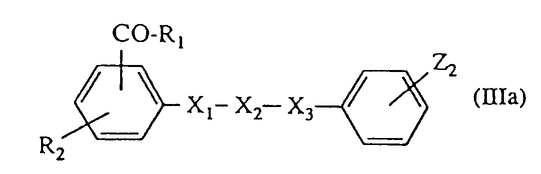 oder einem Salz davon, worin Z₂ ein Rest ist, der in den variablen Rest -C(=NH)-NH-R₃ umgewandelt werden kann, Umwandeln von Z₂ in den variablen Rest -C(=NH)-NH-R₃, oder
c) Umsetzen einer Verbindung der Formel IVa 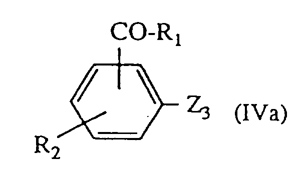 oder eines Salzes davon mit einer Verbindung der Formel IVb 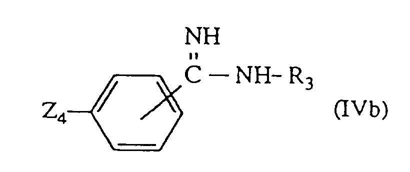 oder einem Salz davon, worin Z₃ eine Gruppe der Formel -X₁-X₂-Z₅ darstellt und Z₄ -Z₆ darstellt, oder Z₃ -Z₆ darstellt und Z₄ eine Gruppe der Formel Z₅-X₂-X₃- darstellt, worin einer der Reste Z₅ und Z₆ Hydroxy oder Mercapto darstellt und der andere Hydroxy, Mercapto oder reaktiv verestertes Hydroxy darstellt, und, falls erwünscht, Umwandeln einer Verbindung der Formel I oder eines Salzes davon, erhältlich nach dem Verfahren oder in anderer Weise in eine andere Verbindung oder ein Salz davon gemäß der Erfindung, Umwandeln einer freien Verbindung der Formel I, erhältlich nach dem Verfahren, in ein Salz, Umwandeln eines Salzes, erhältlich nach dem Verfahren, in die freie Verbindung der Formel I oder in ein anderes Salz oder erneutes Lösen eines Isomerengemisches, erhältlich nach dem Verfahren und Isolieren der gewünschten Verbindung.

21. Pharmazeutische Zubereitung, enthaltend eine Verbindung der Formel I nach Anspruch 1 und übliche Träger.

22. Verwendung von Verbindungen der Formel I nach Anspruch 1 oder Salzen davon und üblichen Trägern zur Herstellung pharmazeutischer Zubereitungen.

23. Verbindung der Formel I nach Anspruch 1 oder Salze davon zur Verwendung in einem Verfahren zur therapeutischen Behandlung des menschlichen oder tierischen Körpers.

24. Verbindungen der Formel I nach Anspruch 1 oder Salze davon zur Verwendung als Antagonist von LTB₄ auf polymorphkernige Human-Leukozyten.

25. Pharmazeutische Zusammensetzung, geeignet zur Gegenwirkung für LTB₄-Rezeptoren bei Säugern, umfassend eine wirksame LTB₄-Rezeptoren entgegenwirkende Menge der Verbindung nach Anspruch 1 oder eines pharmazeutisch verträglichen Salzes davon und einen Träger.

## Patentansprüche (Patentansprüche für folgende(n) Vertragsstaat(en): ES, GR)

1. Verfahren zur Herstellung einer Verbindung der Formel 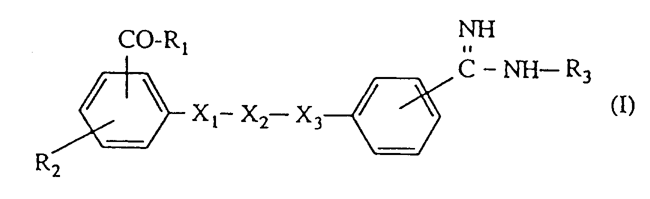 worin:
R₁ Amino darstellt, das mono- oder disubstituiert ist mit einem Substituenten, ausgewählt aus einem aliphatischen Kohlenwasserstoffrest, einem araliphatischen Kohlenwasserstoffrest, einem aromatischen Rest und einem cycloaliphatischen Kohlenwasserstoffrest oder Amino ist, das disubstituiert ist mit einem zweiwertigen aliphatischen Kohlenwasserstoffrest;
R₂ Wasserstoff, Halogen, Trifluormethyl, einen aliphatischen Kohlenwasserstoffrest darstellt oder Hydroxy ist, das mit einem aliphatischen Alkohol, araliphatischen Alkohol oder aromatischen Alkohol verethert ist oder das mit einem aliphatischen oder araliphatischen Carbonsäurerest verestert ist;
R₃ Wasserstoff oder einen Acylrest darstellt, der von einer organischen Kohlensäure, einer organischen Carbonsäure, einer Sulfonsäure oder einer Carbaminsäure abgeleitet ist;
X₁ und X₃ unabhängig voneinander Sauerstoff (-O-) oder Schwefel (-S-) darstellen; und
X₂ einen zweiwertigen aliphatischen Kohlenwasserstoffrest darstellt, der durch einen aromatischen Rest unterbrochen sein kann;
wobei die Phenylringe der Formel I unabhängig voneinander weiterhin substituiert sein können mit einem oder mehreren Substituenten, ausgewählt aus Halogen, Trifluormethyl, einem aliphatischen Kohlenwasserstoffrest, Hydroxy und Hydroxy, das mit einem aliphatischen Alkohol verethert ist oder mit einer aliphatischen oder araliphatischen Carbonsäure verestert ist;
wobei Aryl in den vorstehenden Definitionen unabhängig voneinander zusätzlich substituiert sein kann mit einem oder mehreren Substituenten, ausgewählt aus Halogen, Trifluormethyl, einem aliphatischen Kohlenwasserstoffrest, Hydroxy und Hydroxy, das mit einem aliphatischen Alkohol verethert ist oder mit einer aliphatischen oder araliphatischen Carbonsäure verestert ist;
wobei ein cycloaliphatischer Kohlenwasserstoffrest mit einem aliphatischen Rest substituiert sein kann; oder ein pharmazeutisch verträgliches Salz davon, umfassend
a) Umwandeln in einer Verbindung der Formel IIa 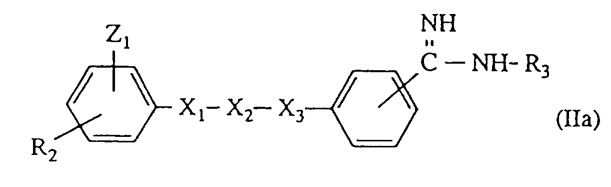 oder einem Salz davon, worin Z₁ ein Rest ist, der in den variablen Rest -CO-R₁ umgewandelt werden kann,
von Z₁ in den variablen Rest -CO-R₁, oder
b) zur Herstellung von Verbindungen der Formel I, worin R₃ Wasserstoff darstellt, in einer Verbindung der Formel IIIa 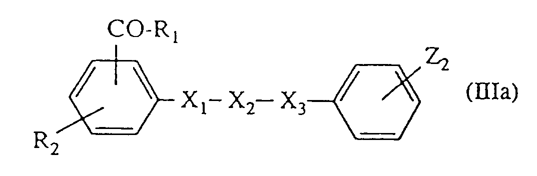 oder einem Salz davon, worin Z₂ ein Rest ist, der in den variablen Rest -C(=NH)-NH-R₃ umgewandelt werden kann, Umwandeln von Z₂ in den variablen Rest -C(=NH)-NH-R₃, oder
c) Umsetzen einer Verbindung der Formel IVa 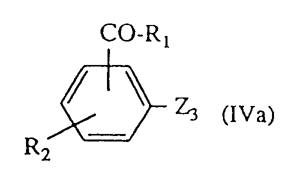 oder eines Salzes davon mit einer Verbindung der Formel IVb 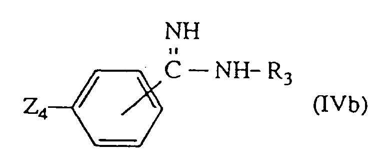 oder einem Salz davon, worin Z₃ eine Gruppe der Formel -X₁-X₂-Z₅ darstellt und Z₄ -Z₆ darstellt, oder Z₃ -Z₆ darstellt und Z₄ eine Gruppe der Formel Z₅-X₂-X₃- darstellt, worin einer der Reste Z₅ und Z₆ Hydroxy oder Mercapto darstellt und der andere Hydroxy, Mercapto oder reaktiv verestertes Hydroxy darstellt, und, falls erwünscht, Umwandeln einer Verbindung der Formel I oder eines Salzes davon, erhältlich nach dem Verfahren oder in anderer Weise in eine andere Verbindung oder ein Salz davon gemäß der Erfindung, Umwandeln einer freien Verbindung der Formel I, erhältlich nach dem Verfahren, in ein Salz, Umwandeln eines Salzes, erhältlich nach dem Verfahren, in die freie Verbindung der Formel I oder in ein anderes Salz oder erneutes Lösen eines Isomerengemisches, erhältlich nach dem Verfahren und Isolieren der gewünschten Verbindung.

2. Verfahren nach Anspruch 1, wobei Verbindungen der Formel I hergestellt werden, worin R₁ Amino, das mono- oder disubstituiert ist mit einem Substituenten, ausgewählt aus C₁-C₇-Alkyl, C₃-C₇-Alkenyl, C₃-C₇-Alkinyl, Phenyl-C₁-C₇-alkyl, Phenyl-C₂-C₇-alkenyl, Phenyl-C₂-C₇-alkinyl, Phenyl, Naphthyl, Indanyl, Fluorenyl, Cycloalkyl und Cycloalkenyl, Cycloalkyl und Cycloalkenyl, jeweils unsubstituiert oder mono- oder polysubstituiert mit C₁-C₇-Alkyl, oder Amino darstellt, das mit C₂-C₆-Alkylen disubstituiert ist;
R₂ Wasserstoff, Halogen, Trifluormethyl, C₁-C₇-Alkyl, C₃-C₇-Alkenyl, C₃-C₇-Alkinyl, C₁-C₇-Alkoxy, C₃-C₇-Alkenyloxy, Phenyl-C₁-C₇-alkoxy, Phenoxy, C₂-C₈-Alkanoyloxy, C₃-C₈-Alkenoyloxy oder Phenyl-C₂-C₈-alkanoyloxy darstellt;
R₃ Wasserstoff, Alkoxycarbonyl oder Alkenyloxycarbonyl darstellt, jeweils unsubstituiert oder substituiert mit Phenyl, Naphthyl, Indanyl oder Fluorenyl oder Cycloalkoxycarbonyl ist, das unsubstituiert oder mono- oder polysubstituiert mit C₁-C₇-Alkyl ist oder C₁-C₇-Alkanoyl oder Phenyl-C₂-C₇-alkanoyl ist oder Benzoyl, Naphthoyl, Indanoyl oder Fluorenoyl ist, oder C₁-C₇-Alkansulfonyl, Phenyl-C₁-C₇-alkansulfonyl, C₃-C₇-Cycloalkansulfonyl oder Phenylsulfonyl ist oder Aminocarbonyl ist, das mit C₁-C₇-Alkyl, Phenyl-C₁-C₇-alkyl oder Phenyl substituiert ist;
X₁ und X₃ unabhängig voneinander O oder S sind;
X₂ C₂-C₈-Alkylen, C₂-C₄-Alkylenphenylen-C₂-C₄-alkylen oder C₂-C₄-Alkylennaphthylen-C₂-C₄-alkylen ist;
wobei die Phenylringe der Formel I unabhängig voneinander mit einem oder mehreren Substituenten substituiert sein können, ausgewählt aus Halogen, Trifluormethyl, C₁-C₇-Alkyl, C₃-C₇-Alkenyl, C₃-C₇-Alkinyl, Hydroxy, C₁-C₇-Alkoxy, C₃-C₇-Alkenyloxy, Phenyl-C₁-C₇-alkoxy, C₂-C₈-Alkanoyloxy, C₃-C₇-Alkenoyloxy und Phenyl-C₂-C₈-alkanoyloxy;
wobei die aromatischen Reste in den vorstehenden Definitionen unabhängig voneinander mit einem oder mehreren Substituenten substituiert sein können, ausgewählt aus Halogen, Trifluormethyl, C₁-C₇-Alkyl, C₃-C₇-Alkenyl, C₃-C₇-Alkinyl, Hydroxy, C₁-C₇-Alkoxy, C₃-C₇-Alkenyloxy, Phenyl-C₁-C₇-alkoxy, C₂-C₈-Alkanoyloxy, C₃-C₈-Alkenoyloxy und Phenyl-C₂-C₈-alkanoyloxy oder ein pharmazeutisch verträgliches Salz davon.

3. Verfahren nach Anspruch 1, wobei Verbindungen der Formel I hergestellt werden, worin R₁ Amino darstellt, das mono- oder disubstituiert ist mit einem Substituenten, ausgewählt aus C₁-C₇-Alkyl, Phenyl-C₁-C₇-alkyl, Phenyl und C₃-C₆-Cycloalkyl, C₃-C₆-Cycloalkyl, unsubstituiert oder mono- oder polysubstituiert mit einem C₁-C₇-Alkyl oder Amino ist, das mit C₃-C₆-Alkylen disubstituiert ist;
R₂ Wasserstoff, C₁-C₇-Alkoxy oder Phenyl-C₁-C₄-alkoxy darstellt;
R₃ Wasserstoff, C₁-C₁₂-Alkoxycarbonyl, C₂-C₅-Alkanoyl, Phenyl-C₂-C₅-alkanoyl, Benzoyl, das unsubstituiert oder substituiert ist mit Halogen, Trifluormethyl, C₁-C₇-Alkyl oder C₁-C₇-Alkoxy, oder C₃-C₆-Cycloalkylcarbonyl ist, das unsubstituiert oder substituiert ist mit C₁-C₇-Alkyl oder Naphthoyl, Indanoyl oder Fluorenoyl oder C₁-C₇-Alkansulfonyl, Phenyl-C₁-C₇-alkansulfonyl, C₃-C₇-Cycloalkansulfonyl oder Phenylsulfonyl ist oder Aminocarbonyl ist, das mit C₁-C₇-Alkyl, Phenyl-C₁-C₇-alkyl oder Phenyl substituiert ist;
X₁ und X₃ jeweils -O- sind oder außerdem unabhängig voneinander -O- oder -S- sind;
X₂ C₂-C₇-Alkylen oder C₂-C₄-Alkylenphenylen-C₂-C₄-alkylen ist;
wobei die Phenylringe der Formel I unsubstituiert sein können oder außerdem unabhängig voneinander mit einem oder mehreren Substituenten substituiert sind, ausgewählt aus Halogen, Trifluormethyl, C₁-C₇-Alkyl und C₁-C₇-Alkoxy;
wobei Phenyl in den vorstehenden Definitionen unsubstituiert ist oder außerdem unabhängig voneinander mit einem Substituenten substituiert ist, ausgewählt aus Halogen, Trifluormethyl, C₁-C₇-Alkyl und C₁-C₇-Alkoxy; oder ein pharmazeutisch verträgliches Salz davon.

4. Verfahren nach Anspruch 1, wobei Verbindungen der Formel I hergestellt werden, worin
-CO-R₁ in Stellung 4 (para) oder 5 (meta) angeordnet ist; R₂- in Stellung 2 (ortho) angeordnet ist; und -C(=NH)-NH-R₃ in Stellung 4 (para) des entsprechenden Phenylrings angeordnet ist.

5. Verfahren nach Anspruch 1, wobei Verbindungen der Formel IA 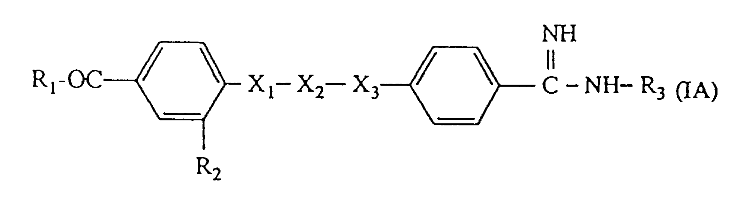 hergestellt werden, worin
R₁ Di-C₁-C₄-alkylamino, C₁-C₄-Alkylphenyl-C₁-C₄-alkylamino, Di-C₃-C₆-cycloalkylamino darstellt, das unsubstituiert oder substituiert ist mit C₁-C₄-Alkyl oder 1-Piperidino, substituiert mit C₁-C₄-Alkyl;
R₂ Wasserstoff oder C₁-C₄-Alkoxy darstellt;
R₃ Wasserstoff, C₁-C₁₂-Alkoxycarbonyl, Phenyl-C₁-C₄-alkoxycarbonyl, C₂-C₅-Alkanoyl, Benzoyl, das unsubstituiert oder substituiert ist mit Halogen, Trifluormethyl, C₁-C₄-Alkyl, oder C₁-C₄-Alkoxy, C₃-C₆-Cycloalkylcarbonyl, das unsubstituiert oder substituiert mit C₁-C₄-Alkyl ist, bedeutet;
X₁ und X₃ -O- sind;
X₂ C₄-C₇-Alkylen darstellt;
wobei die Phenylringe der Formel IA unsubstituiert oder außerdem unabhängig voneinander substituiert sein können mit einem oder mehreren Substituenten, ausgewählt aus Halogen, Trifluormethyl, C₁-C₄-Alkyl und C₁-C₄-Alkoxy; oder ein pharmazeutisch verträgliches Salz davon.

6. Verfahren nach Anspruch 1, wobei Verbindungen der Formel IA hergestellt werden, worin
R₁ Di-C₁-C₄-alkylamino, C₁-C₄-Alkylphenyl-C₁-C₄-alkylamino, Di-C₃-C₆-cycloalkylamino, das unsubstituiert oder substituiert ist mit C₁-C₄-Alkyl, 1-Piperidino, substituiert mit C₁-C₄-Alkyl, darstellt;
R₂ Wasserstoff oder C₁-C₄-Alkoxy darstellt;
R₃ C₁-C₄-Alkansulfonyl, Phenyl-C₁-C₄-alkansulfonyl, C₃-C₇-Cycloalkansulfonyl oder Phenylsulfonyl darstellt oder Aminocarbonyl darstellt, das substituiert ist mit C₁-C₄-Alkyl, Phenyl-C₁-C₄-alkyl oder Phenyl;
X₁ und X₃ -O- sind;
X₂ C₄-C₇-Alkylen darstellt; oder ein pharmazeutisch verträgliches Salz davon.

7. Verfahren nach Anspruch 1, wobei Verbindungen der Formel IA hergestellt werden, worin
R₁ Di-C₁-C₄-alkylamino darstellt;
R₂ Wasserstoff oder C₁-C₄-Alkoxy darstellt;
R₃ C₁-C₁₂-Alkoxycarbonyl, Phenyl-C₁-C₄-alkoxycarbonyl, C₂-C₅-Alkanoyl, Benzoyl, das unsubstituiert oder substituiert ist mit Halogen, Trifluormethyl, C₁-C₄-Alkyl, oder C₁-C₄-Alkoxy, C₃-C₆-Cycloalkylcarbonyl, das unsubstituiert oder substituiert ist mit C₁-C₄-Alkyl, darstellt;
X₁ und X₃ -O- sind;
X₂ C₄-C₇-Alkylen darstellt; oder ein pharmazeutisch verträgliches Salz davon.

8. Verfahren nach Anspruch 1, wobei Verbindungen der Formel IA hergestellt werden, worin
R₁ Di-C₁-C₄-alkylamino darstellt;
R₂ Wasserstoff oder C₁-C₄-Alkoxy darstellt;
R₃ Wasserstoff darstellt;
X₁ und X₃ -O- sind;
X₂ C₄-C₇-Alkylen darstellt; oder ein pharmazeutisch verträgliches Salz davon.

9. Verfahren nach Anspruch 1, wobei Verbindungen der Formel IA hergestellt werden, worin
R₁ Diethylamino oder Diisopropylamino darstellt;
R₂ Wasserstoff oder Methoxy darstellt;
R₃ Wasserstoff darstellt;
X₁ und X₃ -O- sind;
X₂ Pentylen darstellt; oder ein pharmazeutisch verträgliches Salz davon.

10. Verfahren nach Anspruch 1, wobei 4-[5-[4-(Aminoiminomethyl)phenoxy]pentoxy] -3- methoxy -N,N- bis (1-methylethyl)benzamid oder ein pharmazeutisch verträgliches Salz davon nach Anspruch 1 hergestellt wird.

11. Verfahren nach Anspruch 1, wobei 4-[5-[4-(Aminoiminomethyl)phenoxy]pentoxy] -3-methoxy -N,N- diethylbenzamid oder ein pharmazeutisch verträgliches Salz davon hergestellt wird.

12. Verfahren nach Anspruch 1, wobei 4-[5-[4-(Aminoiminomethyl)phenoxy]pentoxy]-3-methoxy-N-(1-methylethyl)-N-(phenylmethyl)benzamid oder ein pharmazeutisch verträgliches Salz davon hergestellt wird.

13. Verfahren nach Anspruch 1, wobei 4-[5-[4-(Aminoiminomethyl)phenoxy]pentoxy] -N,N-bis-(1-methylethyl)benzamid oder ein pharmazeutisch verträgliches Salz davon hergestellt wird.

14. Verfahren nach Anspruch 1, wobei 3-[5-[4-(Aminoiminomethyl)phenoxy]pentoxy] -N,N-bis-(1-methylethyl)benzamid oder ein pharmazeutisch verträgliches Salz davon hergestellt wird.

15. Verfahren nach Anspruch 1, wobei 4-[5-[4-(Aminoiminomethyl) phenoxy] pentoxy] -2- hydroxy -N,N-bis(1-methylethyl)benzamid oder ein pharmazeutisch verträgliches Salz davon hergestellt wird.

16. Verfahren nach Anspruch 1, wobei 4-[5-[4-[(Benzoylamino)iminomethyl]phenoxy]pentoxy]-3-methoxy-N,N-bis-(1-methylethyl)benzamid oder ein pharmazeutisch verträgliches Salz davon hergestellt wird.

17. Verfahren nach Anspruch 1, wobei 4-[5-[4-[Imino[octyloxycarbonyl]amino]methyl]-phenoxy]pentoxy]-3-methoxy-N,N-bis(1-methylethyl)benzamid oder ein pharmazeutisch verträgliches Salz davon hergestellt wird.

18. Verfahren nach Anspruch 1, wobei 4-[5-[4-[Imino[octyloxycarbonyl]amino]methyl]phenoxy]pentoxy]-N,N-bis(1-me thylethyl)benzamid oder ein pharmazeutisch verträgliches Salz davon hergestellt wird.

19. Verfahren nach Anspruch 1, wobei 4-[5-[4-[Imino(phenylmethoxycarbonylamino) methyl]phenoxy]pentoxy] -3-methoxy-N,N-bis(1-methylethyl)benzamid oder ein pharmazeutisch verträgliches Salz davon hergestellt wird.

## Revendications (Revendications pour l'(les) Etat(s) contractant(s) suivant(s): AT, BE, CH, DE, DK, FR, GB, IT, LI, LU, NL, PT, SE)

1. Composé de formule : 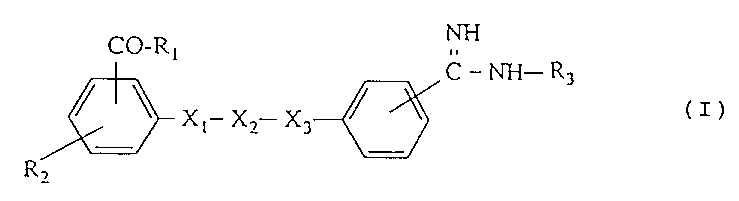 dans laquelle :
R₁ est un groupe amino qui est mono- ou disubstitué par un substituant choisi parmi les radicaux hydrocarbonés aliphatiques, les radicaux hydrocarbonés araliphatiques, les radicaux aromatiques et les radicaux hydrocarbonés cycloaliphatiques, ou encore est un groupe amino qui est disubstitué par un radical hydrocarboné aliphatique divalent ;
R₂ est un hydrogène ou un radical halogéno, trifluorométhyle, un radical hydrocarboné aliphatique, ou encore un groupe hydroxy éthérifié par un alcool aliphatique, un alcool araliphatique ou un alcool aromatique, ou qui est estérifié par un acide carboxylique aliphatique ou araliphatique ;
R₃ est un hydrogène ou un radical acyle qui dérive d'un acide carbonique organique, d'un acide carboxylique organique, d'un acide sulfonique ou d'un acide carbamique ; X₁ et X₃, indépendamment l'un de l'autre, sont chacun un oxygène (-O-) ou un soufre (-S-) ; et
X₂ est un radical hydrocarboné aliphatique divalent qui peut être interrompu par un radical aromatique ;
où les noyaux phényle de formule I peuvent, indépendamment l'un de l'autre, être encore substitués par un ou plusieurs substituants choisis parmi les radicaux halogéno, trifluorométhyle, les radicaux hydrocarbonés aliphatiques, hydroxy, et hydroxy qui est éthérifié par un alcool aliphatique ou qui est estérifié par un acide carboxylique aliphatique ou araliphatique ;
où les groupes aryle, dans les définitions ci-dessus, peuvent, indépendamment les uns des autres, être encore substitués par un ou plusieurs substituants choisis parmi les radicaux halogéno, trifluorométhyle, les radicaux hydrocarbonés aliphatiques, hydroxy, et hydroxy qui est éthérifié par un alcool aliphatique ou qui est estérifié par un acide carboxylique aliphatique ou araliphatique ; où un radical hydrocarboné cycloaliphatique peut être substitué par un radical aliphatique ;
ou l'un de ses sels acceptables d'un point de vue pharmaceutique.

2. Composé selon la revendication 1, dans lequel : R₁ est un groupe amino qui est mono- ou disubstitué par un substituant choisi parmi les radicaux alkyle en C₁-C₇, alcényle en C₃-C₇, alcynyle en C₃-C₇, phényl-(alkyle en C₁-C₇), phényl-(alcényle en C₂-C₇), phényl-(alcynyle en C₂-C₇), phényle, naphtyle, indanyle, fluorényle, cycloalkyle et cycloalcényle, les radicaux cycloalkyle et cycloalcényle étant chacun non-substitués ou mono- ou polysubstitués par des radicaux alkyle en C₁-C₇, ou encore est un radical amino qui est disubstitué par un radical alkylène en C₂-C₆ ;
R₂ est un hydrogène ou un radical halogéno, trifluorométhyle, alkyle en C₁-C₇, alcényle en C₃-C₇, alcynyle en C₃-C₇, alcoxy en C₁-C₇, alcényloxy en C₃-C₇, phényl-(alcoxy en C₁-C₇), phénoxy, alcanoyloxy en C₂-C₈, alcénoyloxy en C₃-C₈ ou phényl-(alcanoyloxy en C₂-C₈) ;
R₃ est un hydrogène ou un radical alcoxycarbonyle ou alcényloxycarbonyle, dont chacun est non-substitué ou substitué par des radicaux phényle, naphtyle, indanyle ou fluorényle, ou encore est un radical cycloalcoxycarbonyle qui est non-substitué ou mono- ou polysubstitué par des radicaux alkyle en C₁-C₇, ou encore est un radical alcanoyle en C₁-C₇ ou phényl-(alcanoyle en C₂-C₇), ou encore est le radical benzoyle, naphtoyle, indanoyle ou fluorénoyle, ou encore est un radical alcanesulfonyle en C₁-C₇, phényl-(alcanesulfonyle en C₁-C₇), cycloalcanesulfonyle en C₃-C₇ ou phénylsulfonyle, ou encore est un radical aminocarbonyle qui est substitué par des radicaux alkyle en C₁-C₇, phényl-(alkyle en C₁-C₇) ou phényle ;
X₁ et X₃, indépendamment l'un de l'autre, sont chacun O ou S ;
X₂ est un radical alkylène en C₂-C₈, (alkylène en C₂-C₄)-phénylène-(alkylène en C₂-C₄) ou (alkylène en C₂-C₄)-naphtylène-(alkylène en C₂-C₄) ;
où les noyaux phényle de la formule I peuvent, indépendamment l'un de l'autre, être substitués par un ou plusieurs substituants choisis parmi les halogènes et les radicaux trifluorométhyle, alkyle en C₁-C₇, alcényle en C₃-C₇, alcynyle en C₃-C₇, hydroxy, alcoxy en C₁-C₇, alcényloxy en C₃-C₇, phényl-(alcoxy en C₁-C₇), alcanoyloxy en C₂-C₈, alcénoyloxy en C₃-C₇ et phényl-(alcanoyloxy en C₂-C₈) ;
où les radicaux aromatiques, dans les définitions ci-dessus, peuvent, indépendamment l'un de l'autre, être substitués par un ou plusieurs substituants choisis parmi les halogènes et les radicaux trifluorométhyle, alkyle en C₁-C₇, alcényle en C₃-C₇, alcynyle en C₃-C₇, hydroxy, alcoxy en C₁-C₇, alcényloxy en C₃-C₇, phényl-(alcoxy en C₁-C₇), alcanoyloxy en C₂-C₈, alcénoyloxy en C₃-C₈ et phényl-(alcanoyloxy en C₂-C₈) ;
ou l'un de ses sels acceptables d'un point de vue pharmaceutique.

3. Composé selon la revendication 1, dans lequel : R₁ est un groupe amino qui est mono- ou disubstitué par un substituant choisi parmi les radicaux alkyle en C₁-C₇, phényl-(alkyle en C₁-C₇), phényle et cycloalkyle en C₃-C₆, le radical cycloalkyle en C₃-C₆ étant non-substitué ou mono- ou polysubstitué par des radicaux alkyle en C₁-C₇, ou encore est un radical amino qui est disubstitué par des radicaux alkylène en C₃-C₆ ;
R₂ est un hydrogène ou un radical alcoxy en C₁-C₇ ou phényl-(alcoxy en C₁-C₄) ;
R₃ est un hydrogène ou un radical (alcoxy en C₁-C₁₂)-carbonyle, alcanoyle en C₂-C₅, phényl-(alcanoyle en C₂-C₅), benzoyle qui est non-substitué ou substitué par des radicaux halogéno, trifluorométhyle, alkyle en C₁-C₇ ou alcoxy en C₁-C₇, ou encore est un radical (cycloalkyl en C₃-C₆)-carbonyle qui est non-substitué ou substitué par des radicaux alkyle en C₁-C₇, ou encore est le radical naphtoyle, indanoyle ou fluorénoyle, ou encore est un radical alcanesulfonyle en C₁-C₇, phényl-(alcanesulfonyle en C₁-C₇), cycloalcanesulfonyle en C₃-C₇ ou phénylsulfonyle, ou encore est un radical aminocarbonyle qui est substitué par des substituants alkyle en C₁-C₇, phényl-(alkyle en C₁-C₇) ou phényle ;
X₁ et X₃, sont chacun -O-, ou de plus représentent, chacun indépendamment l'un de l'autre, -O- ou -S- ;
X₂ est un radical alkylène en C₂-C₇, ou (alkylène en C₂-C₄)-phénylène-(alkylène en C₂-C₄) ;
où les noyaux phényle de formule I peuvent être non-substitués ou encore, indépendamment l'un de l'autre, être chacun substitués par un ou plusieurs substituants choisis parmi les radicaux halogéno, trifluorométhyle, alkyle en C₁-C₇ et alcoxy en C₁-C₇ ;
où les radicaux phényle, dans les définitions ci-dessus, sont non-substitués ou de plus, indépendamment l'un de l'autre, sont chacun substitués par un ou plusieurs substituants choisis parmi les radicaux halogéno, trifluorométhyle, alkyle en C₁-C₇ et alcoxy en C₁-C₇ ;
ou l'un de ses sels acceptables d'un point de vue pharmaceutique.

4. Composé selon la revendication 1, dans lequel -CO-R₁ est placé en position 4 (para) ou 5 (méta) ;
R₂- est situé en position 2 (ortho) ; et -C(=NH)-NH-R₃ est situé en position 4 (para) du noyau phényle correspondant.

5. Composé selon la revendication 1, de formule IA : 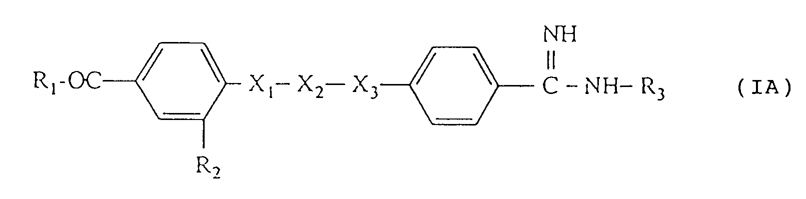 dans laquelle :
R₁ est un radical di-(alkyl en C₁-C₄)-amino, (alkyl en C₁-C₄)-phényl-(alkyl en C₁-C₄)-amino, di-(cycloalkyl en C₃-C₆)-amino qui est non-substitué ou est substitué par des radicaux alkyle en C₁-C₄ ou 1-pipéridino substitué par des radicaux alkyle en C₁-C₄ ;
R₂ est un hydrogène ou un radical alcoxy en C₁-C₄ ;
R₃ est un hydrogène, un radical (alcoxy en C₁-C₁₂)-carbonyle, phényl-(alcoxy en C₁-C₄)-carbonyle, alcanoyle en C₂-C₅, benzoyle qui est non-substitué ou substitué par des substituants halogéno, trifluorométhyle, alkyle en C₁-C₄ ou alcoxy en C₁-C₄, (cycloalkyl en C₃-C₆)-carbonyle qui est non-substitué ou substitué par des substituants alkyle en C₁-C₄ ;
X₁ et X₃ sont -O- ;
X₂ est un radical alkylène en C₄-C₇ ;
où les noyaux phényle de la formule IA peuvent être non-substitués, ou encore, indépendamment l'un de l'autre, être substitués par un ou plusieurs substituants choisis parmi les radicaux halogéno, trifluorométhyle, alkyle en C₁-C₄ et alcoxy en C₁-C₄ ;
ou l'un de ses sels acceptables d'un point de vue pharmaceutique.

6. Composé selon la revendication 1 de formule IA, où :
R₁ est un radical di-(alkyl en C₁-C₄)-amino, (alkyl en C₁-C₄)-phényl-(alkyl en C₁-C₄)-amino, di-(cycloalkyl en C₃-C₆)-amino qui est non-substitué ou est substitué par des radicaux alkyle en C₁-C₄ ou 1-pipéridino substitué par des radicaux alkyle en C₁-C₄ ;
R₂ est un hydrogène ou un radical alcoxy en C₁-C₄ ; R₃ est un radical alcanesulfonyle en C₁-C₄, phényl-(alcanesulfonyle en C₁-C₄), cycloalcanesulfonyle en C₃-C₇ ou phénylsulfonyle, ou encore est un radical aminocarbonyle qui est substitué par des substituants alkyle en C₁-C₄, phényl-(alkyle en C₁-C₄) ou phényle ;
X₁ et X₃ sont -O- ;
X₂ est un radical alkylène en C₄-C₇ ;
ou l'un de ses sels acceptables d'un point de vue pharmaceutique.

7. Composé selon la revendication 5 de formule IA, dans lequel :
R₁ est un radical di-(alkyl en C₁-C₄)-amino ;
R₂ est un hydrogène ou un radical alcoxy en C₁-C₄ ;
R₃ est un radical (alcoxy en C₁-C₁₂)-carbonyle, phényl(alcoxy en C₁-C₄)-carbonyle, alcanoyle en C₂-C₅, benzoyle qui est non-substitué ou substitué par des radicaux halogéno, trifluorométhyle, alkyle en C₁-C₄ ou alcoxy en C₁-C₄, (cycloalkyl en C₃-C₆)-carbonyle qui est non-substitué ou substitué par des radicaux alkyle en C₁-C₄ ;
X₁ et X₃ sont -O- ;
X₂ est un radical alkylène en C₄-C₇ ;
ou l'un de ses sels acceptables d'un point de vue pharmaceutique.

8. Composé selon la revendication 5 de formule IA, dans lequel :
R₁ est un radical di-(alkyl en C₁-C₄)-amino ;
R₂ est un hydrogène ou un radical alcoxy en C₁-C₄ ;
R₃ est un hydrogène ;
X₁ et X₃ sont -O- ;
X₂ est un radical alkylène en C₄-C₇ ;
ou l'un de ses sels acceptables d'un point de vue pharmaceutique.

9. Composé selon la revendication 5 de formule IA, dans lequel :
R₁ est le radical diéthylamino ou diisopropylamino ;
R₂ est un hydrogène ou le radical méthoxy ;
R₃ est un hydrogène ;
X₁ et X₃ sont -O- ;
X₂ est le pentylène ;
ou l'un de ses sels acceptables d'un point de vue pharmaceutique.

10. 4-[5-[4-(aminoiminométhyl)phénoxy]pentoxy]-3-méthoxy-N,N-bis(1-méthyléthyl)benzamide ou l'un de ses sels acceptables d'un point de vue pharmaceutique selon la revendication 1.

11. 4-[5-[4-(aminoiminométhyl)phénoxy]pentoxy]-3-méthoxy-N,N-diéthylbenzamide ou l'un de ses sels acceptables d'un point de vue pharmaceutique selon la revendication 1.

12. 4-[5-[4-(aminoiminométhyl)phénoxy]pentoxy]-3-méthoxy-N-(1-méthyléthyl)-N-(phénylméthyl)benzamide ou l'un de ses sels acceptables d'un point de vue pharmaceutique selon la revendication 1.

13. 4-[5-[4-(aminoiminométhyl)phénoxy]pentoxy]-N,N-bis(1-méthyléthyl)benzamide ou l'un de ses sels acceptables d'un point de vue pharmaceutique selon la revendication 1.

14. 3-[5-[4-(aminoiminométhyl)phénoxy]pentoxy]-N,N-bis(1-méthyléthyl)benzamide ou l'un de ses sels acceptables d'un point de vue pharmaceutique selon la revendication 1.

15. 4-[5-[4-(aminoiminométhyl)phénoxy]pentoxy]-2-hydroxy-N,N-bis(1-méthyléthyl)benzamide ou l'un de ses sels acceptables d'un point de vue pharmaceutique selon la revendication 1.

16. 4-[5-[4-[(benzoylamino)iminométhyl]phénoxy]pentoxy]-3-méthoxy-N,N-bis(1-méthyléthyl)benzamide ou l'un de ses sels acceptables d'un point de vue pharmaceutique selon la revendication 1.

17. 4-[5-[4-[imino[octyloxycarbonyl]amino]méthyl]phénoxy]pentoxy]-3-méthoxy-N,N-bis(1-méthyléthyl)benzamide ou l'un de ses sels acceptables d'un point de vue pharmaceutique selon la revendication 1.

18. 4-[5-[4-[imino[octyloxycarbonyl]amino]méthyl]phénoxy]pentoxy]-N,N-bis(1-méthyléthyl)benzamide ou l'un de ses sels acceptables d'un point de vue pharmaceutique selon la revendication 1.

19. 4-[5-[4-[imino(phénylméthoxycarbonylamino)méthyl]phénoxy]pentoxy]-3-méthoxy-N,N-bis(1-méthyléthyl)benzamide ou l'un de ses sels acceptables d'un point de vue pharmaceutique selon la revendication 1.

20. Procédé pour produire des composés de formule I selon la revendication 1 et leurs sels acceptables d'un point de vue pharmaceutique, qui consiste :
a) à convertir, dans un composé de formule IIA 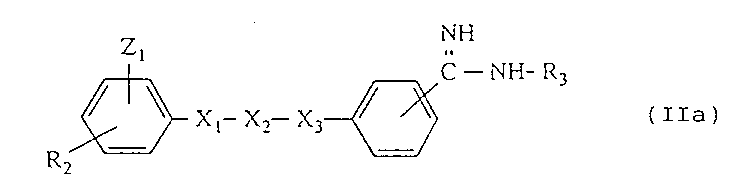 ou l'un de ses sels, où Z₁ est un radical qui peut être converti en la variable -CO-R₁, Z₁ en la variable -CO-R₁, ou bien
b) pour préparer des composés de formule I dans laquelle R₃ est un hydrogène, dans un composé de formule IIIa 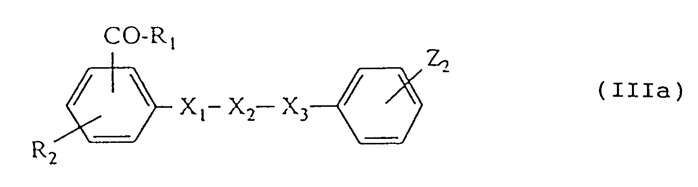 ou l'un de ses sels, où Z₂ est un radical qui peut être converti en la variable -C(=NH)-NH-R₃, à convertir Z₂ en la variable -C(=NH)-NH-R₃, ou bien
c) à faire réagir un composé de formule IVa 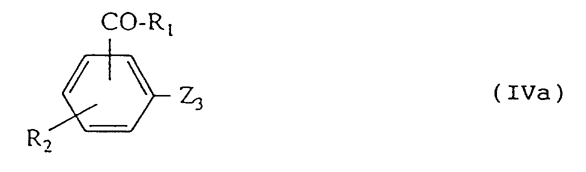 ou l'un de ses sels avec un composé de formule IVb 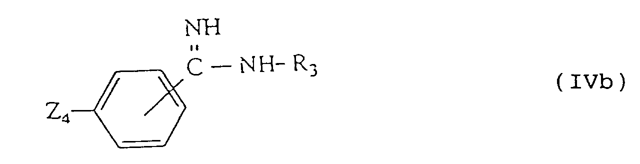 ou l'un de ses sels, où Z₃ est un groupe de formule -X₁-X₂-Z₅ et Z₄ est -Z₆, ou bien Z₃ est -Z₆ et Z₄ est un groupe de formule Z₅-X₂-X₃-, où l'un des radicaux Z₅ et Z₆ est un groupe hydroxy ou mercapto et l'autre est un groupe hydroxy, mercapto ou hydroxy à estérification réactive,
et, si on le souhaite, à convertir un composé de formule I ou l'un de ses sels pouvant être obtenu selon le procédé ou d'une autre manière en un autre composé ou un de ses sels selon l'invention, à convertir en un sel un composé libre de formule I pouvant être obtenu par le procédé, à convertir un sel pouvant être obtenu par le procédé en le composé libre de formule I ou en un autre sel, ou à dédoubler un mélange d'isomères pouvant être obtenus selon le procédé et à isoler le composé recherché.

21. Préparation pharmaceutique contenant un composé de formule I selon la revendication 1 et des excipients usuels.

22. Utilisation des composés de formule I selon la revendication 1 ou de leurs sels et d'excipients usuels pour produire des préparations pharmaceutiques.

23. Composé de formule I selon la revendication 1 ou ses sels, pour utilisation dans une méthode de traitement thérapeutique de l'homme ou d'un organisme animal.

24. Composés de formule I selon la revendication 1 ou leurs sels, pour utilisation comme antagonistes du LTB₄ sur les leucocytes polymorphonucléaires humains.

25. Composition pharmaceutique permettant d'antagoniser les récepteurs du LTB₄ chez les mammifères, qui comprend une quantité efficace antagonisant les récepteurs du LTB₄ d'un composé selon la revendication 1, ou d'un de ses sels acceptables d'un point de vue pharmaceutique et d'un excipient.

## Revendications (Revendications pour l'(les) Etat(s) contractant(s) suivant(s): ES, GR)

1. Procédé pour préparer un composé de formule I : 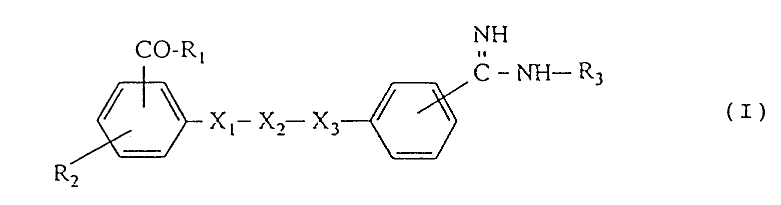 dans laquelle :
R₁ est un groupe amino qui est mono- ou disubstitué par un substituant choisi parmi les radicaux hydrocarbonés aliphatiques, les radicaux hydrocarbonés araliphatiques, les radicaux aromatiques et les radicaux hydrocarbonés cycloaliphatiques, ou encore est un groupe amino qui est disubstitué par un radical hydrocarboné aliphatique divalent ;
R₂ est un hydrogène ou un radical halogéno, trifluorométhyle, un radical hydrocarboné aliphatique, ou encore un groupe hydroxy éthérifié par un alcool aliphatique, un alcool araliphatique ou un alcool aromatique, ou qui est estérifié par un acide carboxylique aliphatique ou araliphatique ;
R₃ est un hydrogène ou un radical acyle qui dérive d'un acide carbonique organique, d'un acide carboxylique organique, d'un acide sulfonique ou d'un acide carbamique ;
X₁ et X₃, indépendamment l'un de l'autre, sont chacun un oxygène (-O-) ou un soufre (-S-) ; et
X₂ est un radical hydrocarboné aliphatique divalent qui peut être interrompu par un radical aromatique ;
où les noyaux phényle de formule I peuvent, indépendamment l'un de l'autre, être encore substitués par un ou plusieurs substituants choisis parmi les radicaux halogéno, trifluorométhyle, les radicaux hydrocarbonés aliphatiques, hydroxy, et hydroxy qui est éthérifié par un alcool aliphatique ou qui est estérifié par un acide carboxylique aliphatique ou araliphatique ;
où les groupes aryle, dans les définitions ci-dessus, peuvent, indépendamment les uns des autres, être encore substitués par un ou plusieurs substituants choisis parmi les radicaux halogéno, trifluorométhyle, les radicaux hydrocarbonés aliphatiques, hydroxy, et hydroxy qui est éthérifié par un alcool aliphatique ou qui est estérifié par un acide carboxylique aliphatique ou araliphatique ;
où un radical hydrocarboné cycloaliphatique peut être substitué par un radical aliphatique ;
ou l'un de ses sels acceptables d'un point de vue pharmaceutique, qui consiste :
a) à convertir, dans un composé de formule IIA 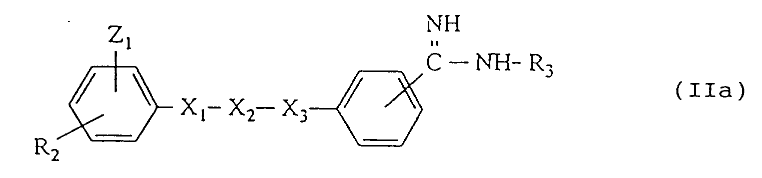 ou l'un de ses sels, où Z₁ est un radical qui peut être converti en la variable -CO-R₁, Z₁ en la variable -CO-R₁, ou bien
b) pour préparer des composés de formule I dans laquelle R₃ est un hydrogène, dans un composé de formule IIIa 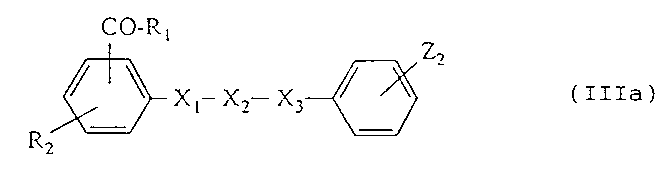 ou l'un de ses sels, où Z₂ est un radical qui peut être converti en la variable -C(=NH)-NH-R₃_{,} à convertir Z₂ en la variable -C(=NH)-NH-R₃, ou bien
c) à faire réagir un composé de formule IVa 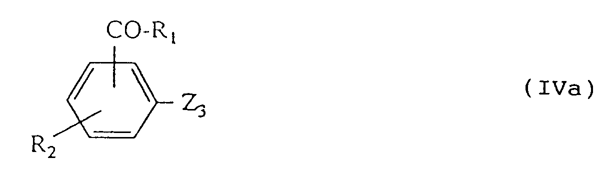 ou l'un de ses sels avec un composé de formule IVb 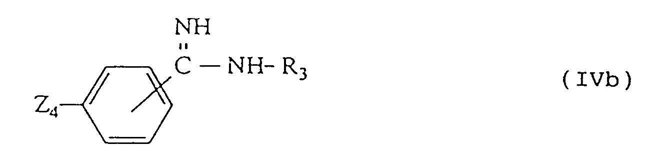 ou l'un de ses sels, où Z₃ est un groupe de formule -X₁-X₂-Z₅ et Z₄ est -Z₆, ou bien Z₃ est -Z₆ et Z₄ est un groupe de formule Z₅-X₂-X₃-, où l'un des radicaux Z₅ et Z₆ est un groupe hydroxy ou mercapto et l'autre est un groupe hydroxy, mercapto ou hydroxy à estérification réactive,
et, si on le souhaite, à convertir un composé de formule I ou l'un de ses sels pouvant être obtenu selon le procédé ou d'une autre manière en un autre composé ou l'un de ses sels selon l'invention, à convertir un composé libre de formule I pouvant être obtenu par le procédé en un sel, à convertir un sel pouvant être obtenu par le procédé en le composé libre de formule I ou en un autre sel, ou à dédoubler un mélange d'isomères pouvant être obtenus selon le procédé et à isoler le composé recherché.

2. Procédé selon la revendication 1, dans lequel on produit des composés de formule I dans laquelle : R₁ est un groupe amino qui est mono- ou disubstitué par un substituant choisi parmi les radicaux alkyle en C₁-C₇, alcényle en C₃-C₇, alcynyle en C₃-C₇, phényl-(alkyle en C₁-C₇), phényl-(alcényle en C₂-C₇), phényl-(alcynyle en C₂-C₇), phényle, naphtyle, indanyle, fluorényle, cycloalkyle et cycloalcényle, les radicaux cycloalkyle et cycloalcényle étant chacun non-substitués ou mono- ou polysubstitués par des radicaux alkyle en C₁-C₇, ou encore est un radical amino qui est disubstitué par un radical alkylène en C₂-C₆ ;
R₂ est un hydrogène ou un radical halogéno, trifluorométhyle, alkyle en C₁-C₇, alcényle en C₃-C₇, alcynyle en C₃-C₇, alcoxy en C₁-C₇, alcényloxy en C₃-C₇, phényl-(alcoxy en C₁-C₇), phénoxy, alcanoyloxy en C₂-C₈, alcénoyloxy en C₃-C₈ ou phényl-(alcanoyloxy en C₂-C₈) ;
R₃ est un hydrogène ou un radical alcoxycarbonyle ou alcényloxycarbonyle, dont chacun est non-substitué ou substitué par des radicaux phényle, naphtyle, indanyle ou fluorényle, ou encore est un radical cycloalcoxycarbonyle qui est non-substitué ou mono- ou polysubstitué par des radicaux alkyle en C₁-C₇, ou encore est un radical alcanoyle en C₁-C₇ ou phényl-(alcanoyle en C₂-C₇), ou encore est le radical benzoyle, naphtoyle, indanoyle ou fluorénoyle, ou encore est un radical alcanesulfonyle en C₁-C₇, phényl-(alcanesulfonyle en C₁-C₇), cycloalcanesulfonyle en C₃-C₇ ou phénylsulfonyle, ou encore est un radical aminocarbonyle qui est substitué par des radicaux alkyle en C₁-C₇, phényl-(alkyle en C₁-C₇) ou phényle ;
X₁ et X₃, indépendamment l'un de l'autre, sont chacun O ou S ;
X₂ est un radical alkylène en C₂-C₈, (alkylène en C₂-C₄)-phénylène-(alkylène en C₂-C₄) ou (alkylène en C₂-C₄)-naphtylène-(alkylène en C₂-C₄) ;
où les noyaux phényle de la formule I peuvent, indépendamment l'un de l'autre, être substitués par un ou plusieurs substituants choisis parmi les halogènes et les radicaux trifluorométhyle, alkyle en C₁-C₇, alcényle en C₃-C₇, alcynyle en C₃-C₇, hydroxy, alcoxy en C₁-C₇, alcényloxy en C₃-C₇, phényl-(alcoxy en C₁-C₇), alcanoyloxy en C₂-C₈, alcénoyloxy en C₃-C₇ et phényl-(alcanoyloxy en C₂-C₈) ;
où les radicaux aromatiques, dans les définitions ci-dessus, peuvent, indépendamment l'un de l'autre, être substitués par un ou plusieurs substituants choisis parmi les halogènes et les radicaux trifluorométhyle, alkyle en C₁-C₇, alcényle en C₃-C₇, alcynyle en C₃-C₇, hydroxy, alcoxy en C₁-C₇, alcényloxy en C₃-C₇, phényl-(alcoxy en C₁-C₇), alcanoyloxy en C₂-C₈, alcénoyloxy en C₃-C₈ et phényl-(alcanoyloxy en C₂-C₈) ;
ou leurs sels acceptables d'un point de vue pharmaceutique.

3. Procédé selon la revendication 1, dans lequel on produit des composés de formule I dans laquelle :
R₁ est un groupe amino qui est mono- ou disubstitué par un substituant choisi parmi les radicaux alkyle en C₁-C₇, phényl-(alkyle en C₁-C₇), phényle et cycloalkyle en C₃-C₆, le radical cycloalkyle en C₃-C₆ étant non-substitué ou mono- ou polysubstitué par des radicaux alkyle en C₁-C₇, ou encore est un radical amino qui est disubstitué par des radicaux alkylène en C₃-C₆ ;
R₂ est un hydrogène ou un radical alcoxy en C₁-C₇ ou phényl-(alcoxy en C₁-C₄) ;
R₃ est un hydrogène ou un radical (alcoxy en C₁-C₁₂)-carbonyle, alcanoyle en C₂-C₅, phényl-(alcanoyle en C₂-C₅), benzoyle qui est non-substitué ou substitué par des radicaux halogéno, trifluorométhyle, alkyle en C₁-C₇ ou alcoxy en C₁-C₇, ou encore est un radical (cycloalkyl en C₃-C₆)-carbonyle qui est non-substitué ou substitué par des radicaux alkyle en C₁-C₇, ou encore est le radical naphtoyle, indanoyle ou fluorénoyle, ou encore est un radical alcanesulfonyle en C₁-C₇, phényl-(alcanesulfonyle en C₁-C₇), cycloalcanesulfonyle en C₃-C₇ ou phénylsulfonyle, ou encore est un radical aminocarbonyle qui est substitué par des substituants alkyle en C₁-C₇, phényl-(alkyle en C₁-C₇) ou phényle ;
X₁ et X₃, sont chacun -O-, ou de plus représentent, chacun indépendamment l'un de l'autre, -O- ou -S- ;
X₂ est un radical alkylène en C₂-C₇, ou (alkylène en C₂-C₄)-phénylène-(alkylène en C₂-C₄) ;
où les noyaux phényle de formule I peuvent être non-substitués ou encore, indépendamment l'un de l'autre, être chacun substitués par un ou plusieurs substituants choisis parmi les radicaux halogéno, trifluorométhyle, alkyle en C₁-C₇ et alcoxy en C₁-C₇ ;
où les radicaux phényle, dans les définitions ci-dessus, sont non-substitués ou de plus, indépendamment l'un de l'autre, sont chacun substitués par un ou plusieurs substituants choisis parmi les radicaux halogéno, trifluorométhyle, alkyle en C₁-C₇ et alcoxy en C₁-C₇ ;
ou leurs sels acceptables d'un point de vue pharmaceutique.

4. Procédé selon la revendication 1, dans lequel on produit des composés de formule I dans laquelle -CO-R₁ est placé en position 4 (para) ou 5 (méta) ; R₂- est situé en position 2 (ortho) ; et -C(=NH)-NH-R₃ est situé en position 4 (para) du noyau phényle correspondant.

5. Procédé selon la revendication 1, dans lequel on produit des composés de formule IA : 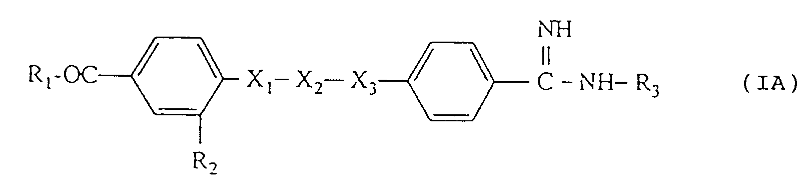 dans laquelle :
R₁ est un radical di-(alkyl en C₁-C₄)-amino, (alkyl en C₁-C₄)-phényl-(alkyl en C₁-C₄)-amino, di-(cycloalkyl en C₃-C₆)-amino qui est non-substitué ou est substitué par des radicaux alkyle en C₁-C₄ ou 1-pipéridino substitué par des radicaux alkyle en C₁-C₄ ;
R₂ est un hydrogène ou un radical alcoxy en C₁-C₄ ; R₃ est un hydrogène, un radical (alcoxy en C₁-C₁₂)-carbonyle, phényl-(alcoxy en C₁-C₄)-carbonyle, alcanoyle en C₂-C₅, benzoyle qui est non-substitué ou substitué par des substituants halogéno, trifluorométhyle, alkyle en C₁-C₄ ou alcoxy en C₁-C₄, (cycloalkyl en C₃-C₆)-carbonyle qui est non-substitué ou substitué par des substituants alkyle en C₁-C₄ ;
X₁ et X₃ sont -O- ;
X₂ est un radical alkylène en C₄-C₇ ;
où les noyaux phényle de la formule IA peuvent être non-substitués, ou encore, indépendamment l'un de l'autre, être substitués par un ou plusieurs substituants choisis parmi les radicaux halogéno, trifluorométhyle, alkyle en C₁-C₄ et alcoxy en C₁-C₄ ;
ou leurs sels acceptables d'un point de vue pharmaceutique.

6. Procédé selon la revendication 1, dans lequel on produit des composés de formule IA, où :
R₁ est un radical di-(alkyl en C₁-C₄)-amino, (alkyl en C₁-C₄)-phényl-(alkyl en C₁-C₄)-amino, di-(cycloalkyl en C₃-C₆)-amino qui est non-substitué ou est substitué par des radicaux alkyle en C₁-C₄ ou 1-pipéridino substitué par des radicaux alkyle en C₁-C₄ ;
R₂ est un hydrogène ou un radical alcoxy en C₁-C₄ ;
R₃ est un radical alcanesulfonyle en C₁-C₄, phényl-(alcanesulfonyle en C₁-C₄), cycloalcanesulfonyle en C₃-C₇ ou phénylsulfonyle, ou encore est un radical aminocarbonyle qui est substitué par des substituants alkyle en C₁-C₄, phényl-(alkyle en C₁-C₄) ou phényle ;
X₁ et X₃ sont -O- ;
X₂ est un radical alkylène en C₄-C₇ ;
ou leurs sels acceptables d'un point de vue pharmaceutique.

7. Procédé selon la revendication 1, dans lequel on produit des composés de formule IA, dans laquelle :
R₁ est un radical di-(alkyl en C₁-C₄)-amino ;
R₂ est un hydrogène ou un radical alcoxy en C₁-C₄ ;
R₃ est un radical (alcoxy en C₁-C₁₂)-carbonyle, phényl-(alcoxy en C₁-C₄)-carbonyle, alcanoyle en C₂-C₅, benzoyle qui est non-substitué ou substitué par des radicaux halogéno, trifluorométhyle, alkyle en C₁-C₄ ou alcoxy en C₁-C₄, (cycloalkyl en C₃-C₆)-carbonyle qui est non-substitué ou substitué par des radicaux alkyle en C₁-C₄ ;
X₁ et X₃ sont -O- ;
X₂ est un radical alkylène en C₄-C₇ ;
ou leurs sels acceptables d'un point de vue pharmaceutique.

8. Procédé selon la revendication 1, dans lequel on produit des composés de formule IA, dans laquelle :
R₁ est un radical di-(alkyl en C₁-C₄)-amino ;
R₂ est un hydrogène ou un radical alcoxy en C₁-C₄ ;
R₃ est un hydrogène ;
X₁ et X₃ sont -O- ;
X₂ est un radical alkylène en C₄-C₇ ;
ou leurs sels acceptables d'un point de vue pharmaceutique.

9. Procédé selon la revendication 1, dans lequel on produit des composés de formule IA, dans laquelle :
R₁ est le radical diéthylamino ou diisopropylamino ;
R₂ est un hydrogène ou le radical méthoxy ;
R₃ est un hydrogène ;
X₁ et X₃ sont -O- ;
X₂ est le pentylène ;
ou leurs sels acceptables d'un point de vue pharmaceutique.

10. Procédé selon la revendication 1, dans lequel on produit le 4-[5-[4-(aminoiminométhyl)phénoxy]pentoxy]-3-méthoxy-N,N-bis(1-méthyléthyl)benzamide ou l'un de ses sels acceptables d'un point de vue pharmaceutique

11. Procédé selon la revendication 1, dans lequel on produit le 4-[5-[4-(aminoiminométhyl)phénoxy]pentoxy]-3-méthoxy-N,N-diéthylbenzamide ou l'un de ses sels acceptables d'un point de vue pharmaceutique.

12. Procédé selon la revendication 1, dans lequel on produit le 4-[5-[4-(aminoiminométhyl)phénoxy]pentoxy]-3-méthoxy-N-(1-méthyléthyl)-N-(phénylméthyl)benzamide ou l'un de ses sels acceptables d'un point de vue pharmaceutique.

13. Procédé selon la revendication 1, dans lequel on produit le 4-[5-[4-(aminoiminométhyl)phénoxy]pentoxy]-N,N-bis(1-méthyléthyl)benzamide ou l'un de ses sels acceptables d'un point de vue pharmaceutique.

14. Procédé selon la revendication 1, dans lequel on produit le 3-[5-[4-(aminoiminométhyl)phénoxy]pentoxy]-N,N-bis(1-méthyléthyl)benzamide ou l'un de ses sels acceptables d'un point de vue pharmaceutique.

15. Procédé selon la revendication 1, dans lequel on produit le 4-[5-[4-(aminoiminométhyl)phénoxy]pentoxy]-2-hydroxy-N,N-bis(1-méthyléthyl)benzamide ou l'un de ses sels acceptables d'un point de vue pharmaceutique.

16. Procédé selon la revendication 1, dans lequel on produit le 4-[5-[4-[(benzoylamino)iminométhyl]phénoxy]pentoxy]-3-méthoxy-N,N-bis(1-méthyléthyl)benzamide ou l'un de ses sels acceptables d'un point de vue pharmaceutique.

17. Procédé selon la revendication 1, dans lequel on produit le 4-[5-[4-[imino[octyloxycarbonyl]amino]méthyl]phénoxy]pentoxy]-3-méthoxy-N,N-bis(1-méthyléthyl)benzamide ou l'un de ses sels acceptables d'un point de vue pharmaceutique.

18. Procédé selon la revendication 1, dans lequel on produit le 4-[5-[4-[imino[octyloxycarbonyl]amino]méthyl]phénoxy]pentoxy]-N,N-bis(1-méthyléthyl)benzamide ou l'un de ses sels acceptables d'un point de vue pharmaceutique.

19. Procédé selon la revendication 1, dans lequel on produit le 4-[5-[4-[imino(phénylméthoxycarbonylamino)méthyl]phénoxy]pentoxy]-3-méthoxy-N,N-bis(1-méthyléthyl)benzamide ou l'un de ses sels acceptables d'un point de vue pharmaceutique.
